# EUROPEAN PATENT APPLICATION

(11) **EP 4 461 728 A1**
(43) Date of publication of application: **13.11.2024**
(21) Application number: 23737416.0
(22) Date of filing: 05.01.2023
(51) Int. Cl.: C07D 251/24, C07D 239/26, C07D 405/04, C07D 495/04, C07D 403/10, C07D 405/14, C07D 405/10, C07D 409/10, C07D 409/14, C07F 7/08

(54) **ORGANIC COMPOUND AND ORGANIC ELECTROLUMINESCENT DEVICE USING SAME**

(30) Priority: 05.01.2022 KR 20220001818
(71) Applicant: Solus Advanced Materials Co., Ltd., Iksan-si, Jeollabuk-do 54584 (KR)
(72) Inventor: BAE, Hyungchan, Yongin-si, Gyeonggi-do 16858 (KR); SIM, Jaeyi, Yongin-si, Gyeonggi-do 16858 (KR); SON, Hyosuk, Yongin-si, Gyeonggi-do 16858 (KR); LEE, Yonghwan, Yongin-si, Gyeonggi-do 16858 (KR); HAN, Kyuwon, Yongin-si, Gyeonggi-do 16858 (KR); KIM, Jinwoong, Yongin-si, Gyeonggi-do 16858 (KR)
(74) Representative: Office Freylinger
(86) International application number: PCT/KR2023/000248
(87) International publication number: WO 2023/132668

(57) **Abstract**

The present invention relates to a novel organic compound and an organic electroluminescent device using the same, and more specifically, to an organic compound having excellent heat resistance, carrier transport ability, light emission capability, and the like, and an organic electroluminescent device having improved characteristics, such as light emission efficiency, driving voltage, and lifespan, by containing the organic compound in at least one organic layer.

## Description

### Technical Field

The present invention relates to a novel organic compound and an organic electroluminescent device using the same and, more specifically, to a compound with excellent electron transport ability and an organic electroluminescent device having improved characteristics, such as light emission efficiency, driving voltage, and lifespan, by containing the compound in at least one organic layer.

### Background Art

In an organic electroluminescent device (hereinafter, referred to as "organic EL device"), upon the application of voltage between two electrodes, holes are injected into organic layers from an anode and electrons are injected into organic layers from a cathode. The injected holes and electrons meet each other to form excitons, and the excitons fall down to the ground state to emit light. Particularly, materials used for the organic layers may be classified into light emission materials, hole injection materials, hole transport materials, electron transport materials, electron injection materials, and the like, according to functions thereof.

Materials for forming a light emission layer of the organic EL device may be classified into blue, green and red light emission materials according to the color of light emission. In addition, yellow and orange light emission materials may be used as light emission material for displaying better natural colors. In addition, host/dopant-based light emission materials may be used to increase color purity and improve light emission efficiency through energy transfer. Dopant materials may be classified into fluorescent dopants using organic materials and phosphorescent dopants using metal complex compounds containing heavy atoms, such as Ir and Pt. These phosphorescent materials can theoretically improve the light emission efficiency up to four times compared to fluorescent materials, so attention is focused on phosphorescent host materials as well as phosphorescent dopants.

To date, NPB, BCP, Alq₃, and the like are widely known for a hole injection material layer, a hole transport layer, an electron transport layer, and an electron injection layer, and as for light emission materials, anthracene derivatives have been reported as fluorescent dopant/host materials. Particularly, as for phosphorescent materials having a large advantage in terms of efficiency improvement among light emission materials, metal complex compounds containing Ir, such as Firpic, Ir(ppy)₃, and (acac)Ir(btp)₂, are used as blue, green, and red dopant materials. Until now, CBP shows excellent properties as a phosphorescent host material.

However, conventional organic layer materials have advantages in terms of light emission characteristics, but are not satisfactory in terms of lifespan of organic EL devices due to a low glass transition temperature and very poor thermal stability. Accordingly, there is a need to develop organic layer materials with excellent performance.

### Disclosure of Invention

### Technical Problem

An aspect of the present invention is to provide a novel organic compound, which is excellent in carrier injection and transport ability (especially, electron injection and transport ability), light emission ability, thermal stability, and the like and thus can be used as organic layer materials, such as a light emission layer material, a lifespan improvement layer material, a light emission auxiliary layer material, an electron transport auxiliary layer, and/or an electron transport layer material.

Another aspect of the present invention is to provide an organic electroluminescent device exhibiting a low driving voltage and high light emission efficiency by containing the above-described novel organic compound.

### Solution to Problem

In accordance with an aspect of the present invention, there is provided a compound represented by Chemical Formula 1: 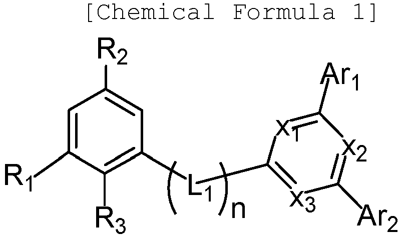 (where, in Chemical Formula 1,
X₁ to X₃ are the same as or different from each other and are each independently N or C(Ar₃), but at least one of X₁ to X₃ is N, where when C (Ar₃) is present in a plurality of numbers, a plurality of Ar₃s are the same as or different from each other,
Ar₁ to Ar₃ are the same or different from each other and are each independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkyl boron group, a C₆ to C₆₀ arylboron group, a C₆ to C₆₀ arylphosphine group, a C₆ to C₆₀ arylphosphine oxide group, and a C₆ to C₆₀ arylamine group, or bind to an adjacent group to form a fused ring;
n is an integer of 0 to 3;
L₁ is a single bond or is selected from the group consisting of a C₆ to C₆₀ arylene group and a heteroarylene group having 5 to 60 nuclear atoms; and
R₁ to R₃ are the same as or different from each other and are each independently selected from the group consisting of a C₆ to C₆₀ aryl group and a heteroaryl group having 5 to 60 nuclear atoms,
provided that at least one of R₁ to R₃ is a substituent represented by any one of Chemical Formulas 2 to 6: 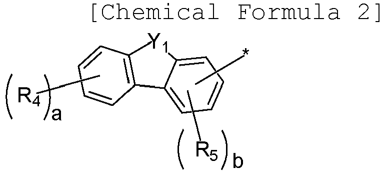 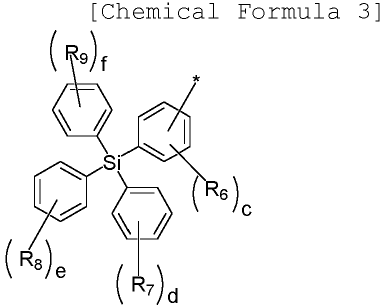 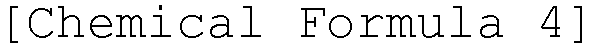 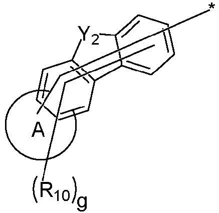 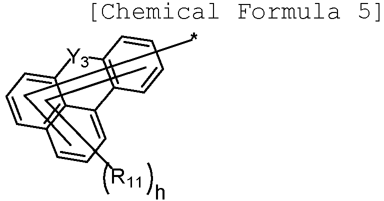 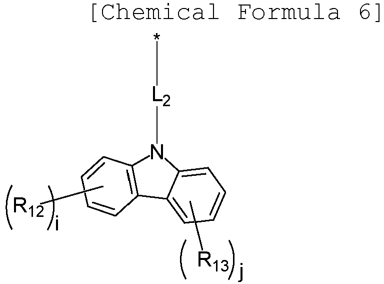
where, in Chemical Formulas 2 to 6,
   Y₁ is selected from the group consisting of C(Ar₄)(Ar₅), N(Ar₆), O, and S;
   Y₂ and Y₃ are the same as or different from each other and are each independently O or S;
   ring A is a benzene ring;
   L₂ is a single bond or is selected from the group consisting of a C₆ to C₆₀ arylene group and a heteroarylene group having 5 to 60 nuclear atoms;
   a, c, i, and j are each an integer of 0 to 4;
   b is an integer of 0 to 3;
   d, e, and f are each an integer of 0 to 5;
   g is an integer of 0 to 8;
   h is an integer of 0 to 9;
   Ar₄ to Ar₆ and R₄ to R₁₃ are the same as or different from each other and are each independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₂ to C₄₀ alkylsilyl group, a C₆ to C₆₀ arylsilyl group, a C₂ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and
   the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, arylamine group, and fused ring of Ar₂ to Ar₃, the arylene group and heteroarylene group of L₁, the aryl group and heteroaryl group of R₁ to R₃, and the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, and arylamine of Ar₄ to Ar₆ and R₄ to R₁₃ are each independently substituted or unsubstituted with at least one selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₂ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkyl boron group, a C₆ to C₆₀ arylboron group, a C₆ to C₆₀ arylphosphine group, a C₆ to C₆₀ arylphosphine oxide group, and a C₆ to C₆₀ arylamine group, and when the substituent is present in a plurality of numbers, a plurality of substituents are the same as or different from each other).

In accordance with another aspect of the present invention, there is provided an organic electroluminescent device, including: an anode; a cathode; and one or more organic layers interposed between the anode and the cathode, wherein at least one of the one or more organic layers contains the above-described compound.

According to an embodiment, the organic layer containing the compound may be at least one selected from the group consisting of a light emission layer, an electron transport layer, and an electron transport auxiliary layer.

### Advantageous Effects of Invention

The compounds of the present invention have excellent electron injection and transport ability, light emission ability, thermal stability, and electrochemical stability and thus can be used as an organic layer material of an organic electroluminescent device. Especially, when the compounds of the present invention are used as any one of a host material, an electron transport layer material, and an electron transport auxiliary layer material, electroluminescent devices with a low deriving voltage and high efficiency and lifespan characteristics compared to conventional materials can be manufactured, and furthermore, full color display panels can with improved performance and lifespan also be manufactured.

The advantageous effects according to the present invention are not limited by the contents exemplified above, and a wider variety of advantageous effects are included herein.

### Brief Description of Drawings

FIG. 1 is a cross-sectional view schematically showing an organic electroluminescent device according to a first embodiment of the present invention.
FIG. 2 is a cross-sectional view schematically showing an organic electroluminescent device according to a second embodiment of the present invention.
FIG. 3 is a cross-sectional view schematically showing an organic electroluminescent device according to a third embodiment of the present invention.

### <Explanation of reference numerals>

100: anode, 200: cathode
300: organic layer, 310: hole injection layer
320: hole transport layer, 330: light emission layer
340: electron transport layer, 350: electron injection layer
360: electron transport auxiliary layer

### Best Mode for Carrying out the Invention

Hereinafter, the present invention will be described in detail.

### <Novel Compound>

A compound according to the present invention is represented by Chemical Formula 1, which includes a basic structure in which a nitrogen-containing heteroaromatic ring is bonded directly or via a linker group (e.g., a phenylene group, etc.) to the 3-carbon position of a benzene moiety and various substituents (e.g., an aryl group, a heteroaryl group, etc.) are attached to the 1-, 4-, and 5-carbon positions of the benzene moiety, respectively, wherein a specific substituent (e.g., a monovalent fluorene group, a monovalent dibenzofuran group, a monovalent dibenzothiophene group, a monovalent carbazole group, a monovalent tetraphenylsilane group, a monovalent naphthobenzofuran group, a monovalent naphthobenzothiophen group, a monovalent benzoxanthene group, a monovalent benzothioxanthene group, etc.) is attached to at least one of the 1-, 4-, and 5-carbon positions of the benzene moiety. Such a compound of Chemical Formula 1 according to the present invention is excellent in terms of thermal stability, carrier transport ability (e.g., electron transport ability), light emission ability, and the like and thus may be used as an electron transport layer material or electron transport auxiliary layer material capable of improving high efficiency, long lifespan, and driving voltage characteristics of organic electroluminescent devices. Particularly, numbering of carbon atoms of the benzene moiety is as follows.

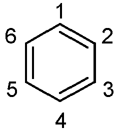

Specifically, in the compound represented by Chemical Formula 1, various substituents (e.g., an aryl group, a heteroaryl group, etc.) are attached to the 1-, 4-, and 5-carbon positions of the benzene moiety, respectively, wherein a substituent represented by any one of Chemical Formulas 2 to 6 is introduced to at least any one of the 1-, 4-, and 5-carbon positions of the benzene moiety, so that the compound of Chemical Formula 1 has an increase in structural steric hindrance, thereby improving the efficiency of organic electroluminescent devices. Especially, the substituent represented by any one of Chemical Formulas 2 to 6 can raise the glass transition temperature (Tg) of the compound to improve thermal stability of the compound. Furthermore, a nitrogen-containing heteroaromatic ring (e.g., an azine group), which is an electron withdrawing group (EWG) with excellent electron transport ability, is bonded directly or via a linker group to the 3-carbon position of the benzene moiety, to increase the electron movement rate, thereby enhancing electron transport and injection properties of the compound.

Furthermore, the compound represented by Chemical Formula 1 has a high triplet energy and thus can prevent excitons generated from a light emission layer from diffusing (movement) into an electron transport layer or a hole transport layer adjacent to the light emission layer. Therefore, the number of excitons contributing to light emission in the light emission layer is increased to improve the light emission efficiency of devices, and the lifespan of devices can be efficiently increased through improved durability and stability of devices. Therefore, when the compound of the present invention is used to form a separate organic layer (hereinafter, referred to as "electron transport auxiliary layer") disposed between a light emission layer and an electron transport layer, the diffusion of excitons is prevented by the compound, and thus the number of excitons substantially contributing to light emission in the light emission layer is increased to improve the light emission efficiency of devices, unlike conventional organic electroluminescent devices not including the electron transport auxiliary layer. In addition, when the compound of Chemical Formula 1 is applied as a material for an electron transport layer or electron transport auxiliary layer of an organic electroluminescent device, electrons can be smoothly transported from a cathode (or electron injection layer) to the light emission layer, thereby lowering the driving voltage of devices and realizing high efficiency and long lifespan characteristics.

As described above, the compound represented by Chemical Formula 1 according to the present invention is excellent in term of carrier transport ability (e.g., electron transport ability), light emission ability, thermal stability, electrochemical stability, and the like. Therefore, the compound represented by Chemical Formula 1 of the present invention may be used as an organic layer material of an organic electroluminescent device, preferably a light emission layer material (blue, green and/or red phosphorescent host materials), an electron transport layer/injection layer material, a hole transport layer/injection layer material, a light emission auxiliary layer material, or a lifespan improvement layer material, and more preferably an electron transport layer material or electron transport auxiliary layer material. Organic electroluminescent devices containing the compound of the present invention can attain significantly improved performance and lifespan characteristics, and consequently maximizing the performance of full-color organic light-emitting panels.

In the compound represented by Chemical Formula 1 according to the present invention, X₁ to X₃ are the same as or different from each other and are each independently N or C(Ar₃), provided that at least one of X₁ to X₃ is N. Particularly, when C(Ar₃) is present in a plurality of numbers, a plurality of Ar₃s are the same as or different from each other. According to one embodiment, two of X₁ to X₃ may be N and the other may be C(Ar₃). According to another embodiment, X₁ to X₃ may each be N. Such X₁- to X₃-containing rings each are a type of nitrogen (N)-containing heteroaromatic ring and are a monocyclic heteroaryl group (e.g., an azine group) containing at least one nitrogen atom. As such, the compound of the present invention shows superb electron absorption properties and thus is advantageous in electron injection and transport, by including a heteroaromatic ring containing at least one nitrogen atom.

Additionally, Ar₁ to Ar₃ are the same as or different from each other and may each be independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or may bind to an adjacent group (e.g., Ar₂-Ar₃, Ar₂-Ar₃, or Ar₁-Ar₂) to form a fused ring; specifically, may each be independently selected from the group consisting of deuterium, a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, or may bind to an adjacent group (e.g., Ar₁-Ar₃, Ar₂-Ar₃, or Ar₁-Ar₂) to form a fused ring; and more specifically, may each be independently selected from the group consisting of deuterium, a cyano group, a C₁ to C₂₀ alkyl group, a C₃ to C₂₀ cycloalkyl group, a heterocycloalkyl group having 3 to 20 nuclear atoms, a C₆ to C₃₀ aryl group, and a heteroaryl group having 5 to 30 nuclear atoms, or may bind to an adjacent group (e.g., Ar₁-Ar₃, Ar₂-Ar₃, or Ar₁-Ar₂) to form a fused ring. Particularly, the fused ring may be at least one selected from the group consisting of a C₃ to C₆₀ fused aliphatic ring (specifically, a C₃ to C₃₀ fused aliphatic ring), a C₆ to C₆₀ fused aromatic ring (specifically, a C₆ to C₃₀ fused aromatic ring), a 5- to 60-membered fused heteroaromatic ring containing at least one heteroatom (e.g., N, O, S, Se, etc.) (specifically, a 5- to 30-membered fused heteroaromatic ring containing at least one heteroatom), and a C₃ to C₆₀ spiro ring, and a combination thereof.

Particularly, the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, and arylamine group of Ar₁ to Ar₃ and the fused ring may each be independently substituted or unsubstituted with at least one substituent selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and specifically, may each be independently substituted or unsubstituted with at least one substituent selected from the group consisting of deuterium, a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, and a heteroaryl group having 5 to 60 nuclear atoms. Particularly, when the substituent is present in a plurality of numbers, a plurality of substituents are the same as or different from each other.

According to one embodiment, Ar₁ and Ar₂ may be the same as or different from each other and may each be independently selected from the group consisting of Substituents S2-1 to S2-15. 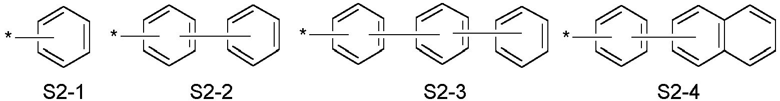 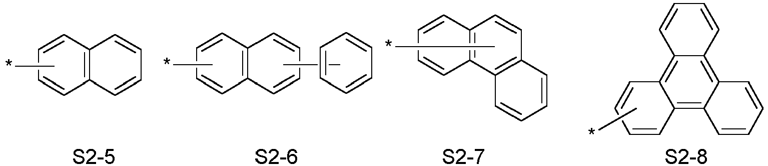 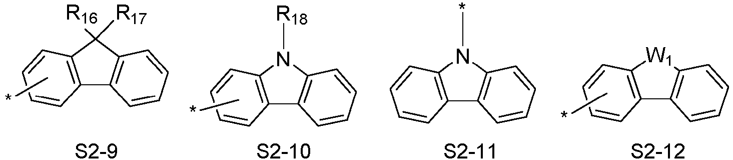 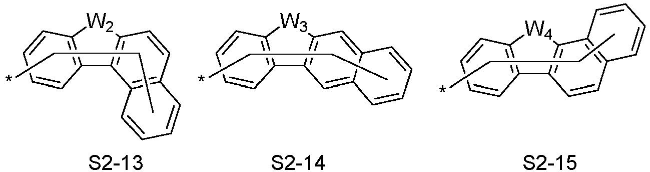 where, in Substituents S2-1 to S2-15,
the mark * is a site linked to chemical formula 1,
W₁ to W₄ are each O or S;
R₁₆ to R₁₈ are the same as or different from each other and are each independently selected from the group consisting of hydrogen, deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and specifically, may each be independently selected from the group consisting of hydrogen, deuterium, a halogen, a cyano group, a C₁ to C₄₀ alkyl group, a C₆ to C₆₀ aryl group, and a heteroaryl group having 5 to 60 nuclear atoms.

According to the above-described X₁ to X₃ and Ar₁ to Ar₃, X₁- to X₃-containing ring 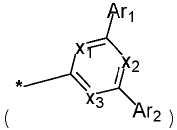 in the compound of Chemical Formula 1 may be any one of Structural Formulas Az1-1 to Az1-7, but is not limited thereto. 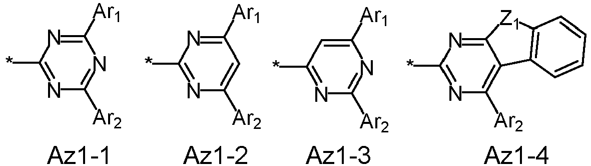 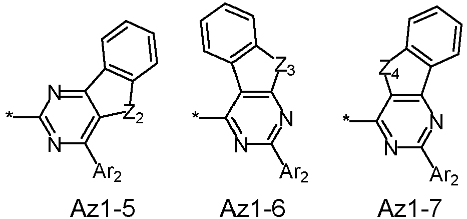 where, in Structural Formulas Az1-1 to Az1-7,
Ar₁ and Ar₂ are each as defined in Chemical Formula 1 and, specifically, may be the same as or different from each other and may each independently selected from the group consisting of a C₁ to C₄₀ alkyl group, a C₃ to C₄₀ cycloalkyl group, a heterocycloalkyl group having 3 to 40 nuclear atoms, a C₆ to C₆₀ aryl group, and a heteroaryl group having 5 to 60 nuclear atoms;
Z₁ to Z₄ may each be selected from the group consisting of C(Ar₇)(Ar₈), N(Ar₉), O, and S;
Ar₇ to Ar₉ may be the same as or different from each other and may each be independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C6 to C60 arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and
the alkyl group, cycloalkyl group, heterocycloalkyl group, aryl group, and heteroaryl group of Ar₁ and Ar₂ and the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, and arylamine group of Ar₇ to Ar₉ may each be independently substituted or unsubstituted with at least one selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and when the substituent is present in a plurality of numbers, a plurality of substituents are the same as or different from each other.

In the compound represented by Chemical Formula 1 according to the present invention, n may be an integer of 0 to 3 and, specifically, an integer of 0 to 2.

Particularly, when n is 0, L₁ means a direct bond (single bond). Meanwhile, when n is an integer of 1 to 3, L₁ is a divalent linker, and may be selected from the group consisting of C₆ to C₃₀ arylene group and a heteroarylene group of 5 to 30 nuclear atoms, and specifically, may be selected from the group consisting of C₆ to C₁₈ arylene group and a heteroarylene group of 5 to 18 nuclear atoms. Particularly, a plurality of L₁s may be the same as or different from each other.

Particularly, the arylene group and heteroarylene group of L₁ may each be independently substituted or unsubstituted with at least one substituent selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and specifically, may each be independently substituted or unsubstituted with at least one substituent selected from the group consisting of deuterium, a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, and a heteroaryl group having 5 to 60 nuclear atoms. Particularly, when the substituent is present in a plurality of numbers, a plurality of substituents are the same as or different from each other.

According to one embodiment, L₁ may be a direct bond, or may be selected from the group consisting of a phenylene group, a biphenylene group, a terphenylene group, and a naphthalene group. Particularly, hydrogen of the phenylene group, biphenylene group, terphenylene group, and naphthalene group may be substituted or unsubstituted with at least one selected from the group consisting of deuterium (D), a halogen, a cyano group, a nitro group, a C₁ to C₁₂ alkyl group, a C₆ to C₁₀ aryl group, and a heteroaryl group having 5 to 10 nuclear atoms.

According to another embodiment, L₁ may be a direct bond or may be any one of linker groups L-1 to L-6.

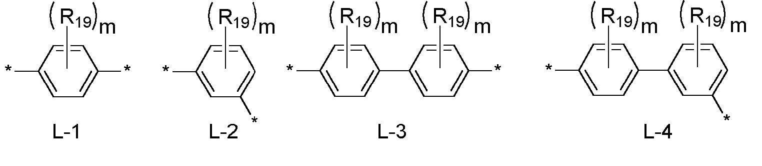

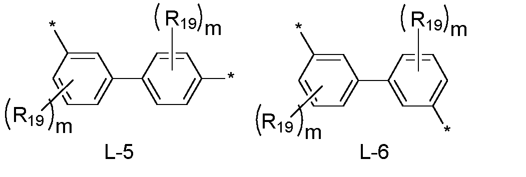

In Linker Groups L-1 to L-6,
the mark * is a site linked to Chemical Formula 1,
a plurality of ms are the same as or different from each other,
m is an integer of 0 to 4,
a plurality of R₁₉s are the same as or different from each other,
R₁₉ may be selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and specifically, may be selected from the group consisting of deuterium (D), a halogen, a cyano group, a nitro group, a C₁ to C₁₂ alkyl group, a C₆ to C₁₀ aryl group, and a heteroaryl group having 5 to 10 nuclear atoms.

In the compound represented by Chemical Formula 1 according to the present invention, R₃ to R₃ are the same as or different from each other and may each be independently selected from the group consisting of a C₆ to C₆₀ aryl group and a heteroaryl group of 5 to 60 nuclear atoms, provided that at least any one of R₁ to R₃ is a substituent represented by any one of Chemical Formulas 2 to 6.

According to one embodiment, any one of R₁ to R₃ is a substituent represented by any one of Chemical Formulas 2 to 6 and the others are the same as or different from each other and are each independently a C₆ to C₆₀ aryl group.

According to another embodiment, R₁ may be a substituent represented by any one of Chemical Formulas 2 to 6, R₂ and R₃ may be the same as or different from each other and may be independently a C₆ to C₁₈ aryl group.

The substituent represented by any one of Chemical Formulas 2 to 6 may be selected from the group consisting of Substituents S1-1 to S1-16, but is not limited thereto.

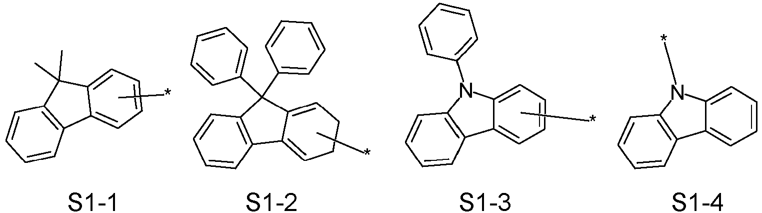

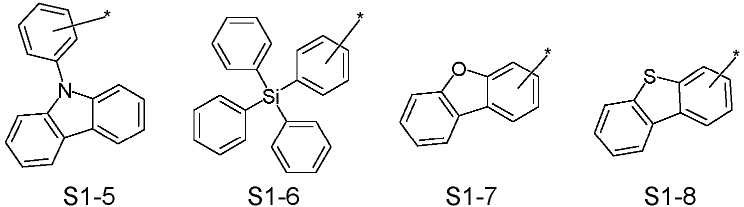

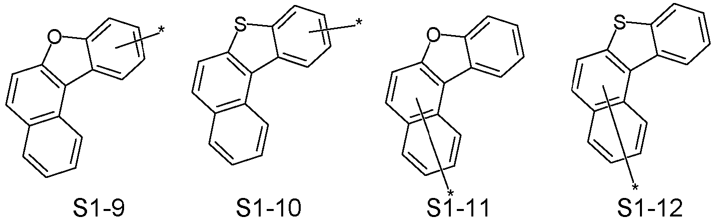

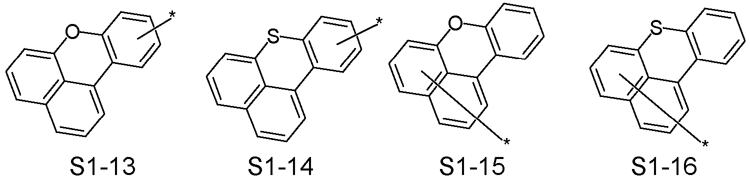

In Substituents S1-1 to S1-16,
the mark * is a site linked to Chemical Formula 1.

The hydrogen of the above-described Substituents S1-1 to S1-16 may be substituted or unsubstituted with at least one selected from the group consisting of deuterium (D), a halogen, a cyano group, a nitro group, a C₁ to C₁₂ alkyl group, a C₆ to C₁₀ aryl group, and a heteroaryl group having 5 to 10 nuclear atoms.

The compound represented by Chemical Formula 1 may be a compound represented by any one of Chemical Formulas 7 to 11 depending on R₁, but is not limited thereto. 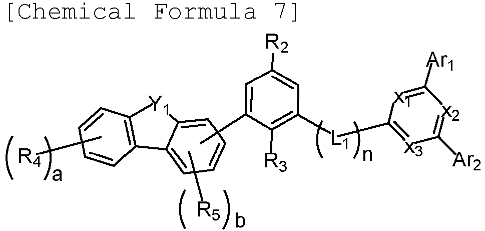 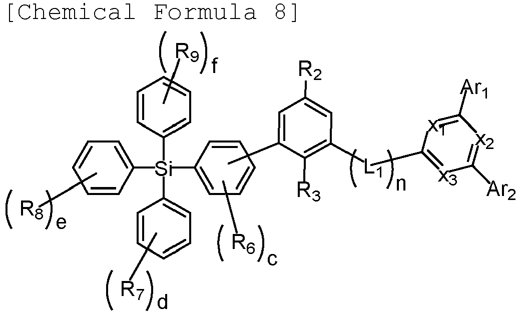 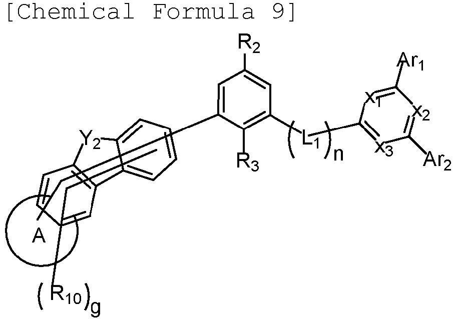 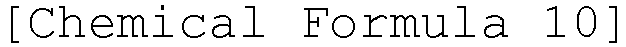 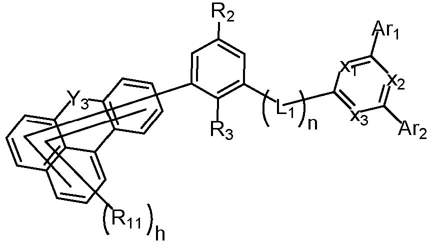 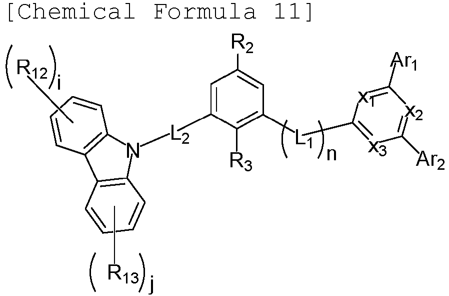 where, in Chemical Formulas 7 to 11,
R₂ to R₁₃, a, b, c, d, e, f, g, h, i, j, X₁ to X₃, Ar₁, Ar₂, n, L₁, L₂, Y₁ to Y₃, and ring A are each as defined in Chemical Formula 1.

In addition, the compound represented by Chemical Formula 1 is a compound represented by any one of Chemical Formulas 12 to 16 depending on to R₃: 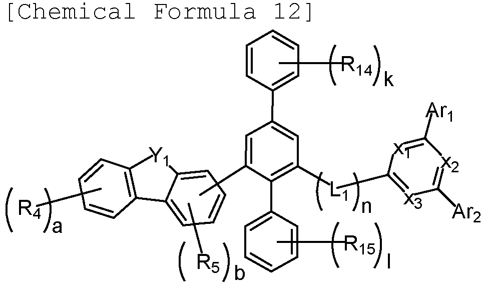 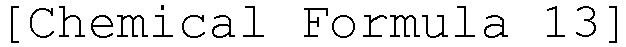 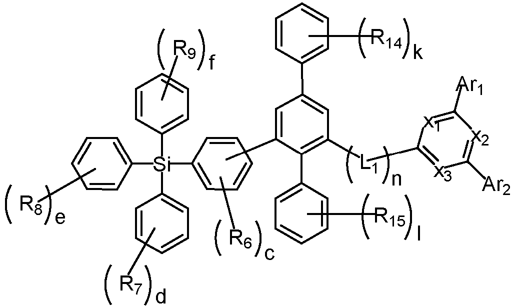 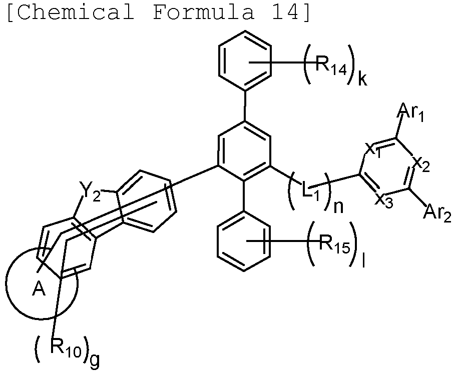 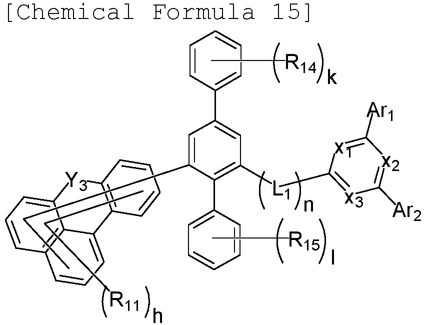 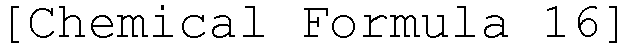 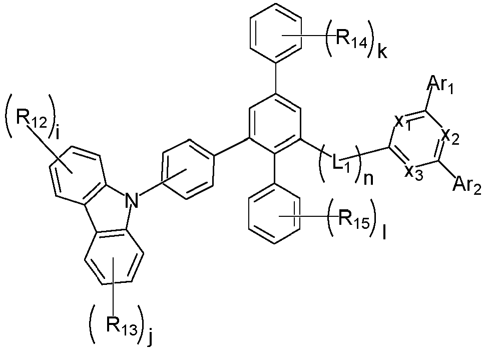 where, in Chemical Formulas 12 to 16,
R₂ to R₁₃, a, b, c, d, e, f, g, h, i, j, X₁ to X₃, Ar₁, Ar₂, n, L₁, Y₁ to Y₃, and ring A are each as defined in Chemical Formula 1;
k and l are each an integer of 0 to 5;
a plurality of R₁₄s are the same as or different from each other;
a plurality of R₁₅s are the same as or different from each other;
R₁₄ to R₁₅ are the same as or different from each other and are each independently selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and specifically, may each be independently selected from the group consisting of deuterium, a cyano group, a C₃ to C₄₀ alkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, and a C₆ to C₆₀ arylamine group.

The compound represented by Chemical Formula 1 may be a compound represented by any one of Chemical Formulas 17 to 21 depending on R₁ to R₃ and L₁, but is not limited thereto. 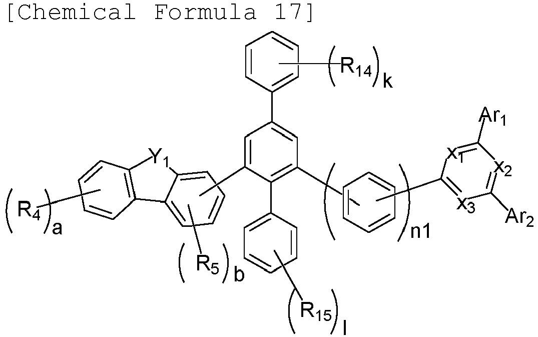 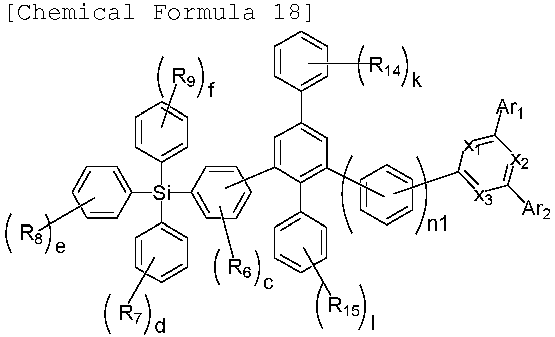 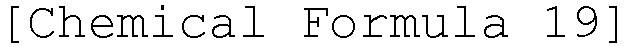 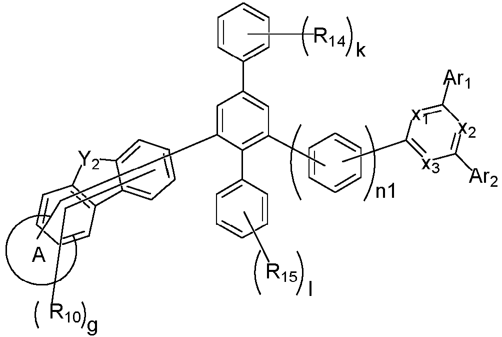 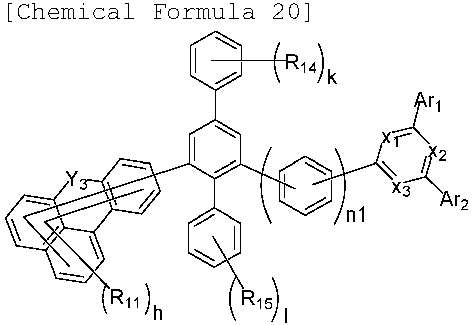 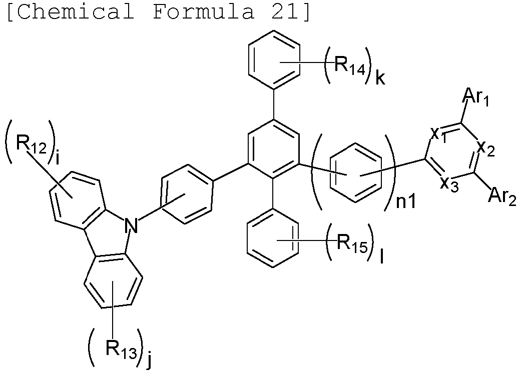 where, in Chemical Formulas 17 to 21,
R₂ to R₁₃, a, b, c, d, e, f, g, h, i, j, X₁ to X₃, Ar₁, Ar₂, Y₁ to Y₃, and ring A are each as defined in Chemical Formula 1;
n1 is an integer of 0 to 2;
k and l are each an integer of 0 to 5;
a plurality of R₁₄s are the same as or different from each other;
a plurality of R₁₅s are the same as or different from each other;
R₁₄ to R₁₅ may be the same as or different from each other and may each be independently selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group and, specifically, the group consisting of deuterium, a cyano group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, and a C₆ to C₆₀ arylamine group.

The compound represented by Chemical Formula 1 according to the present invention described above may be further specified as compounds Inv 1 to Inv 480 below, but is not limited thereto.

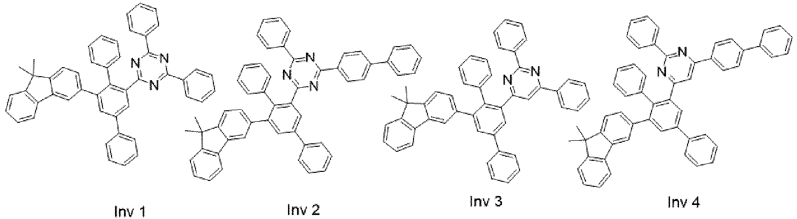

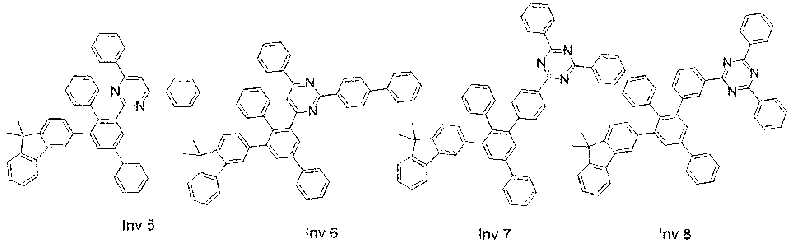

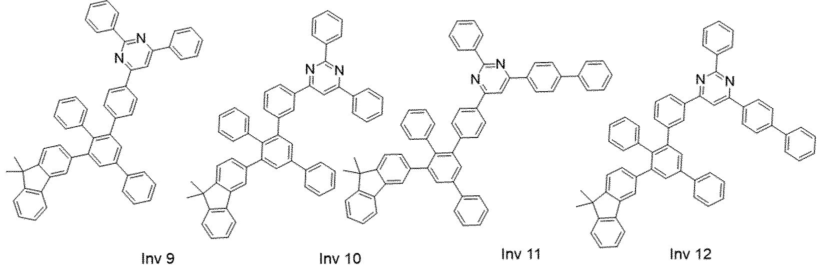

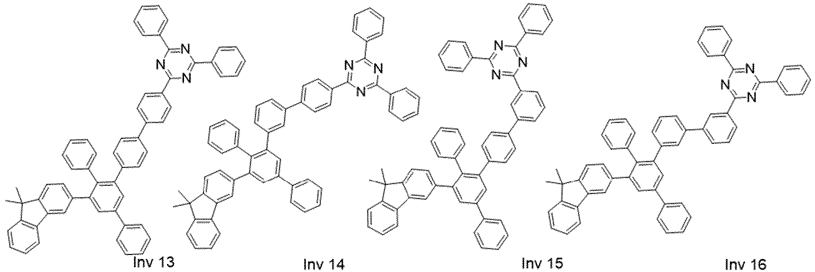

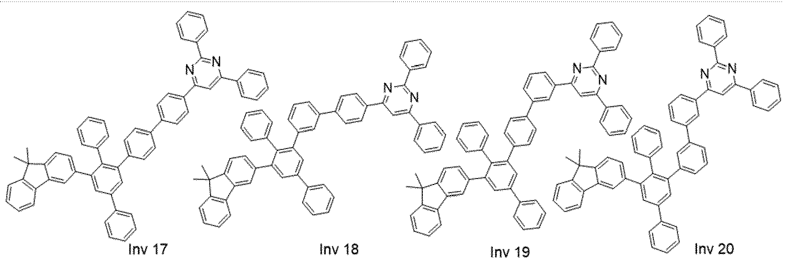

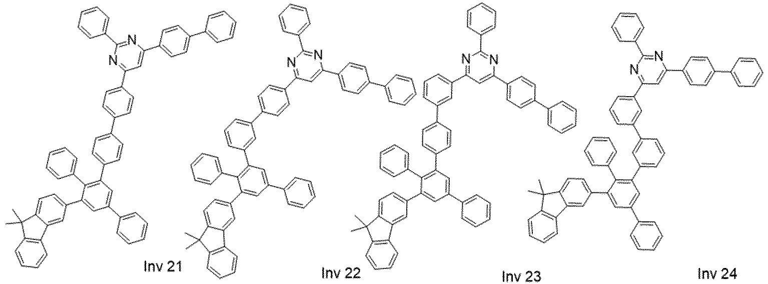

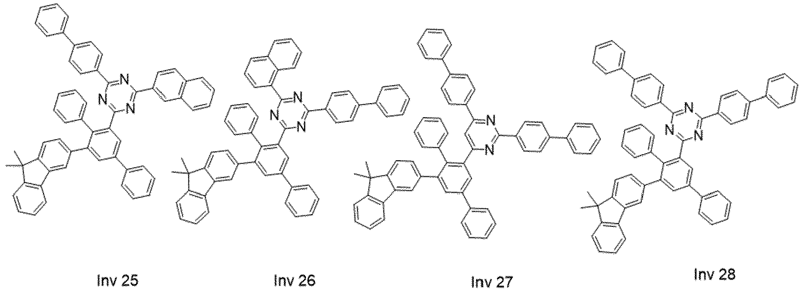

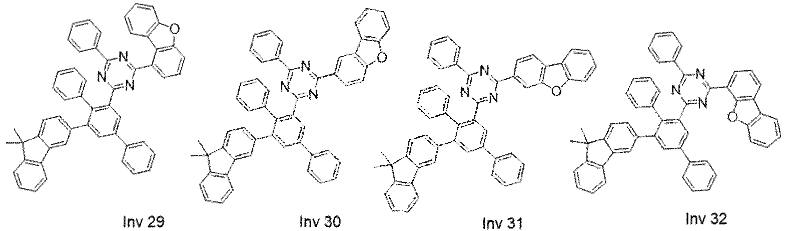

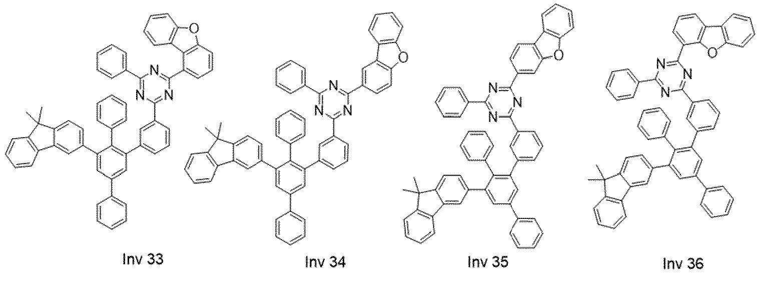

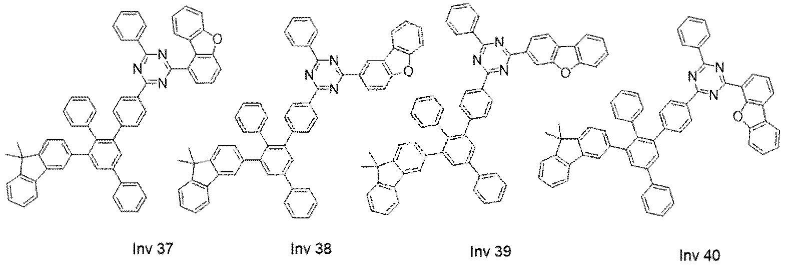

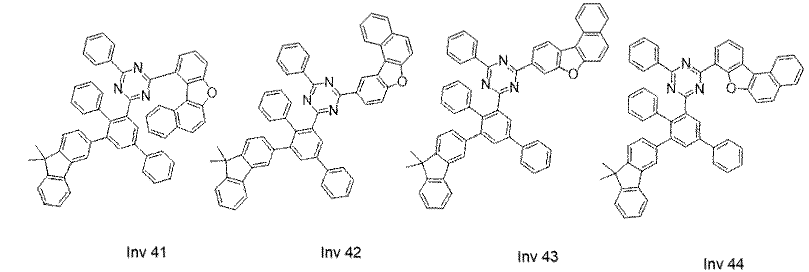

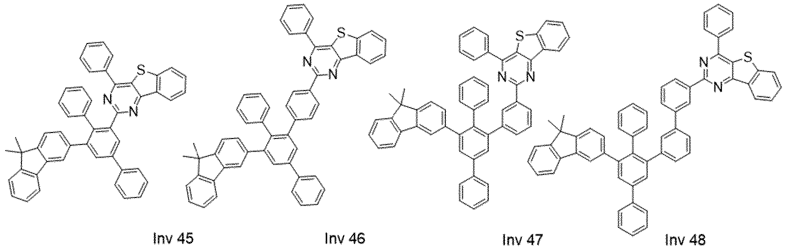

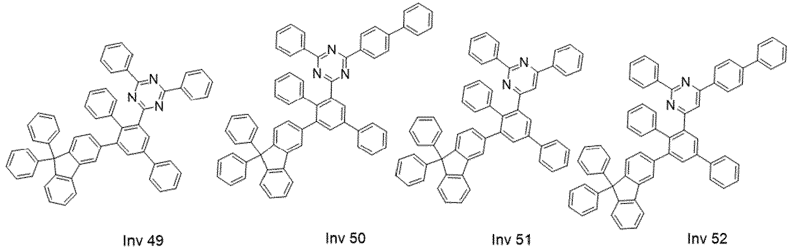

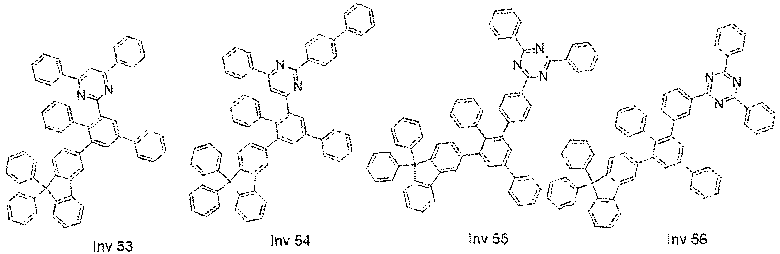

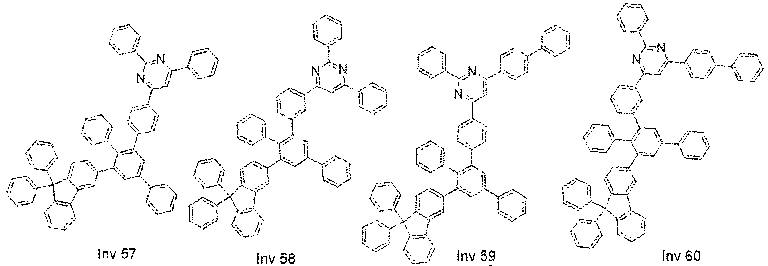

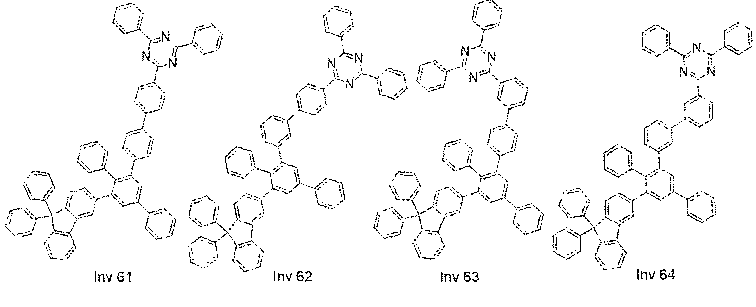

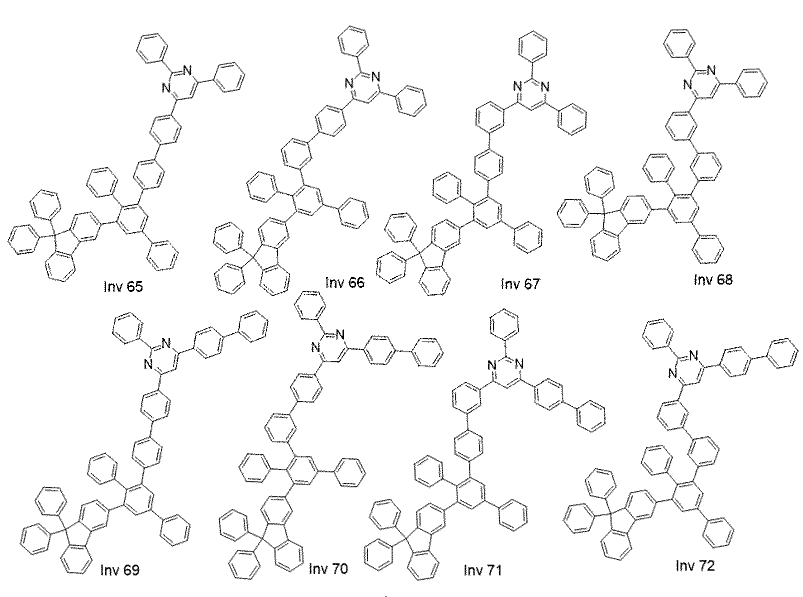

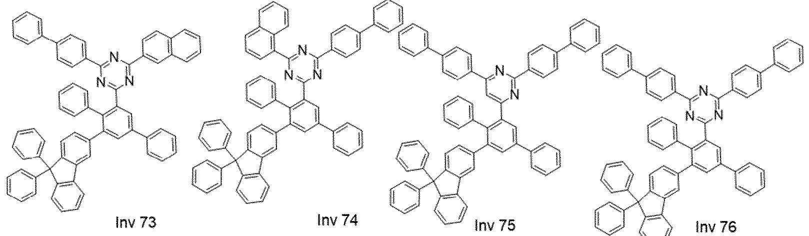

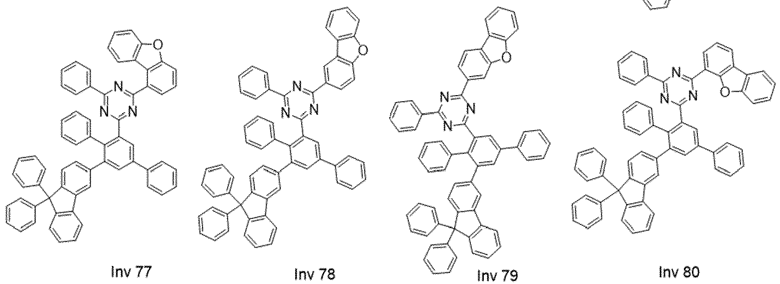

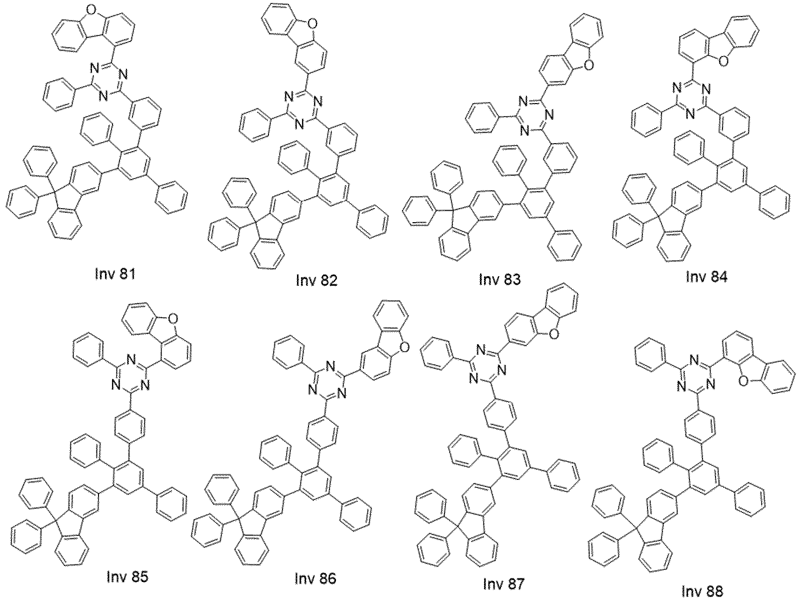

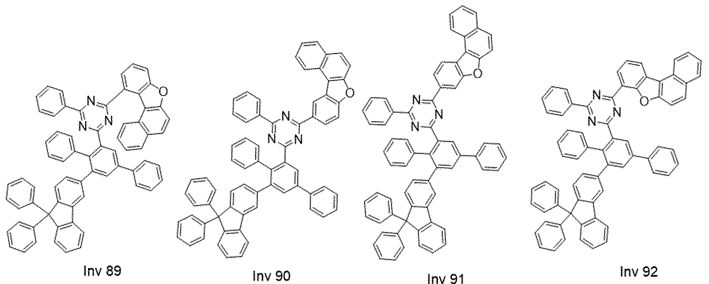

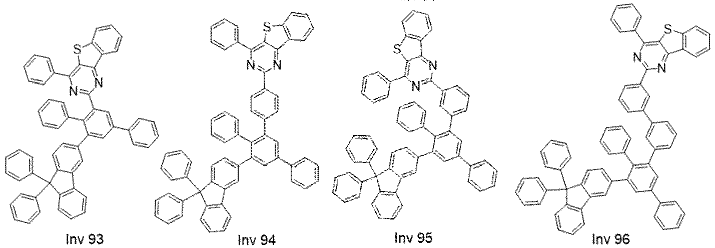

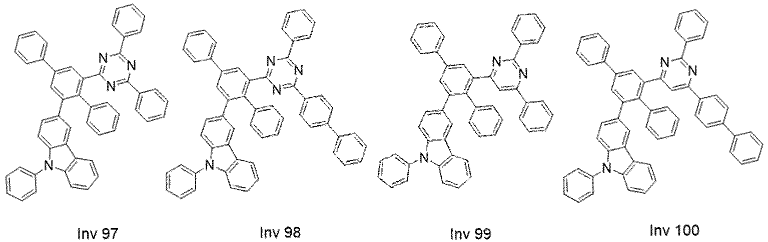

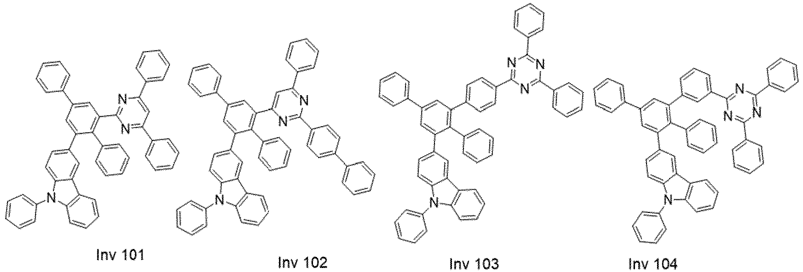

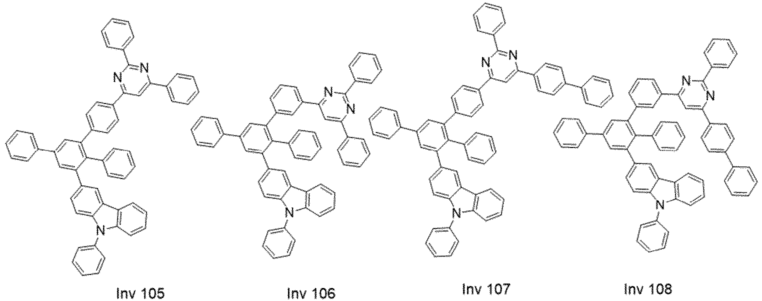

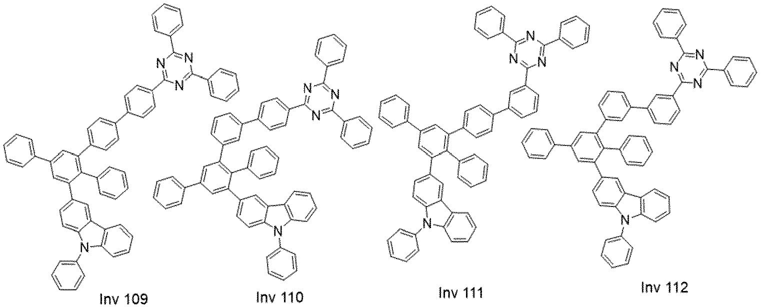

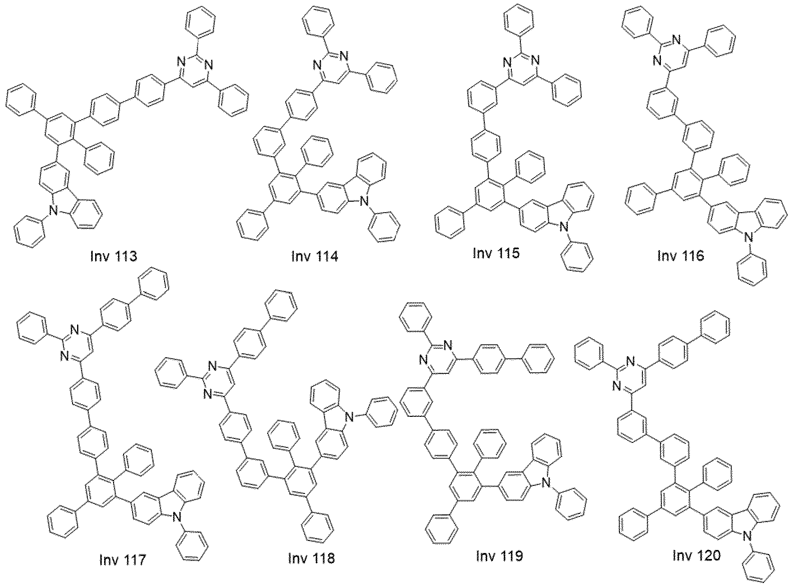

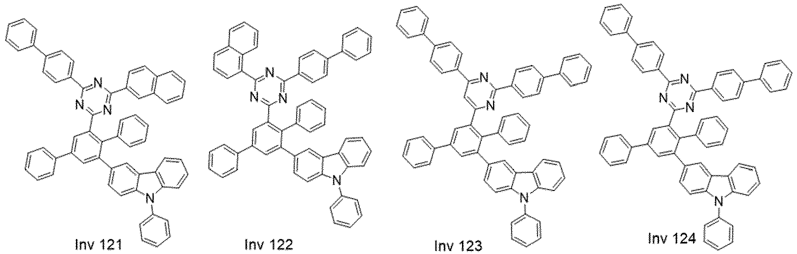

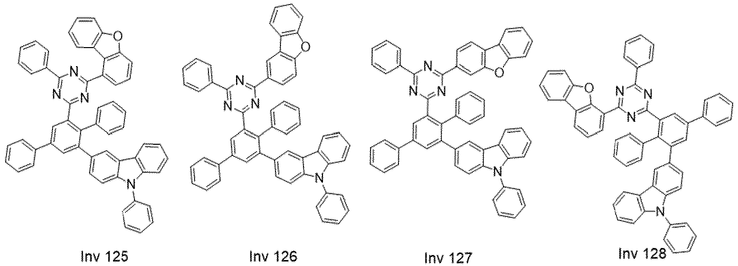

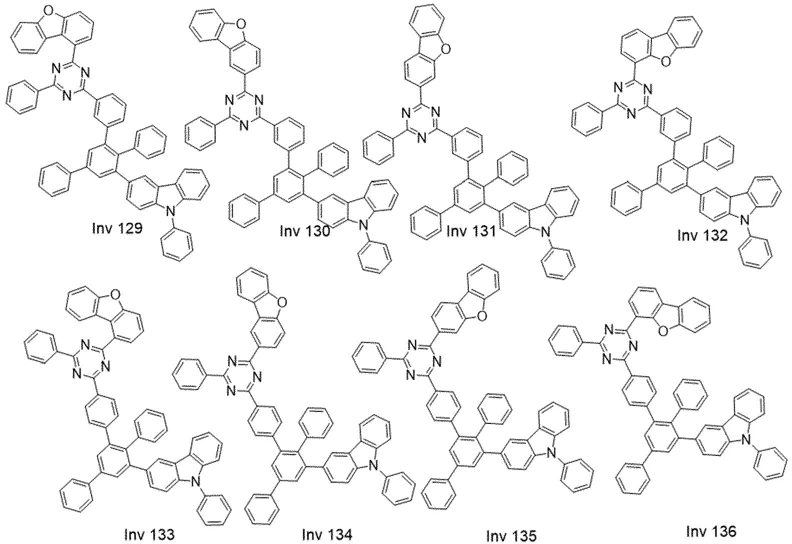

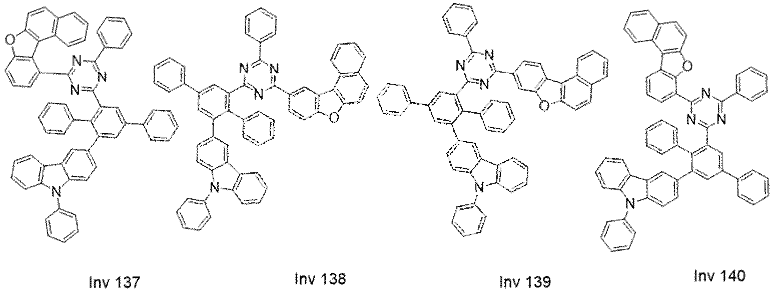

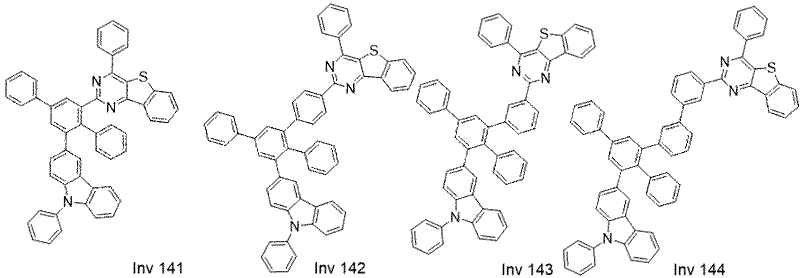

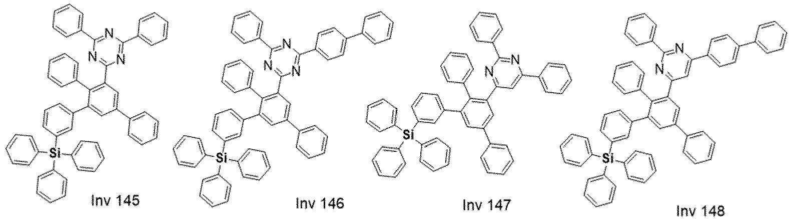

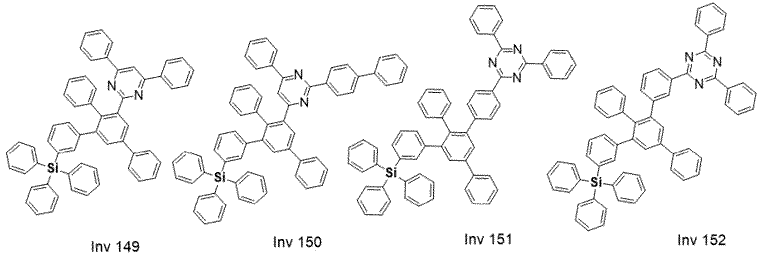

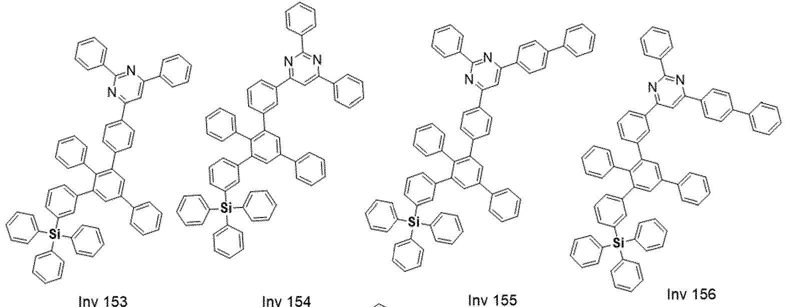

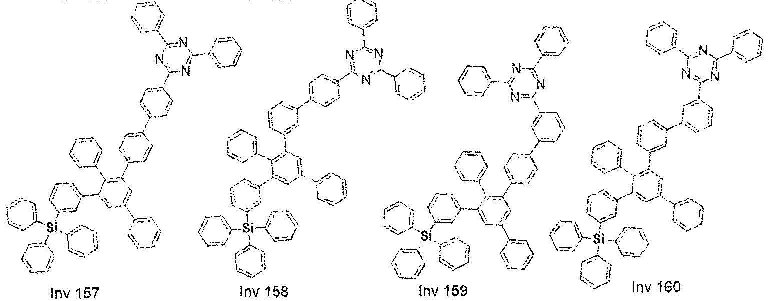

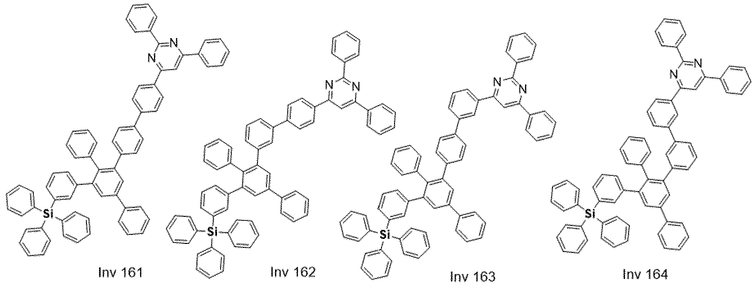

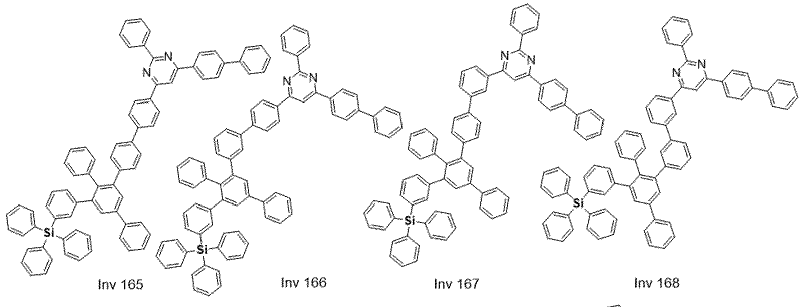

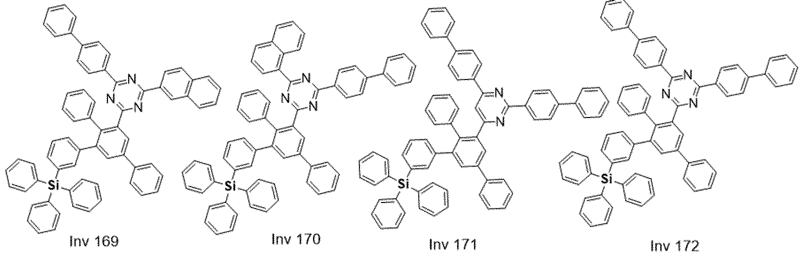

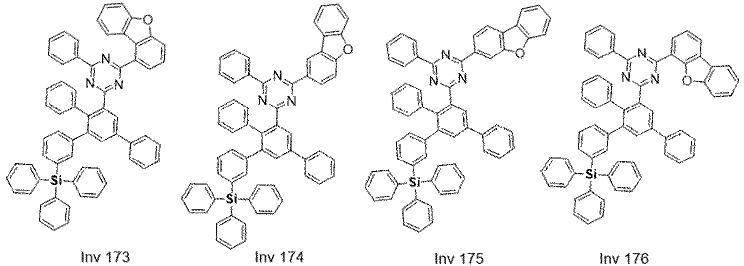

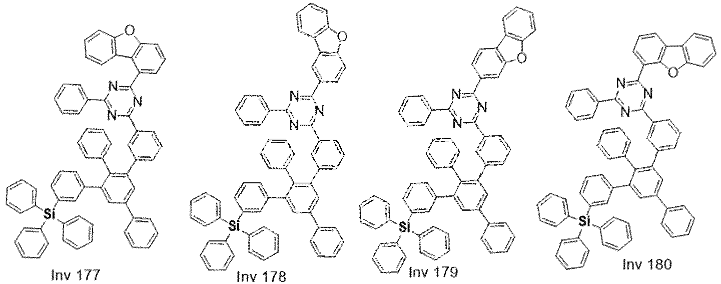

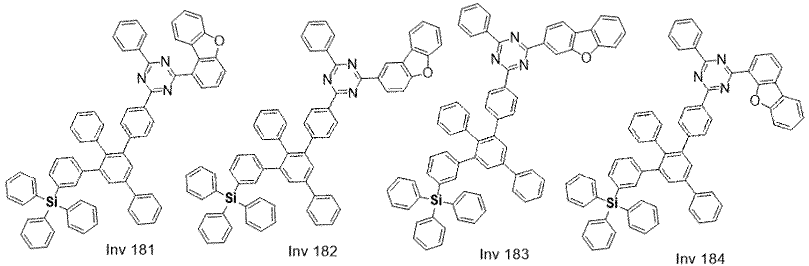

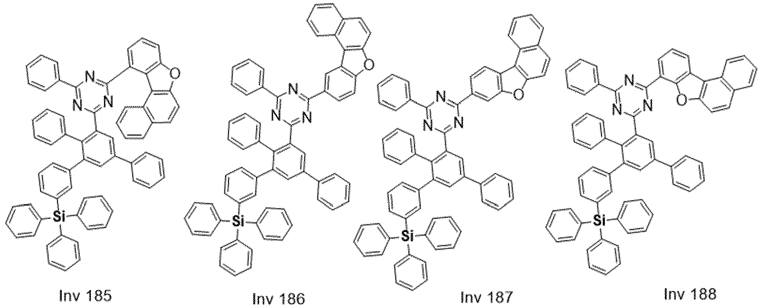

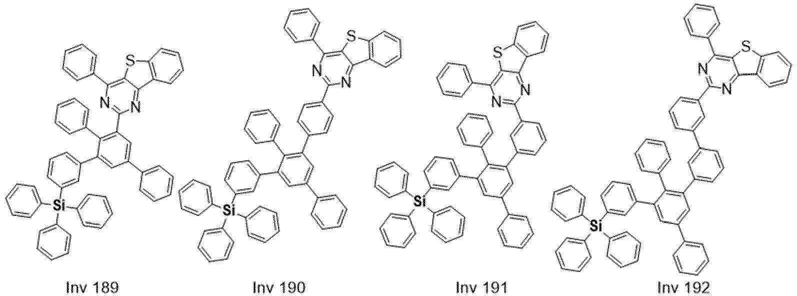

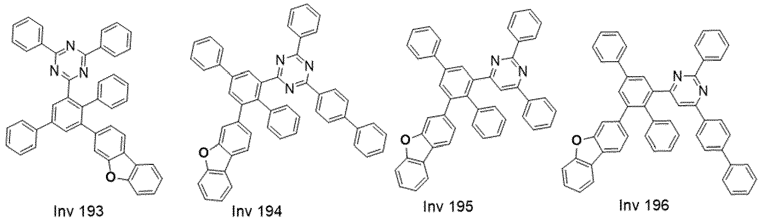

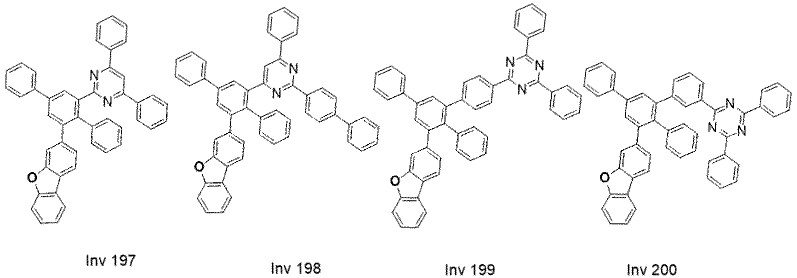

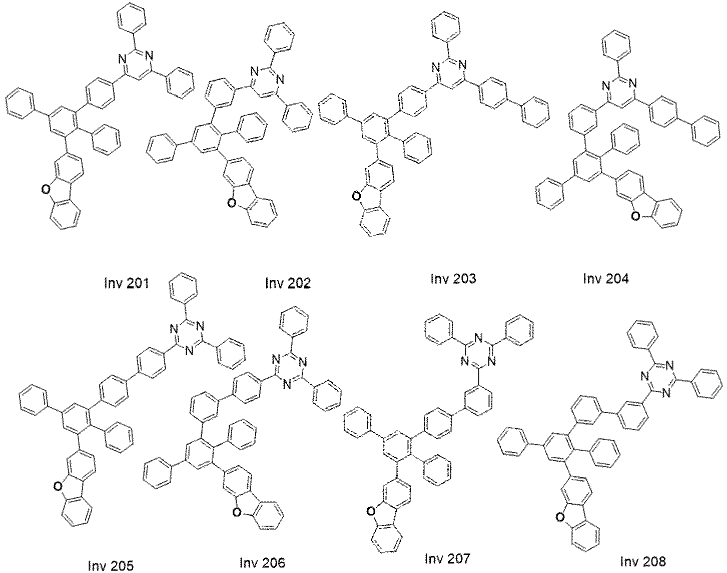

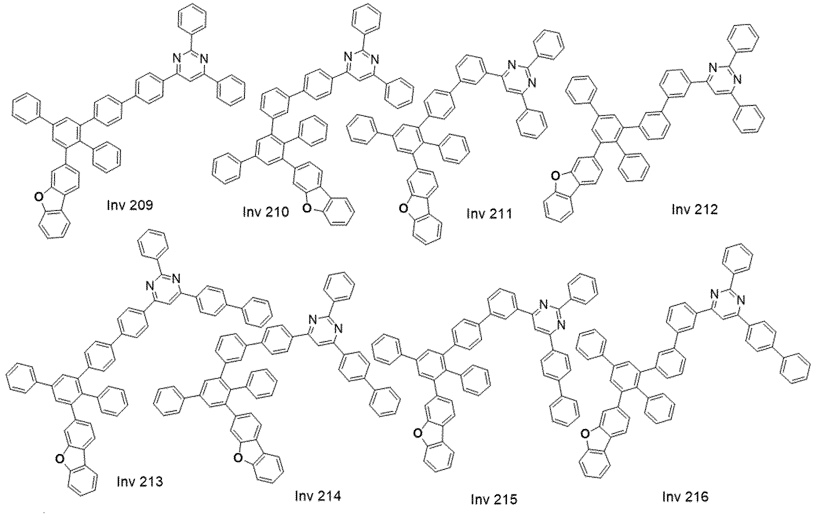

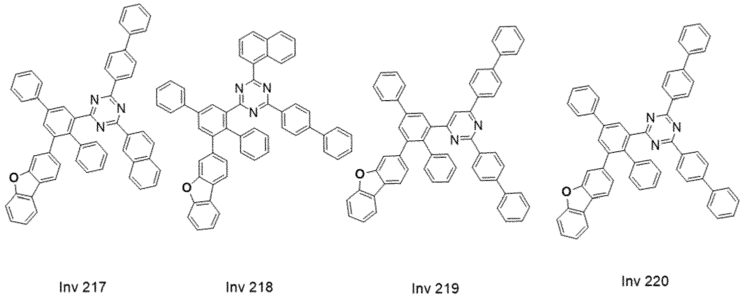

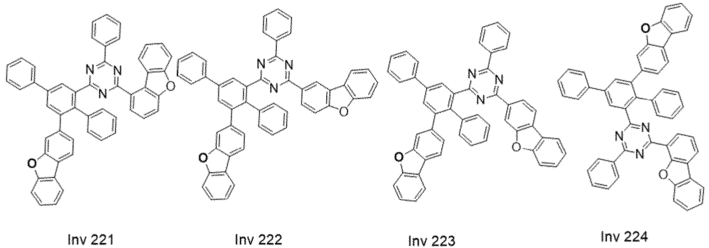

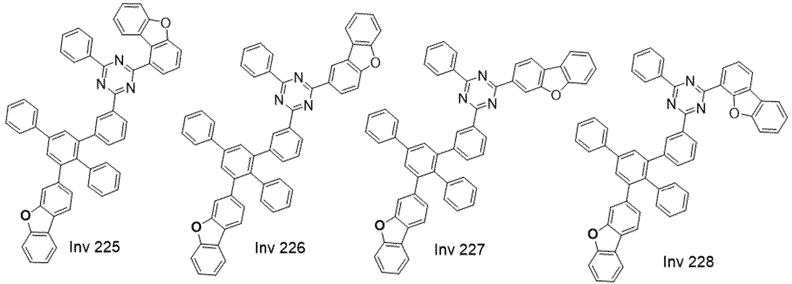

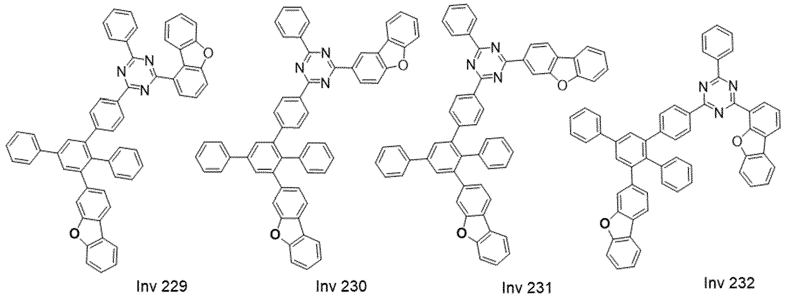

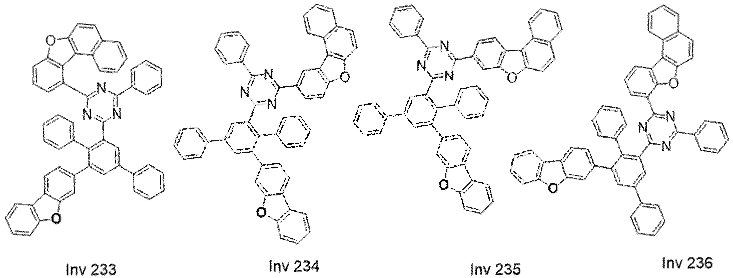

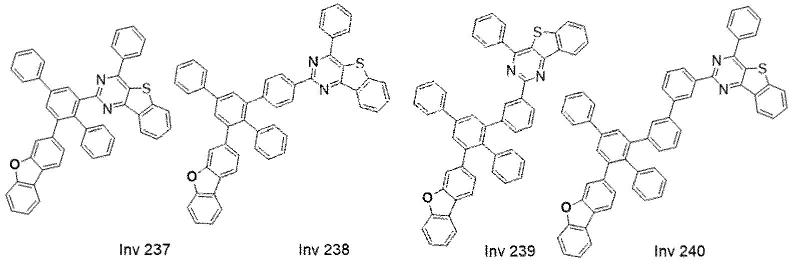

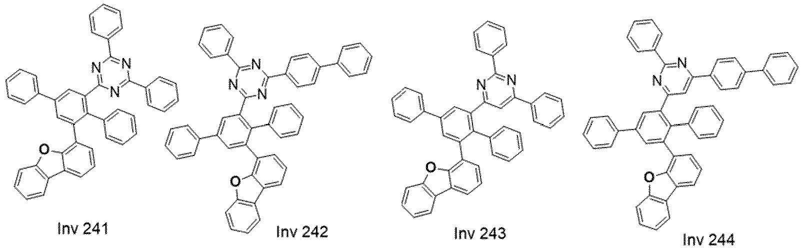

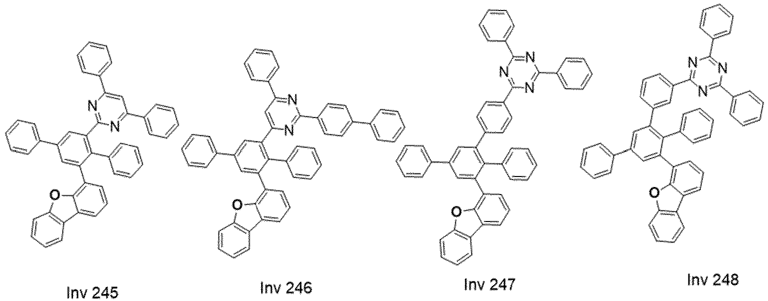

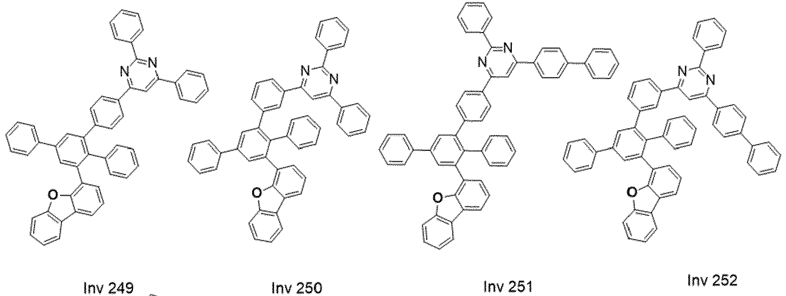

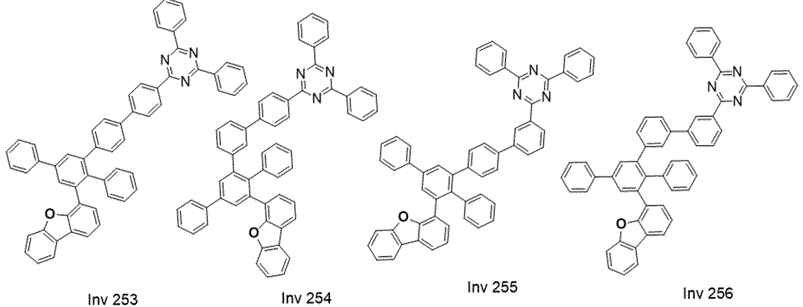

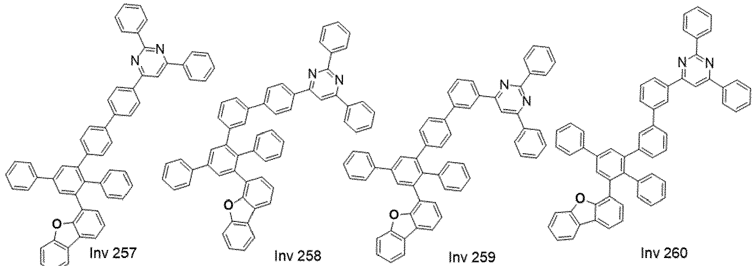

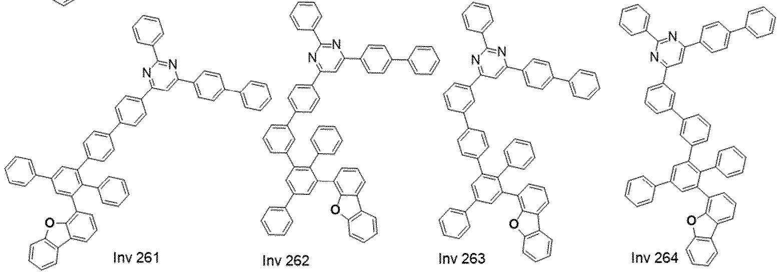

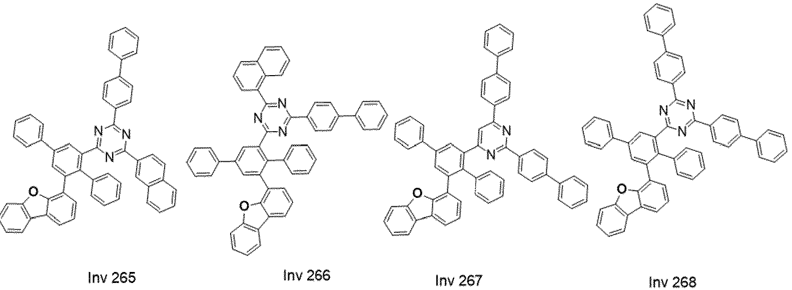

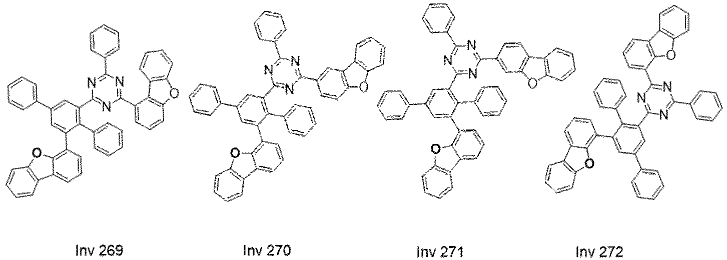

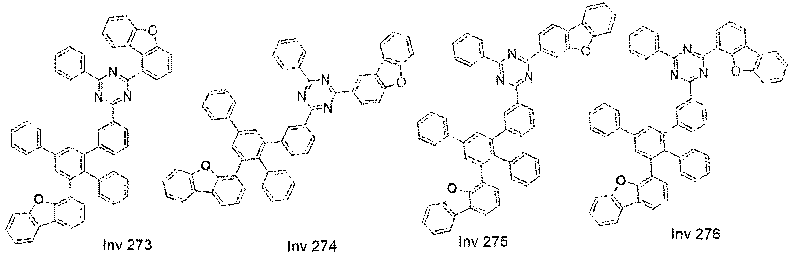

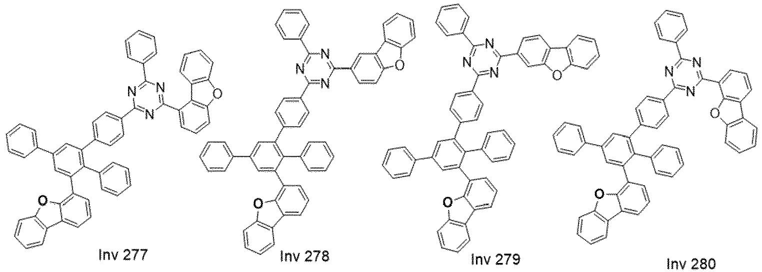

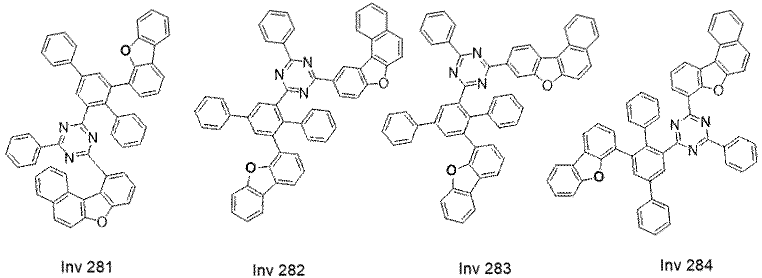

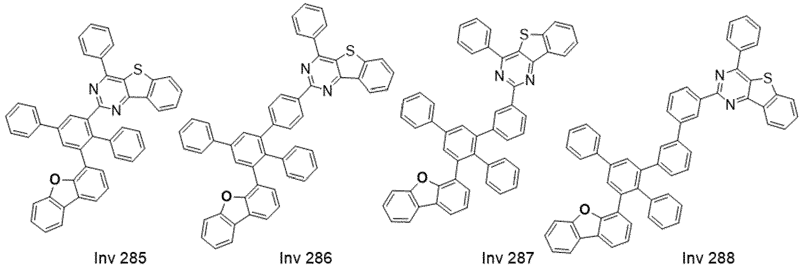

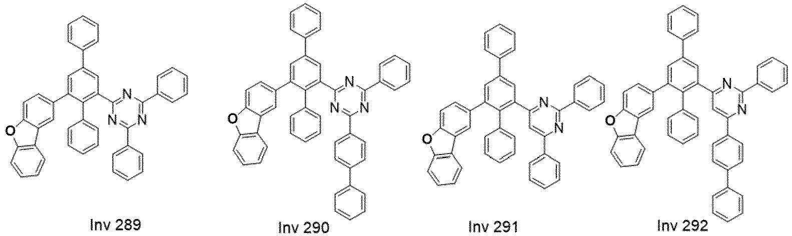

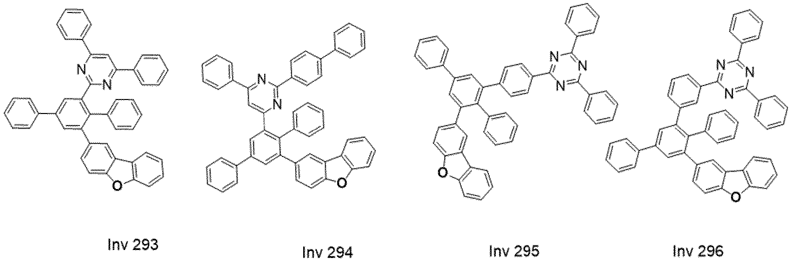

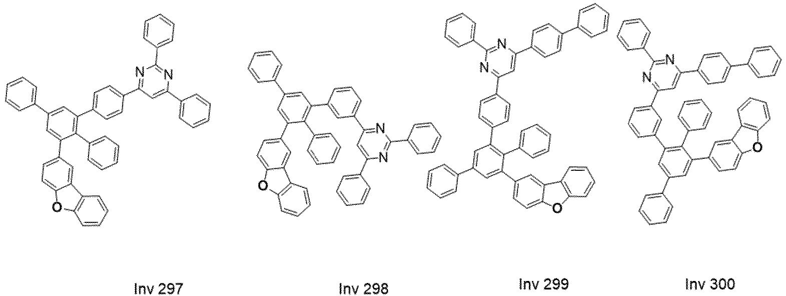

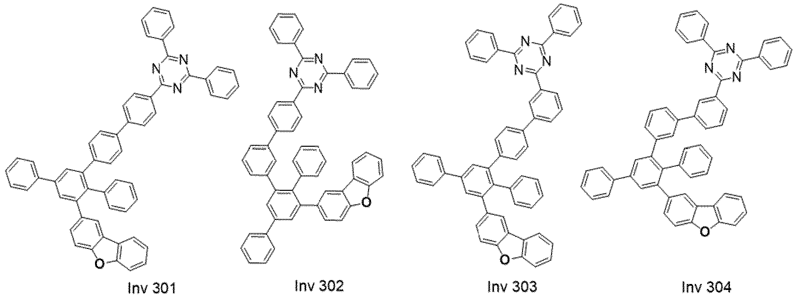

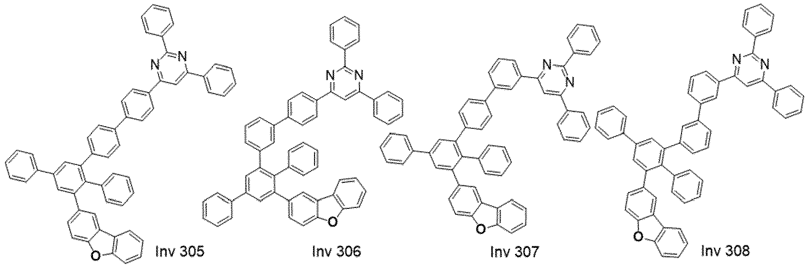

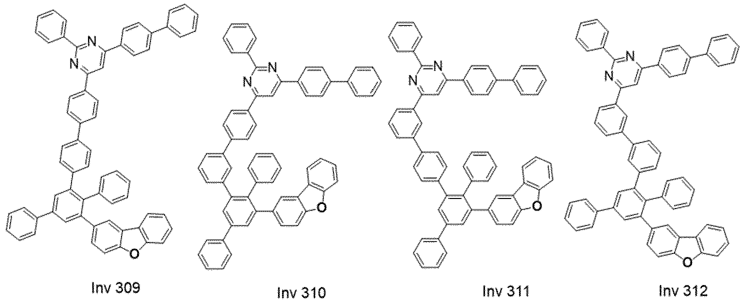

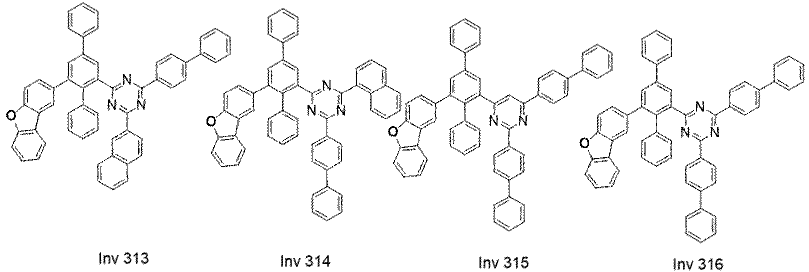

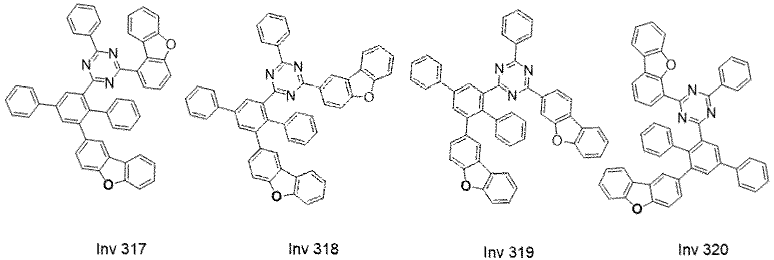

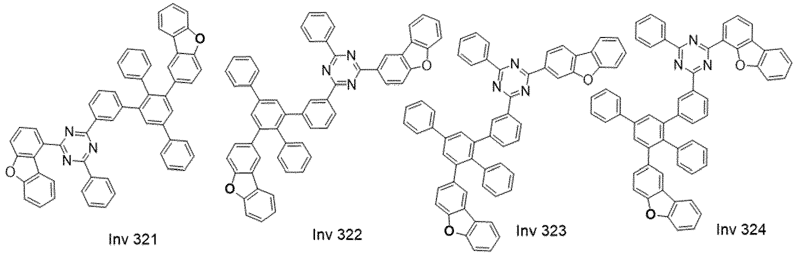

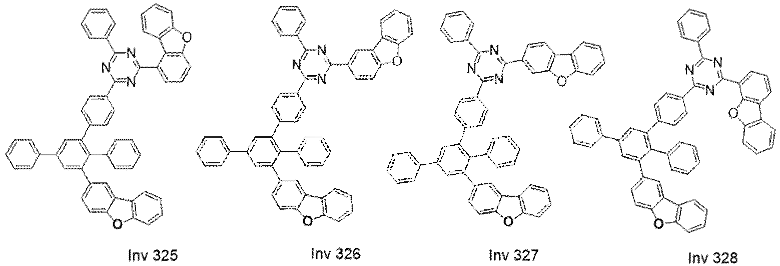

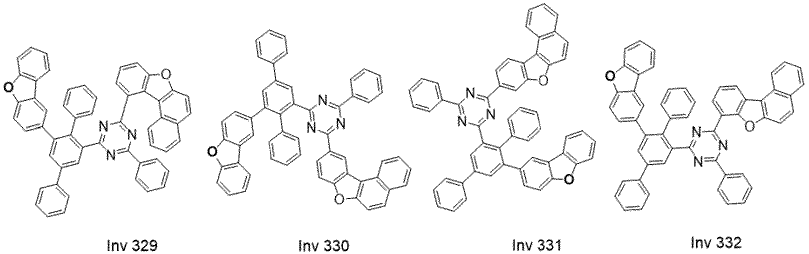

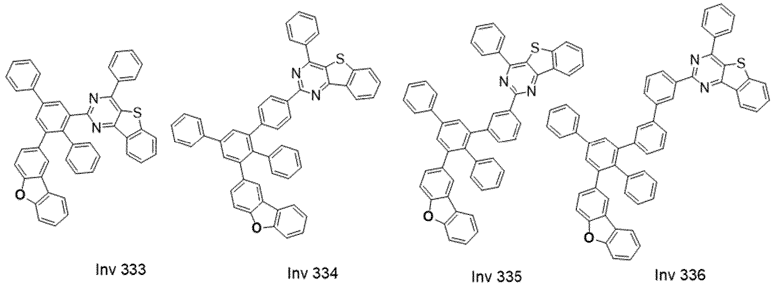

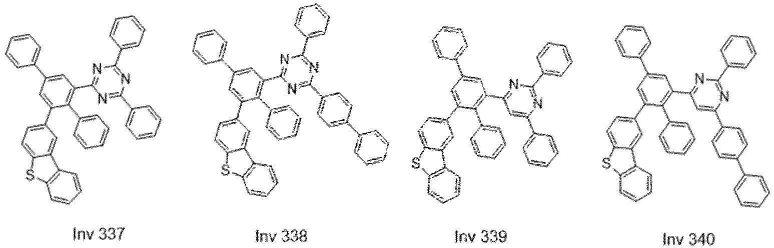

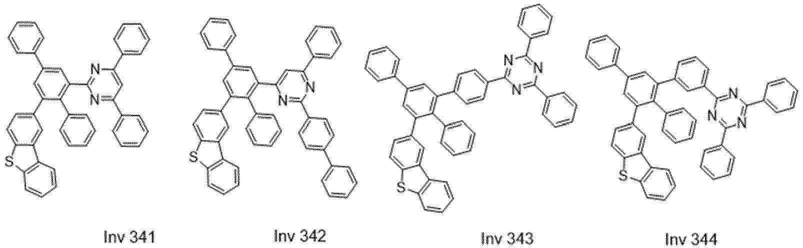

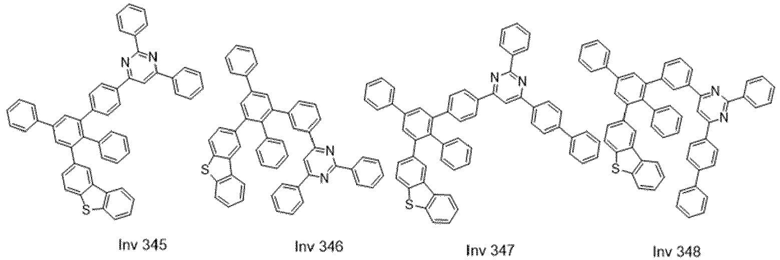

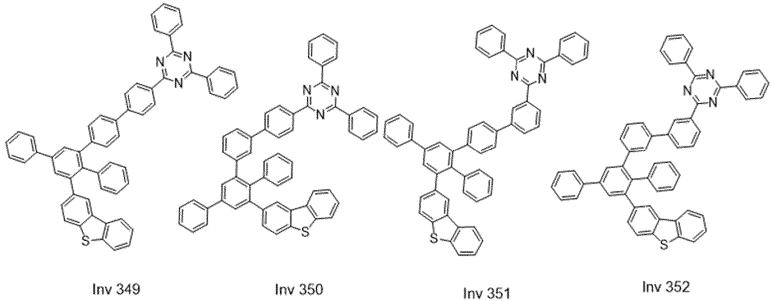

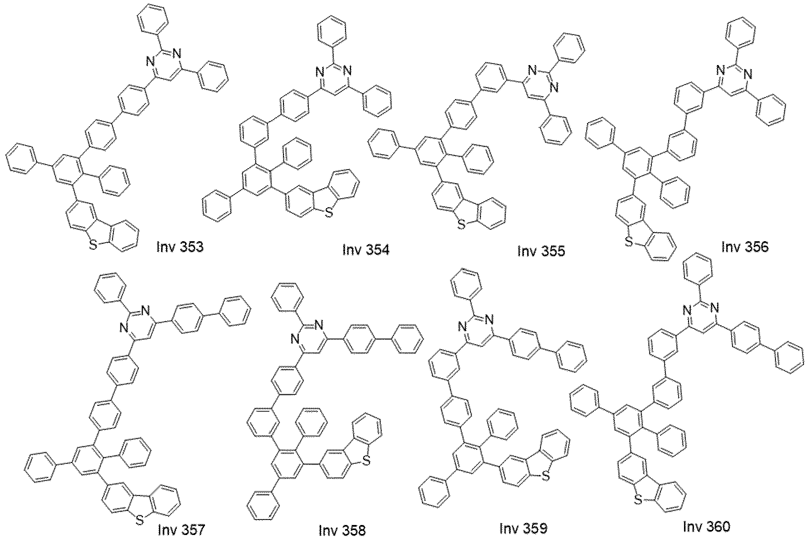

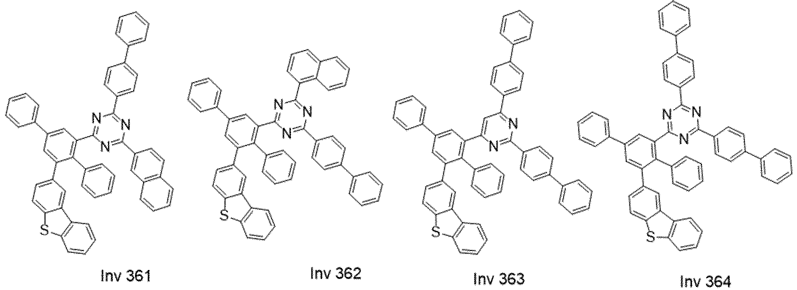

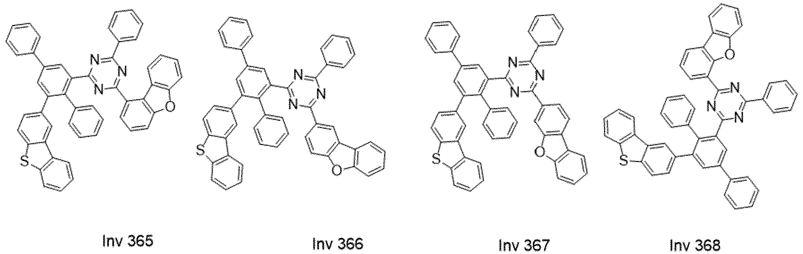

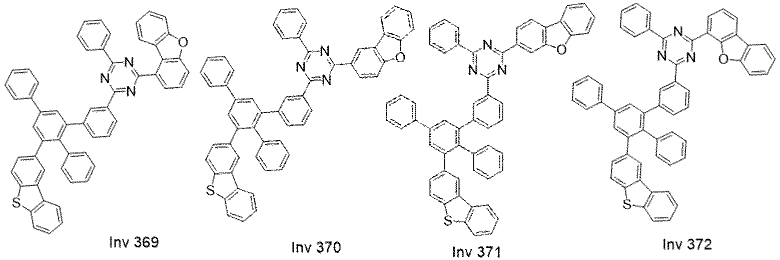

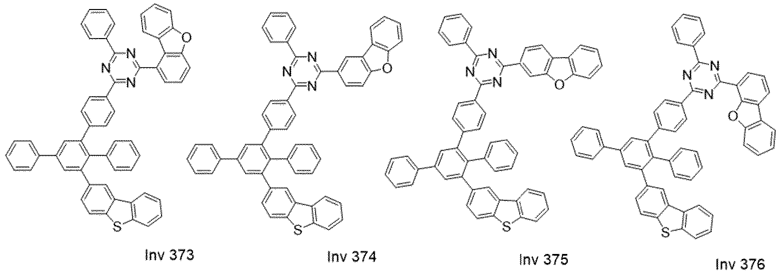

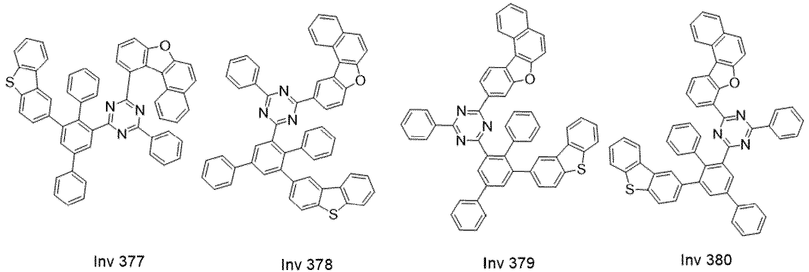

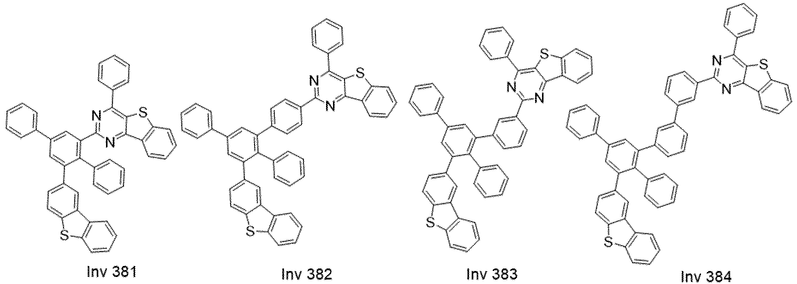

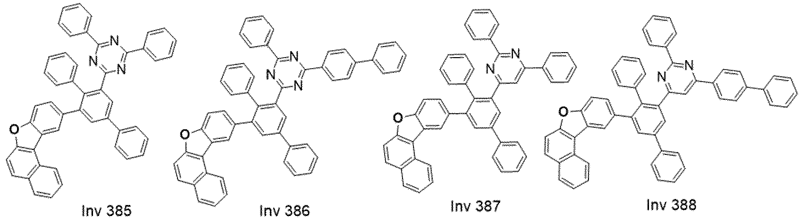

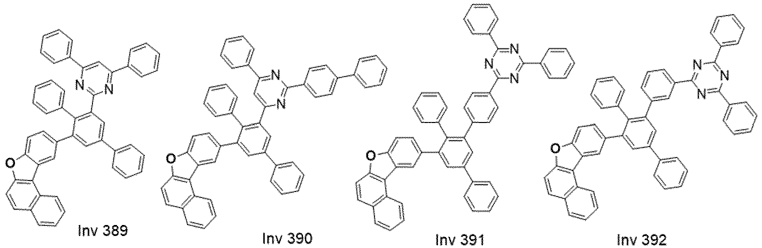

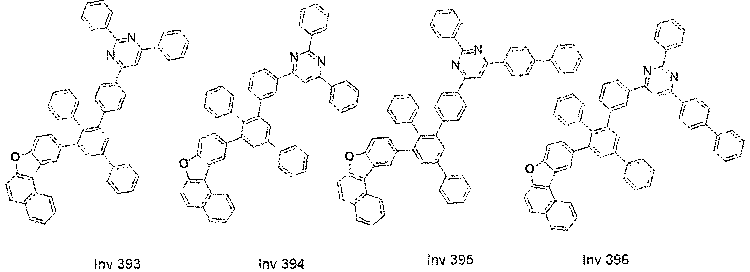

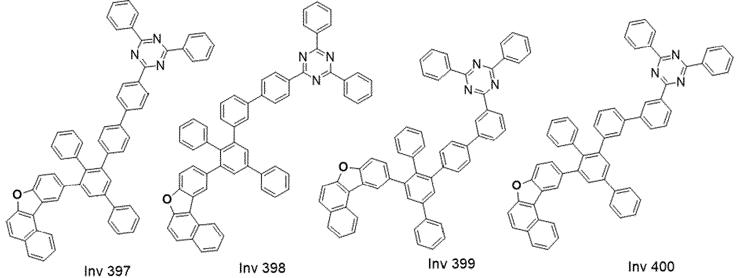

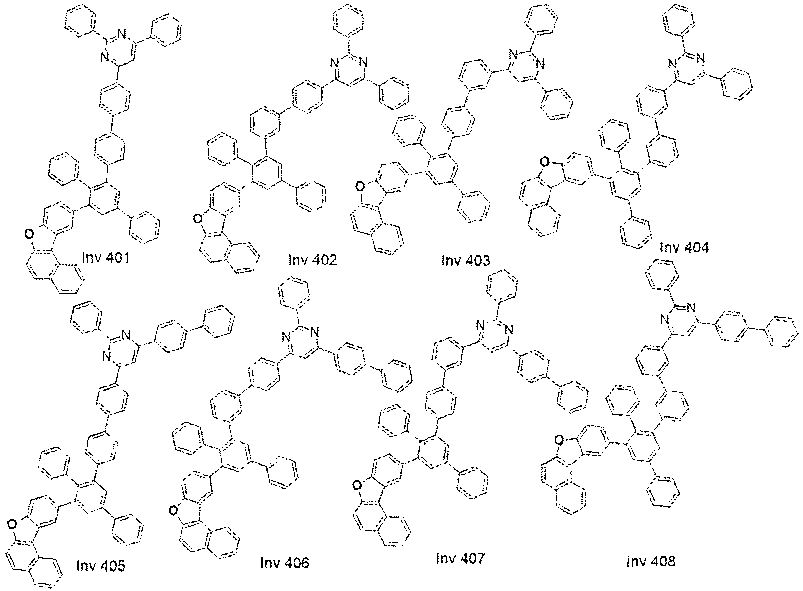

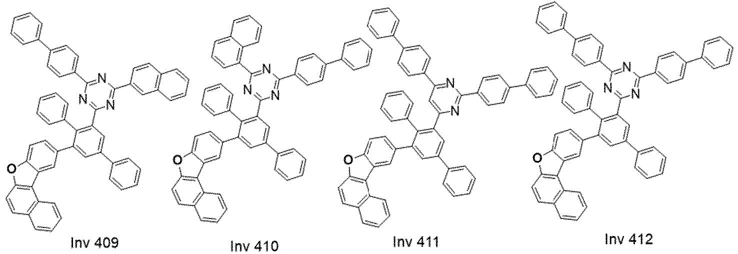

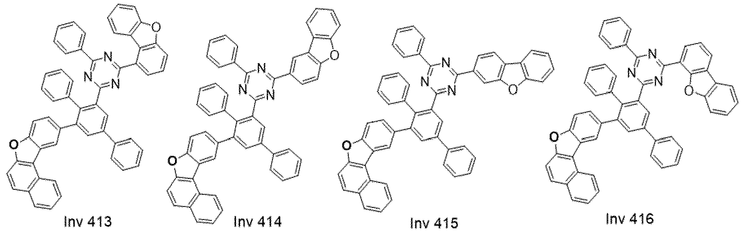

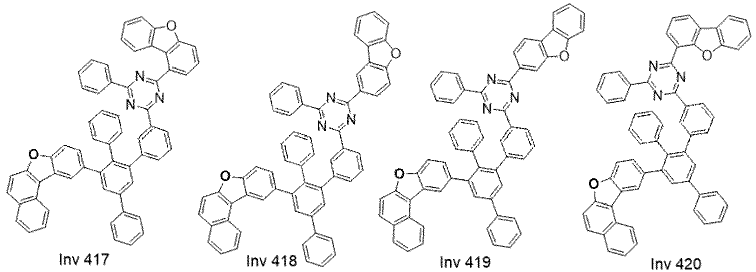

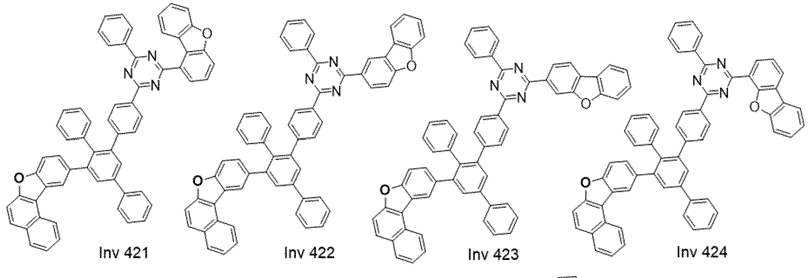

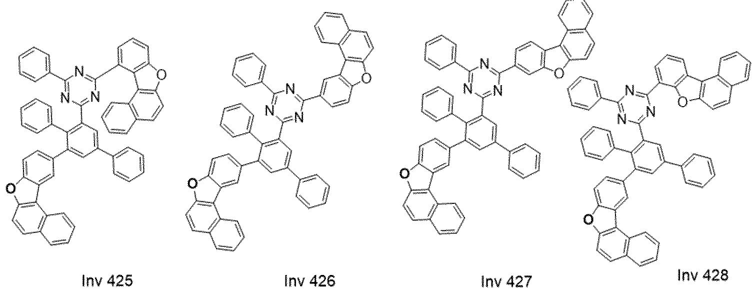

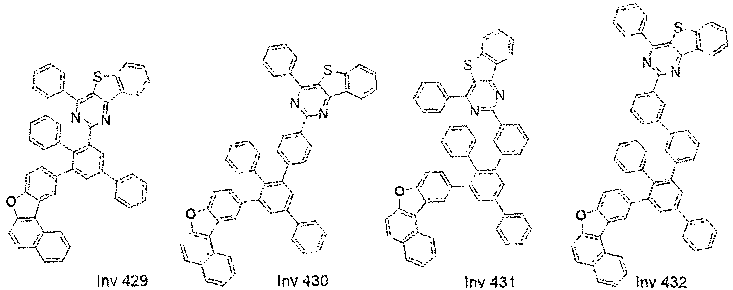

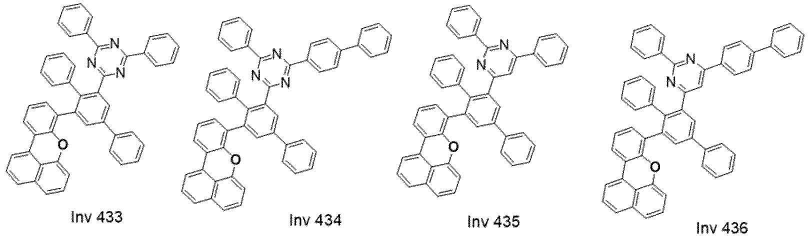

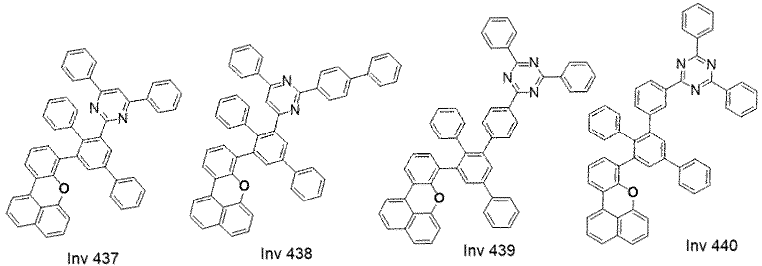

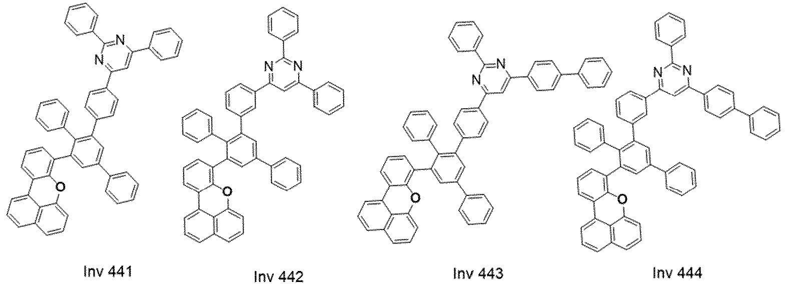

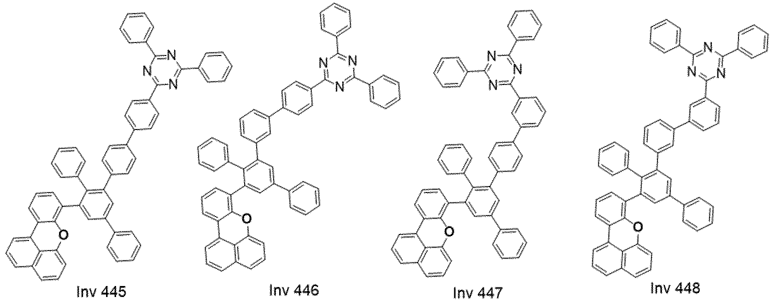

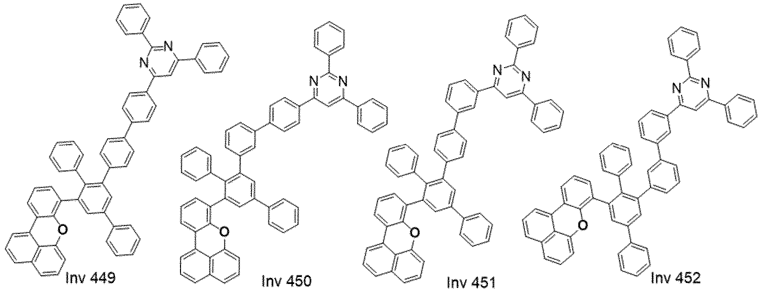

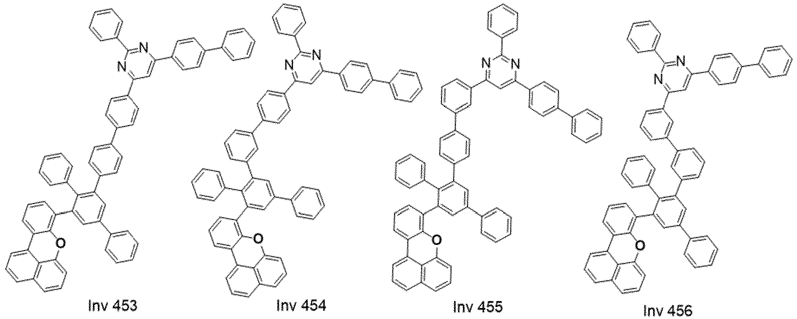

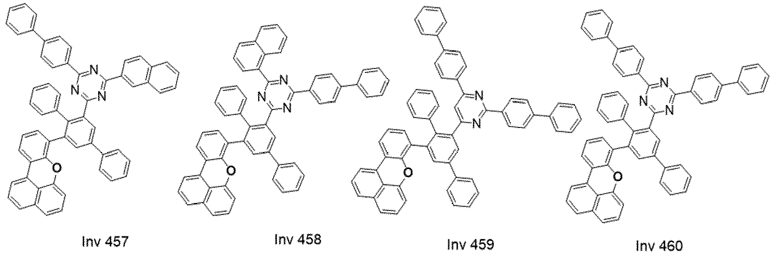

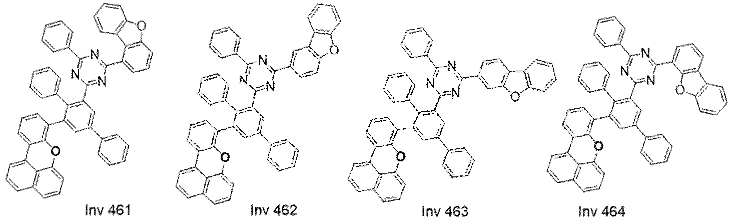

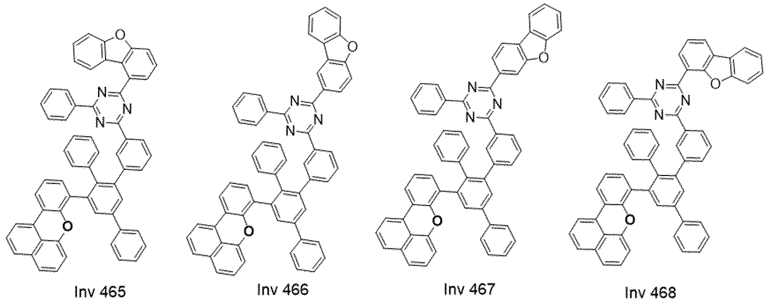

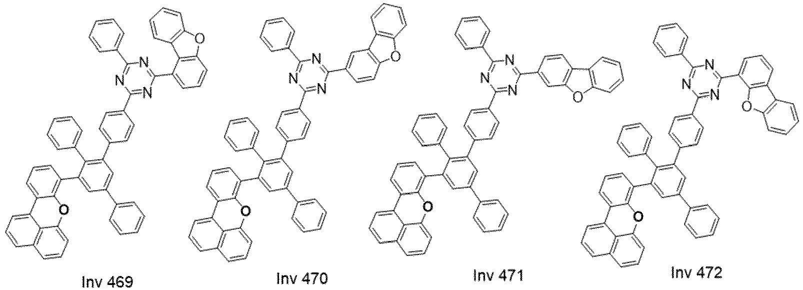

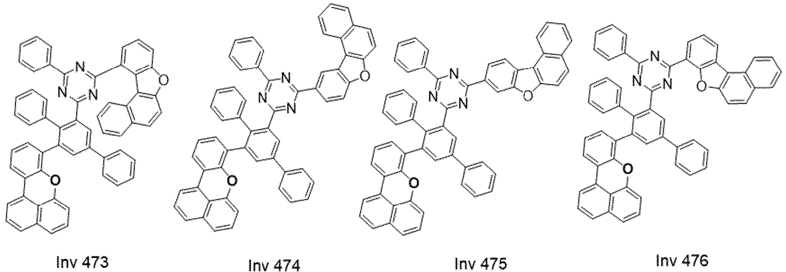

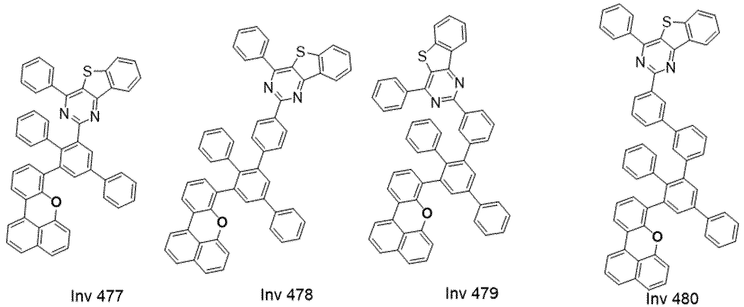

As used herein, the term "alkyl" refers to a monovalent substituent derived from a linear or branched, saturated hydrocarbon having 1 to 40 carbon atoms. Examples thereof may include methyl, ethyl, propyl, isobutyl, sec-butyl, pentyl, iso-amyl, hexyl, and the like, but are not limited thereto.

As used herein, the term "alkenyl" refers to a monovalent substituent derived from a linear or branched, unsaturated hydrocarbon having 2 to 40 carbon atoms having one or more carbon-carbon double bonds. Examples thereof may include vinyl, allyl, isopropenyl, 2-butenyl, and the like, but are not limited thereto.

As used herein, the term "alkynyl" refers to a monovalent substituent derived from a linear or branched, unsaturated hydrocarbon having 2 to 40 carbon atoms with one or more carbon-carbon triple bonds. Examples thereof may include ethynyl, 2-propynyl, and the like, but are not limited thereto.

As used herein, the term "cycloalkyl" refers to a monovalent substituent derived from a monocyclic or polycyclic non-aromatic hydrocarbon having 3 to 40 carbon atoms. Examples of the cycloalkyl may include cyclopropyl, cyclopentyl, cyclohexyl, norbornyl, adamantine, and the like, but are not limited thereto.

As used herein, the term "heterocycloalkyl" refers to a monovalent substituent derived from a non-aromatic hydrocarbon having 3 to 40 nuclear atoms, of which one or more carbons on the ring, preferably 1 to 3 carbons, are substituted with a heteroatom, such as N, O, Se, or S. Examples of the heterocycloalkyl may include morpholine, piperazine, and the like, but are not limited thereto.

As used herein, the term "aryl" refers to a monovalent substituent derived from an aromatic hydrocarbon having 6 to 60 carbon atoms, in which a single ring or two or more rings are combined. Additionally, the aryl may also include a form in which two or more rings are simply pendant to each other or fused to each other. Examples of the aryl may include phenyl, naphthyl, phenanthryl, anthryl, and the like, but are not limited thereto.

As used herein, the term "heteroaryl" refers to a monovalent substituent derived from a monoheterocyclic or polyheterocyclic aromatic hydrocarbon having 5 to 60 nuclear atoms. Particularly, at least one carbon, preferably one to three carbon atoms on the ring are substituted with a heteroatom, such as N, O, S, or Se. In addition, the heteroaryl may include a form in which two or more rings may be simply pendant to each other or fused to each other, and may furthermore include a form of being fused with an aryl group. Examples of the heteroaryl may include: a 6-membered monocyclic ring, such as pyridyl, pyrazinyl, pyrimidinyl, pyridazinyl, and triazinyl; a polycyclic ring, such as phenoxathienyl, indolizinyl, indolyl, purinyl, quinolyl, benzothiazole, and carbazolyl; and 2-furanyl, N-imidazolyl, 2-isoxazolyl, 2-pyridinyl, 2-pyrimidinyl, and the like, but are not limited thereto.

As used herein, the "alkyloxy" refers to a monovalent substituent represented by R'O-, wherein R' is an alkyl having 1 to 40 carbon atoms, and may include a linear, branched, or cyclic structure. Examples of the alkyloxy may include methoxy, ethoxy, n-propoxy, 1-propoxy, t-butoxy, n-butoxy, pentoxy, and the like, but are not limited thereto.

As used herein, the term "aryloxy" refers to a monovalent substituent represented by RO-, wherein R is aryl having 5 to 40 carbon atoms. Examples of the aryloxy may include phenyloxy, naphthyloxy, diphenyloxy, and the like, but are not limited thereto.

As used herein, the term "alkylsilyl" refers to a silyl substituted with an alkyl having 1 to 40 carbon atoms, and examples thereof may include di- and tri-alkylsilyl. As used herein, the term "arylsilyl" refers to a silyl substituted with an aryl having 5 to 60 carbon atoms, and examples thereof may include mono-arylsilyl and polyarylsilyl such as di- and tri-arylsilyl.

As used herein, the term "alkylboron group" refers to a boron group substituted with an alkyl having 1 to 40 carbon atoms, and the term "arylboron group" refers to a boron group substituted with an aryl having 6 to 60 carbon atoms.

As used herein, the term "alkylphosphinyl group" refers to a phosphine group substituted with an alkyl having 1 to 40 carbon atoms, and examples thereof may include mono- and di-alkylphosphinyl. As used herein, the term "arylphosphinyl group" refers to a phosphine group substituted with a monoaryl or diaryl having 6 to 60 carbon atoms, and examples thereof may include mono- and di-arylphosphinyl group.

As used herein, the term "arylamine" refers to an amine substituted with an aryl having 6 to 60 carbon atoms, and examples thereof may include mono-and di-arylamine.

As used herein, the term "heteroarylamine" refers to an amine substituted with a heteroaryl having 5 to 60 nuclear atoms, and examples thereof may include mono-and di-heteroarylamine.

As used herein, the (aryl)(heteroaryl)amine refers to an amine substituted with an aryl having 6 to 60 carbon atoms or a heteroaryl having 5 to 60 nuclear atoms.

As used herein, the term "fused ring" refers to a fused aliphatic ring having 3 to 40 carbon atoms, a fused aromatic ring having 6 to 60 carbon atoms, a fused heteroaliphatic ring having 3 to 60 nuclear atoms, a fused heteroaromatic ring having 5 to 60 nuclear atoms, a spiro ring having 3 to 60 carbon atoms, or a combination thereof.

### <Organic electroluminescent device>

The present invention provides an organic electroluminescence device (hereinafter, "organic EL device") containing the compound represented by Chemical Formula 1.

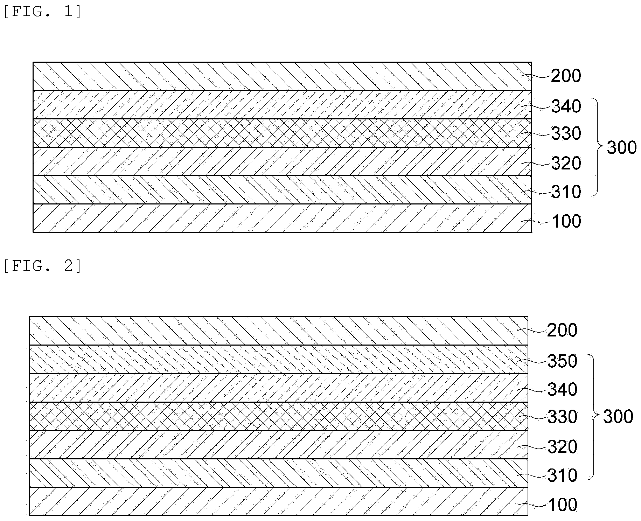

Specifically, the organic electroluminescent device according to the present invention includes, as shown in FIGS. 1 to 3, an anode 100, a cathode 200, and one or more organic layers 300 interposed between the anode and the cathode, wherein at least one of the one or more organic layers include the compound represented by Chemical Formula 1. Particularly, the compound may be used alone or as a combination of two or more kinds thereof.

The one or more organic layers 300 may include any one or more of a hole injection layer 310, a hole transport layer 320, a light emission layer 330, an electron transport layer 340, and an electron injection layer 350, and optionally may further include an electron transport auxiliary layer 360. Particularly, at least one of the organic layers 300 contain the compound represented by Chemical Formula 1. Specifically, the organic layer containing the compound of Chemical Formula 1 may at least any one of the light emission layer 330, the electron transport layer 340, and the electron transport auxiliary layer 360.

According to one embodiment, the one or more organic layers may include a hole injection layer, a hole transport layer, a light emission layer, an electron transport layer, and an electron injection layer, and optionally may further include an electron transport auxiliary layer. The electron transport layer contains the compound represented by Chemical Formula 1. Particularly, the compound represented by Chemical Formula 1 is an electron transport layer material and included in the organic electroluminescent device. In this organic electroluminescent device, electrons are easily injected from the cathode or the electron injection layer into the electron transport layer due to the compound of Chemical Formula 1, and can also quickly moved from the electron transport layer to the light emission layer, so that the binding force between holes and electrons in the light emission layer is enhanced. Therefore, the organic electroluminescent device of the present invention is excellent in terms of light emission efficiency, power efficiency, brightness, and the like. Furthermore, the compound of Chemical Formula 1 has excellent thermal stability and electrochemical stability and thus can improve the performance of the organic electroluminescent device.

The compound of Chemical Formula 1 may be used alone or mixed with an electron transport layer material known in the art.

In the present invention, the electron transport layer material that can be used in mix with the compound of Formula 1 includes electron transport materials commonly known in the art. Non-limiting examples of usable electron transport materials may include oxazole-based compounds, isoxazole-based compounds, triazole-based compounds, isothiazole-based compounds, oxadiazole-based compounds, thiadiazole-based compounds, perylene-based compounds, aluminum complexes (e.g., Alq₃, tris(8-quinolinolato)-aluminum), and gallium complexes (e.g., Gaq'2OPiv, Gaq'2OAc, and 2(Gaq'2)). These may be used alone or in combination of two or more.

In the present invention, when the compound of Chemical Formula 1 and the electron transport layer material are mixed, the mixing ration thereof is not particularly limited and may be appropriately adjusted within a range known in the art.

According to another embodiment, the one or more organic layers may include a hole injection layer, a hole transport layer, a light emission layer, an electron transport auxiliary layer, an electron transport layer, and an electron injection layer, wherein the electron transport auxiliary layer may include the compound represented by Chemical Formula 1. Particularly, the compound represented by Chemical Formula 1 is an electron transport auxiliary layer material and included in the organic electroluminescent device. Particularly, the compound of Chemical Formula 1 has a high triplet energy. Therefore, when the compound of Chemical Formula 1 is included as an electron transport auxiliary layer material, the efficiency of the organic electroluminescent device can be improved due to a triplet-triplet fusion (TTF) effect. In addition, the compound of Chemical Formula 1 can prevent excitons or holes generated in the light emission layer from diffusing into the electron transport layer adjacent to the light emission layer. Therefore, the number of excitons contributing to light emission in the light emission layer is increased to improve the light emission efficiency of devices, and the lifespan of devices can be efficiently increased through improved durability and stability of devices.

The compound of Chemical Formula 1 may be used alone or mixed with an electron transport auxiliary layer material known in the art.

In the present invention, the electron transport auxiliary layer material that can be used in mix with the compound of Formula 1 includes electron transport materials commonly known in the art, and examples thereof may include oxadiazole derivatives, triazole derivatives, phenanthroline derivatives (e.g., BCP), nitrogen-containing heterocyclic derivatives, and the like, but are not limited thereto.

According to another embodiment, the one or more organic layers include a hole injection layer, a hole transport layer, a light emission layer, an electron transport layer, and an electron injection layer, and may optionally further include an electron transport auxiliary layer, and the light emission layer may include a host and a dopant, wherein the host may be a compound represented by Chemical Formula 1 above. Particularly, the light emission layer of the present invention may contain a compound known in the art as a second host, besides the compound of Chemical Formula 1.

In the present invention, the content of the host may be about 70 to 99.9 wt% based on the total amount of the light emission layer, and the content of the dopant may be about 0.1 to 30 wt% based on the total amount of the light emission layer.

When the compound represented by Chemical Formula 1 is included as a light emission layer material of an organic electroluminescent device, specifically a blue, green, or red phosphorescent host material, the binding force between holes and electrons in the light emission layer can be enhanced, leading to improvements in efficiency (light emission efficiency and power efficiency), lifespan, brightness, and driving voltage of the organic electroluminescent device. Specifically, the compound represented by Chemical Formula 1 may preferably be included in the organic electroluminescent device as a green and/or red phosphorescent host, a fluorescent host, or a dopant material. Especially, the compound represented by Chemical Formula 1 is preferably a green phosphorescent exciplex N-type host material with high efficiency of a light emission layer.

The structure of the organic electroluminescent device of the present invention described above is not particularly limited, but for example, the anode 100, the one or more organic layers 300, and the cathode 200 may be sequentially stacked on the substrate (FIGS. 1 to 3). Although not shown, the organic electroluminescent device may have a structure where an insulating layer or an adhesive layer is inserted in the interface between the electrode and the organic material layer.

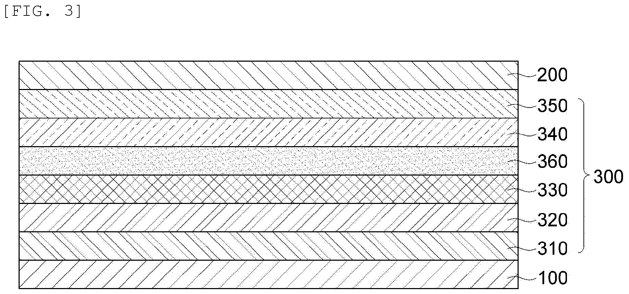

According to one embodiment, the organic electroluminescent device, as shown in FIG. 1, may have a structure in which the anode 100, the hole injection layer 310, the hole transport layer 320, the light emission layer 330, the electron transport layer 340, and the cathode 200 are sequentially stacked on the substrate. Optionally, as shown in FIG. 2, the electron injection layer 350 may be disposed between the electron transport layer 340 and the cathode 200. Additionally, the electron transport auxiliary layer 360 may be disposed between the light emission layer 330 and the electron transport layer 340 (see FIG. 3).

The organic electroluminescent device of the present invention may be manufactured by forming organic layers and electrodes using materials and methods known in the art, except that at least one of the organic materials 300 (e.g., the light emission layer 330, the electron transport layer 340, or the electron transport auxiliary layer 360) contains the compound represented by Chemical Formula 1.

The organic layers may be formed by vacuum deposition or solution application. Examples of the solution application may include spin coating, dip coating, doctor blading, inkjet printing, thermal transfer, and the like, but are not limited thereto.

The substrate that can be used in the present invention is not particularly limited, and non-limiting examples thereof include silicon wafers, quartz, glass plates, metal plates, plastic films and sheets.

Examples of the anode material include: a metal, such as vanadium, chromium, copper, zinc, or gold, or an alloy thereof; a metal oxide, such as zinc oxide, indium oxide, indium tin oxide (ITO), or indium zinc oxide (IZO); a combination of a metal and an oxide, such as ZnO:Al or SnO₂:Sb; a conductive polymer, such as polythiophene, poly(3-methylthiophene), poly[3,4-(ethylene-1,2-dioxy)thiophene] (PEDT), polypyrrole, or polyaniline; and carbon black, but are not limited thereto.

Examples of the cathode material include a metal, such as magnesium, calcium, sodium, potassium, titanium, indium, yttrium, lithium, gadolinium, aluminum, silver (Ag), tin, or lead, or an alloy thereof; and a multilayer structure material, such as LiF/Al or LiO₂/Al, but are not limited thereto.

Additionally, the hole injection layer, hole transport layer, light emission layer, and electron injection layer are not particularly limited, and common materials known in the art may be used therefor.

Hereinafter, the present invention will be described in detail with reference to examples. However, the following examples are merely for illustrating the present disclosure and are not intended to limit the scope of the present disclosure.

### [Preparation Example 11 Synthesis of Core 1

### <Step 1> Synthesis of 3-(6'-chloro-[1,1':4',1"-terphenyl]-2'-yl)-9,9-dimethyl-9H-fluorene

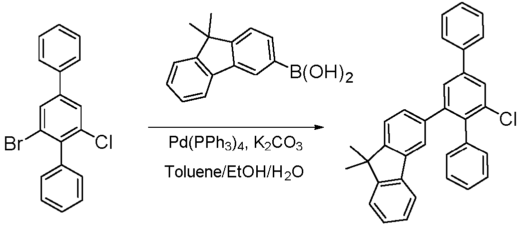

2'-Bromo-6'-chloro-1,1':4',1"-terphenyl (50 g, 145.5 mmol), (9,9-dimethyl-9H-fluoren-3-yl)boronic acid (41.6 g, 174.6 mmol), Pd(PPh₃)₄ (5.04 g, 4.36 mmol), and K₂CO₃ (60.3 g, 436.54 mmol)were added to 400 ml of toluene, 100 ml of EtOH, and 100 ml of H₂O, and the resultant mixture was stirred with heating under reflux for 3 hours. After the completion of the reaction, the organic layer was extracted with methylene chloride, and filtered by addition of MgSO₄. The filtered organic layer was subjected to solvent removal, and then purified by column chromatography to give 3-(6'-chloro-[1,1' :4',1"'-terphenyl]-2'-yl)-9,9-dimethyl-9H-fluorene (48.7 g, yield: 73%).
Mass : [(M+H)⁺] : 458

### <Step 2> Synthesis of Core1

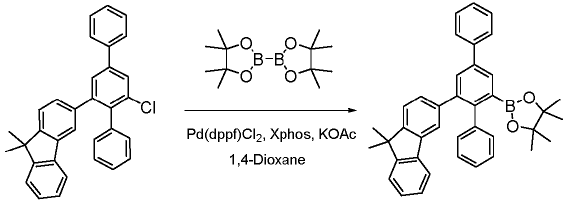

3-(6'-Chloro-[1,1':4',1''-terphenyl]-2'-yl)-9,9-dimethyl-9H-fluorene (48.7 g, 106.5 mmol), 4,4,4',4',5,5,5',5'-octamethyl-2,2'-bi(1,3,2-dioxaborolane) (32 g, 127.8 mmol), Pd(dppf)Cl₂ (2.34 g, 3.19 mmol), KOAc (31.3 g, 319.6 mmol), and Xphos (5.08 g, 10.65 mmol) were added to 500 ml of 1,4-dioxane, and the resultant mixture was heated under reflux for 12 hours. After the completion of the reaction, the organic layer was extracted with methylene chloride, and filtered by addition of MgSO₄. The filtered organic layer was subjected to solvent removal, and then purified by column chromatography to give Core 1 (38.7 g, yield: 66%).
[LCMS] . 549

### [Preparation Example 2] Synthesis of Core 2

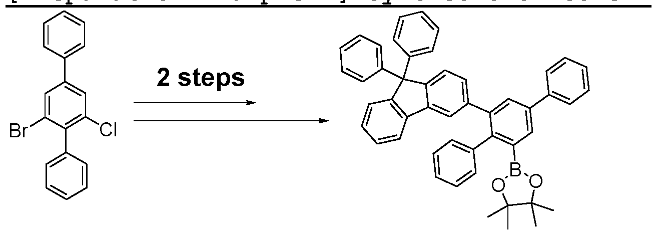

Core 2 (68.6 g, yield: 70%) was obtained by performing the same method as in [Preparation Example 1] except that (9,9-diphenyl-9H-fluoren-3-yl)boronic acid (63.2 g, 174.6 mmol) was used instead of (9,9-dimethyl-9H-fluoren-3-yl)boronic acid use d as a reactant in <Step 1> of [Preparation Example 1].
[LCMS] . 673

### [Preparation Example 31 Synthesis of Core 3

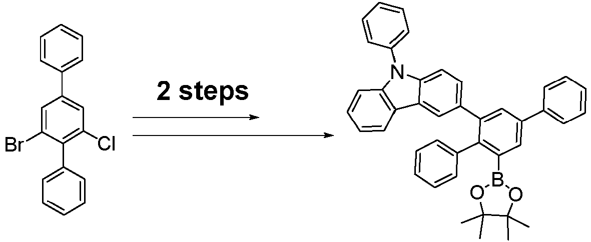

Core 3 (63.4 g, yield: 73%) was obtained by performing the same method as in [Preparation Example 1] except that (9,9-dimethyl-9H-fluoren-3-yl)boronic acid (50.1 g, 174.6 mmol) was used instead of (9-dimethyl-9H-fluoren-3-yl)boronic acid used as a reactant in <Step 1> of [Preparation Example 1] .
[LCMS] . 598

### [Preparation Example 4] Synthesis of Core 4

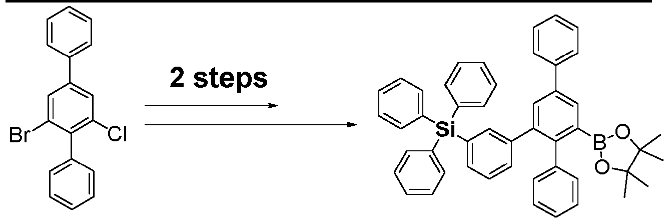

Core 4 (69.4 g, yield: 69%) was obtained by performing the same method as in [Preparation Example 1] except that (3-(triphenylsilyl)phenyl)boronic acid (66.4 g, 174.6 mmol) was used instead of (9,9-dimethyl-9H-fluoren-3-yl)boronic acid used as a reactant in <Step 1> of [Preparation Example 1].
[LCMS] . 691

### [Preparation Example 5] Synthesis of Core 5

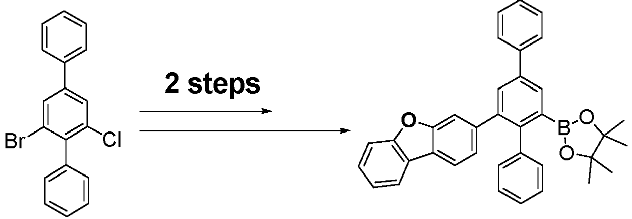

Core 5 (53.2 g, yield: 70%) was obtained by performing the same method as in [Preparation Example 1] except that dibenzo[b,d]furan-3-ylboronic acid (37 g, 174.6 mmol) was used instead of (9,9-dimethyl-9H-fluoren-3-yl)boronic acid used as a reactant in <Step 1> of [Preparation Example 1].
[LCMS] . 523

### [Preparation Example 61 Synthesis of Core 6

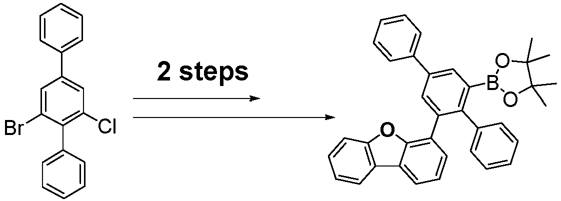

Core 6 (53.8 g, yield: 71%) was obtained by performing the same method as in [Preparation Example 1] except that dibenzo[b,d]furan-4-ylboronic acid (37 g, 174.6 mmol) was used instead of (9,9-dimethyl-9H-fluoren-3-yl)boronic acid used as a reactant in <Step 1> of [Preparation Example 1].
[LCMS] . 523

### [Preparation Example 7] Synthesis of Core 7

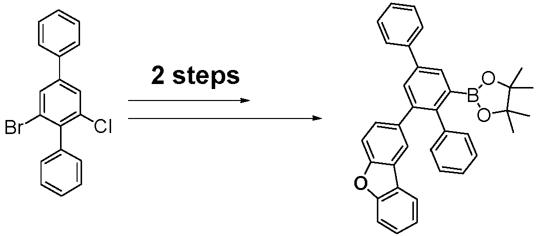

Core 7 (55.6g, yield: 73%) was obtained by performing the same method as in [Preparation Example 1] except that dibenzo[b,d]furan-2-ylboronic acid(37 g, 174.6 mmol) was used instead of (9,9-dimethyl-9H-fluoren-3-yl)boronic acid used as a reactant in <Step 1> of [Preparation Example 1].
[LCMS] . 523

### [Preparation Example 8] Synthesis of Core 8

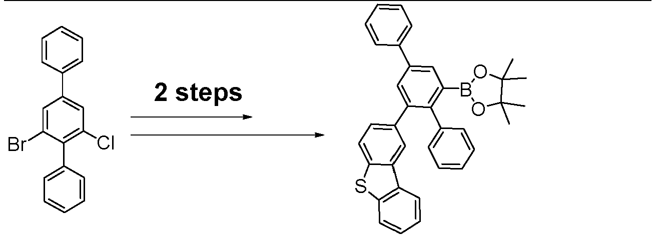

Core 8 (57.2g, yield: 73%) was obtained by performing the same method as in [Preparation Example 1] except that dibenzo[b,d]thiophen-2-ylboronic acid(39.8 g, 174.6 mmol) was used instead of (9,9-dimethyl-9H-fluoren-3-yl)boronic acid used as a reactant in <Step 1> of [Preparation Example 1].
[LCMS] . 539

### [Preparation Example 9] Synthesis of Core 9

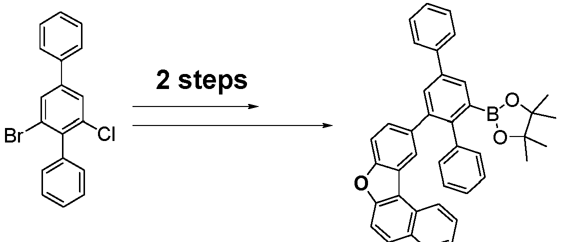

Core 9 (59.3 g, yield: 71%) was obtained by performing the same method as in [Preparation Example 1] except that naphtho[2,1-b]benzofuran-10-ylboronic acid(45.7 g, 174.6 mmol) was used instead of (9,9-dimethyl-9H-fluoren-3-yl)boronic acid used as a reactant in <Step 1> of [Preparation Example 1] .
[LCMS] . 573

### [Preparation Example 10] Synthesis of Core 10

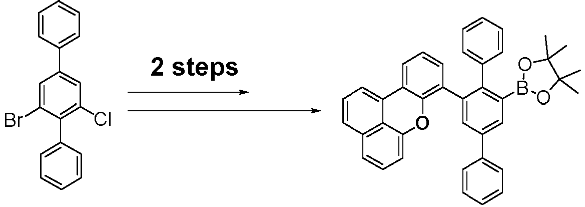

Core 10 (58.6 g, yield: 70%) was obtained by performing the same method as in [Preparation Example 1] except that benzo[kl]xanthen-8-ylboronic acid(45.7 g, 174.6 mmol) was used instead of (9,9-dimethyl-9H-fluoren-3-yl)boronic acid used as a reactant in <Step 1> of [Preparation Example 1].
[LCMS] . 573

### [Synthesis Example 11 Synthesis of Compound Inv 11

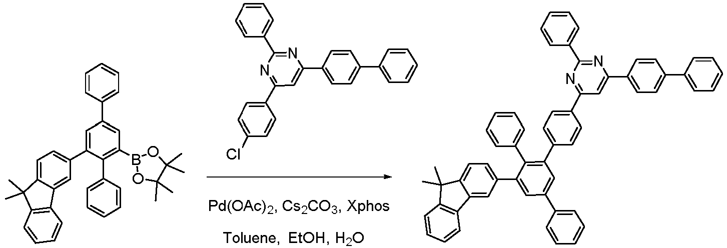

Core 1 (10 g, 18.23 mmol) in [Preparation Example 1] and 4-([1, 1'-biphenyl]-4-yl)-6-(4-chlorophenyl)-2-phenylpyrimidine (6.3 g, 15.19 mmol), together with Pd(OAc)₂ (0.1 g, 0.45 mmol), Cs₂CO₃ (14.8 g, 45.5 mmol), and Xphos (0.72 g, 1.52 mmol) were added to 100 ml of toluene, 25 ml of EtOH, and 25 ml of H₂O, and the mixture was heated under reflux for 12 hours. After the completion of the reaction, the organic layer was extracted with methylene chloride, and filtered by addition of MgSO₄. The filtered organic layer was subjected to solvent removal, and then purified by column chromatography to give Compound Inv 11 (80 g, yield: 65%).
[LCMS] . 806

### [Synthesis Example 2] Synthesis of Compound Inv 34

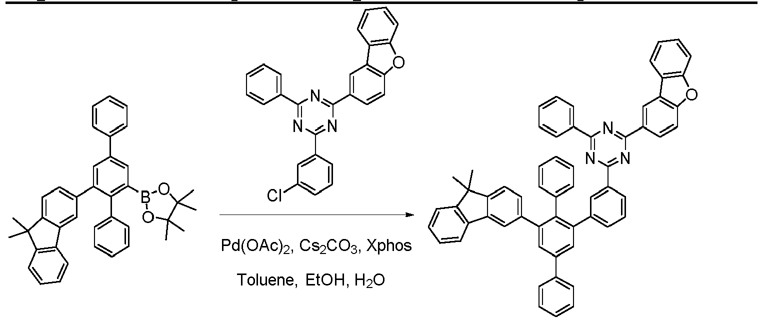

Compound Inv 34 (7.7 g, yield: 62 %) was obtained by performing the same method as in [Synthesis Example 1] except that 2-(3-chlorophenyl)-4-(dibenzo[b,d]furan-2-yl)-6-phenyl-1,3,5-triazine (6.6 g, 15.19 mmol) was used instead of 4-([1,1'-biphenyl]-4-yl)-6-(4-chlorophenyl)-2-phenylpyrimidine used in Synthesis Example 1.
[LCMS] . 821

### [Synthesis Example 31 Synthesis of Compound Inv 55

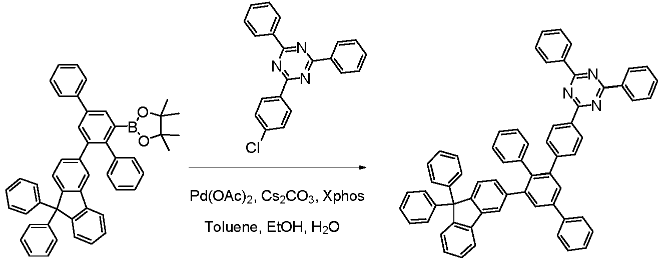

Compound Inv 55 (6.7 g, yield: 63 %) was obtained by performing the same method as in [Synthesis Example 1] except that Core 2 (10 g, 14.86 mmol) and 2-(4-chlorophenyl)-4,6-diphenyl-1,3,5-triazine (4.26 g, 12.38mmol) were respectively used instead of Core 1 and 4-([1,1'-biphenyl]-4-yl)-6-(4-chlorophenyl)-2-phenylpyrimidine used in Synthesis Example 1.
[LCMS] . 855

### [Synthesis Example 4] Synthesis of Compound Inv 60

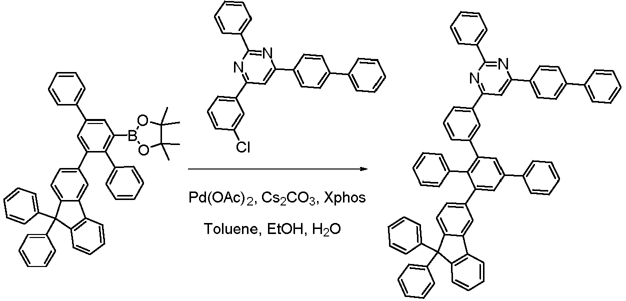

Compound Inv 60 (7.3 g, yield: 63 %) was obtained by performing the same method as in [Synthesis Example 3] except that 4-([1,1'-biphenyl]-4-yl)-6-(3-chlorophenyl)-2-phenylpyrimidine (5.19 g 12.38 mmol) was used instead of 2-(4-chlorophenyl)-4,6-diphenyl-1,3,5-triazine used in Synthesis Example 3.
[LCMS] . 930

### [Synthesis Example 5] Synthesis of Compound Inv 121

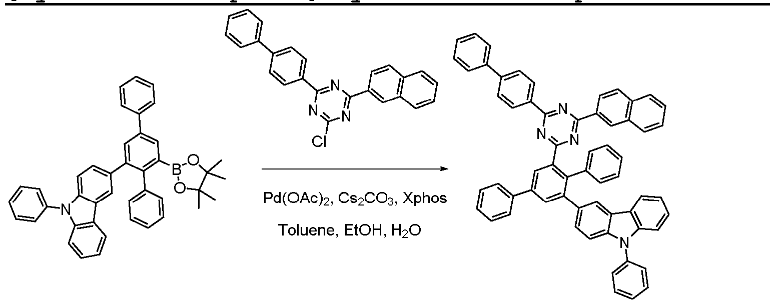

Compound Inv 121 (7.8 g, yield: 67 %) was obtained by performing the same method as in [Synthesis Example 1] except that Core 3 (10 g, 16.73 mmol) and 2-([1,1'-biphenyl]-4-yl)-4-chloro-6-(naphthalen-2-yl)-1,3,5-triazine (5.49 g 13.94 mmol) were respectively used instead of Core 1 and 4-([1,1'-biphenyl]-4-yl)-6-(4-chlorophenyl)-2-phenylpyrimidine used in Synthesis Example 1.
[LCMS] . 830

### [Synthesis Example 6] Synthesis of Compound Inv 97

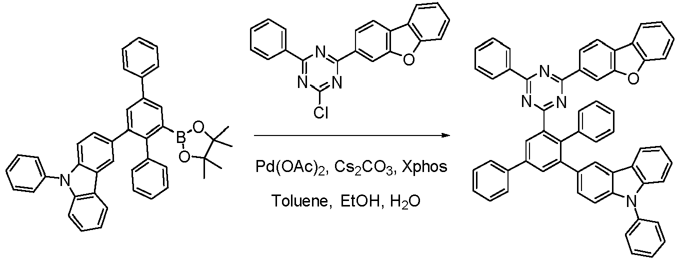

Compound Inv 97 (7.2 g, yield: 65 %) was obtained by performing the same method as in [Synthesis Example 5] except that 2-chloro-4-(dibenzo[b,d]furan-3-yl)-6-phenyl-1,3,5-triazine (4.98 g 13.94 mmol) was used instead of 2-([1,1'-biphenyl]-4-yl)-4-chloro-6-(naphthalen-2-yl)-1,3,5-triazine used in Synthesis Example 5.
[LCMS] . 793

### [Synthesis Example 7] Synthesis of Compound Inv 154

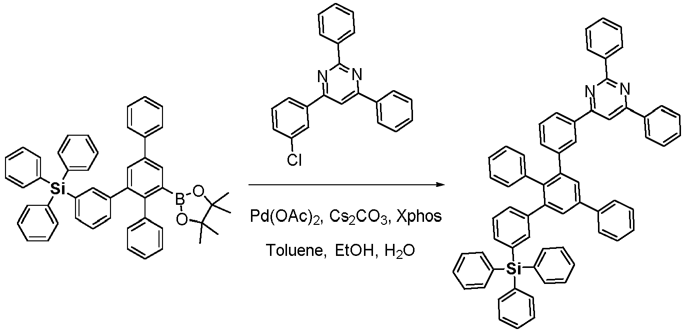

Compound Inv 154 (6.7 g, yield: 64 %) was obtained by performing the same method as in [Synthesis Example 1] except that Core 4 (10 g, 14.47 mmol) and 4-(3-chlorophenyl)-2,6-diphenylpyrimidine (4.13 g, 12.06 mmol) were respectively used instead of Core 1 and 4-([1,1'-biphenyl]-4-yl)-6-(4-chlorophenyl)-2-phenylpyrimidine used in Synthesis Example 1.
[LCMS] . 872

### [Synthesis Example 8] Synthesis of Compound Inv 189

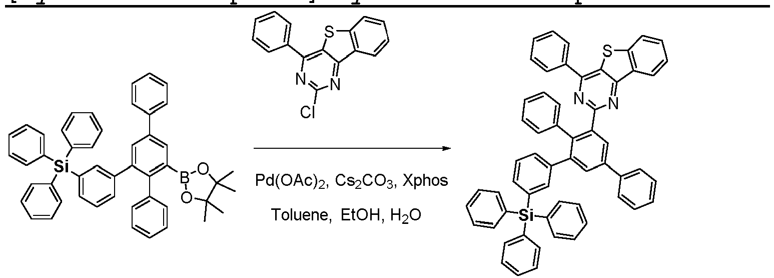

Compound Inv 189 (6.4 g, yield: 64 %) was obtained by performing the same method as in [Synthesis Example 7] except that 2-chloro-4-phenylbenzo[4,5]thieno[3,2-d]pyrimidine (3.58 g, 12.06 mmol) was used instead of 4-(3-chlorophenyl)-2,6-diphenylpyrimidine used in Synthesis Example 7.
[LCMS] . 826

### [Synthesis Example 9] Synthesis of Compound Inv 196

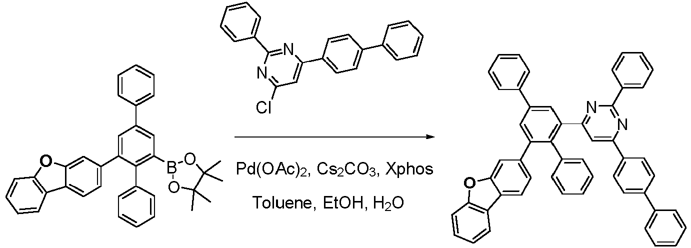

Compound Inv 196 (7.1 g, yield: 63 %) was obtained by performing the same method as in [Synthesis Example 1] except that Core 5 (10 g, 19.14 mmol) and 4-([1,1'-biphenyl]-4-yl)-6-chloro-2-phenylpyrimidine (5.46 g, 15.95 mmol) were respectively used instead of Core 1 and 4-([1,1'-biphenyl]-4-yl)-6-(4-chlorophenyl)-2-phenylpyrimidine used in Synthesis Example 1.
[LCMS] . 703

### [Synthesis Example 10] Synthesis of Compound Inv 220

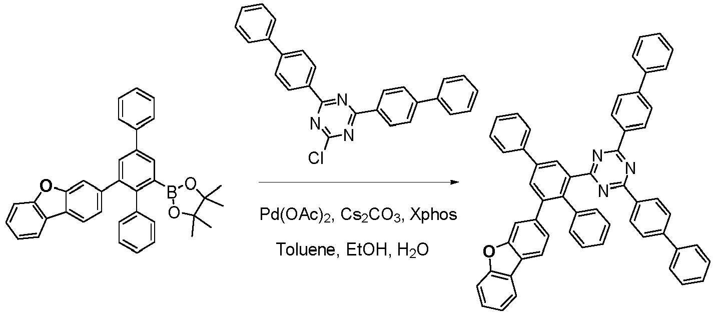

Compound Inv 220 (7.8 g, yield: 63 %) was obtained by performing the same method as in [Synthesis Example 9] except that 2,4-di([1,1'-biphenyl]-4-yl)-6-chloro-1,3,5-triazine (6.69 g, 15.95 mmol) was used instead of 4-([1,1'-biphenyl]-4-yl)-6-chloro-2-phenylpyrimidine used in Synthesis Example 9.
[LCMS] . 780

### [Synthesis Example 11] Synthesis of Compound Inv 224

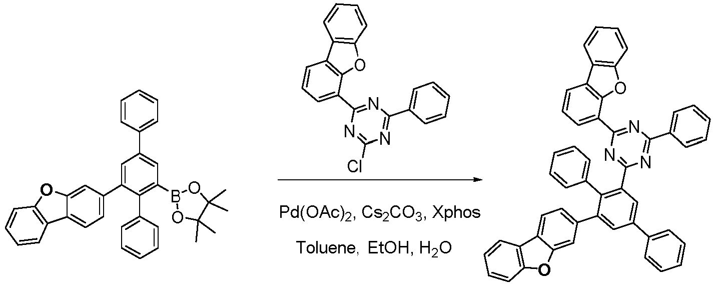

Compound Inv 224 (7.6 g, yield: 66 %) was obtained by performing the same method as in [Synthesis Example 9] except that 2-chloro-4-(dibenzo[b,d]furan-4-yl)-6-phenyl-1,3,5-triazine (5.71 g, 15.95 mmol) was used instead of 4-([1,1'-biphenyl]-4-yl)-6-chloro-2-phenylpyrimidine used in Synthesis Example 9.
[LCMS] . 718

### [Synthesis Example 121 Synthesis of Compound Inv 239

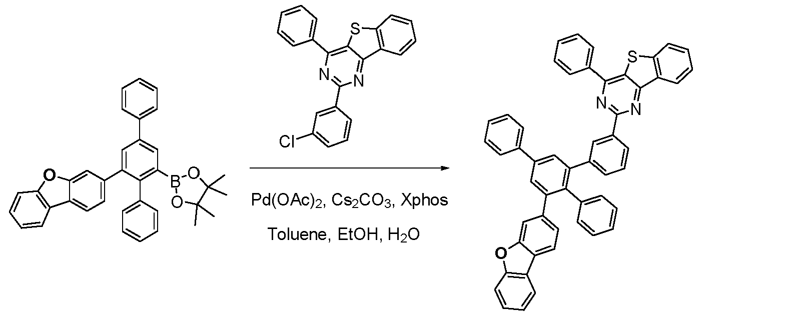

Compound Inv 239 (7.4 g, yield: 63 %) was obtained by performing the same method as in [Synthesis Example 9] except that 2-(3-chlorophenyl)-4-phenylbenzo[4,5]thieno[3,2-d]pyrimidine (5.94g, 15.95mmol) was used instead of 4-([1,1'-biphenyl]-4-yl)-6-chloro-2-phenylpyrimidine used in Synthesis Example 9.
[LCMS] . 733

### [Synthesis Example 13] Synthesis of Compound Inv 254

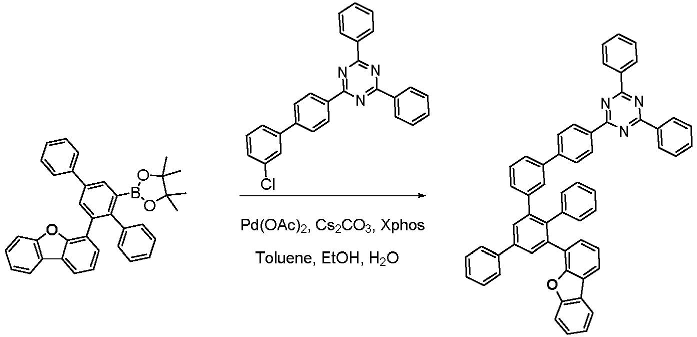

Compound Inv 254 (7.5 g, yield: 60 %) was obtained by performing the same method as in [Synthesis Example 1] except that Core 6 (10 g, 19.14mmol) and 2-(3'-chloro-[1,1'-biphenyl]-4-yl)-4,6-diphenyl-1,3,5-triazine (6.69 g, 15.95 mmol) were respectively used instead of Core 1 and 4-([1,1'-biphenyl]-4-yl)-6-(4-chlorophenyl)-2-phenylpyrimidine used in Synthesis Example 1.
[LCMS] . 780

### [Synthesis Example 14] Synthesis of Compound Inv 263

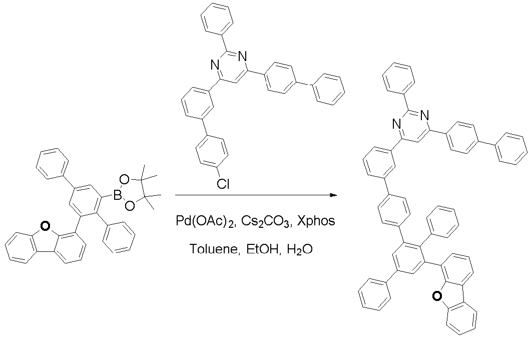

Compound Inv 263 (841 g, yield: 61 %) was obtained by performing the same method as in [Synthesis Example 13] except that 4-([1,1'-biphenyl]-4-yl)-6-(4'-chloro-[1,1'-biphenyl]-3-yl)-2-phenylpyrimidine (7.89 g, 15.95 mmol) was used instead of 2-(3'-chloro-[1,1'-biphenyl]-4-yl)-4,6-diphenyl-1,3,5-triazine used in Synthesis Example 13.
[LCMS] . 856

### [Synthesis Example 15] Synthesis of Compound Inv 269

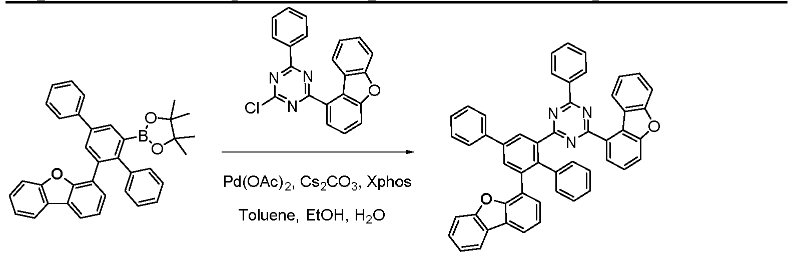

Compound Inv 269 (7.3 g, yield: 63 %) was obtained by performing the same method as in [Synthesis Example 13] except that 2-chloro-4-(dibenzo[b,d]furan-1-yl)-6-phenyl-1,3,5-triazine (5.7 g, 15.95 mmol) was used instead of 2-(3'-chloro-[1,1'-biphenyl]-4-yl)-4,6-diphenyl-1,3,5-triazine used in Synthesis Example 13.
[LCMS] . 718

### [Synthesis Example 16] Synthesis of Compound Inv 280

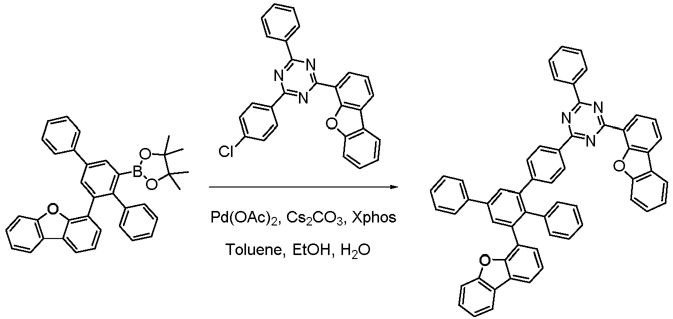

Compound Inv 280 (7.9 g, yield: 62 %) was obtained by performing the same method as in [Synthesis Example 13] except that 2-(4-chlorophenyl)-4-(dibenzo[b,d]furan-4-yl)-6-phenyl-1,3,5-triazine (6.92 g, 15.95 mmol) was used instead of 2-(3'-chloro-[1,1'-biphenyl]-4-yl)-4,6-diphenyl-1,3,5-triazine used in Synthesis Example 13.
[LCMS] . 794

### [Synthesis Example 17] Synthesis of Compound Inv 295

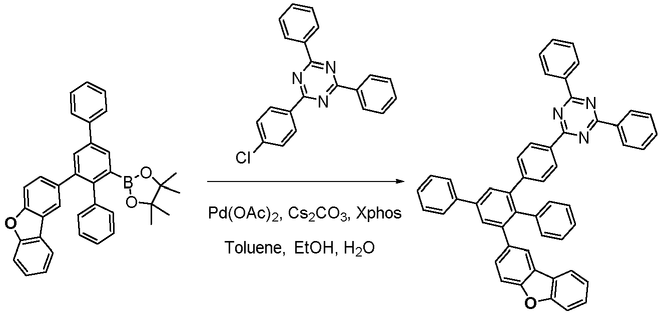

Compound Inv 295 (7.4 g, yield: 66 %) was obtained by performing the same method as in [Synthesis Example 1] except that Core 7 (10 g, 19.14mmol) and 2-(4-chlorophenyl)-4,6-diphenyl-1,3,5-triazine (5.48 g, 15.95 mmol) were respectively used instead of Core 1 and 4-([1,1'-biphenyl]-4-yl)-6-(4-chlorophenyl)-2-phenylpyrimidine used in Synthesis Example 1.
[LCMS] . 704

### [Synthesis Example 18] Synthesis of Compound Inv 296

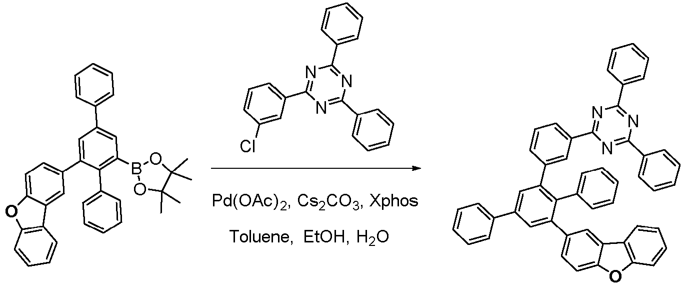

Compound Inv 296 (7.3 g, yield: 65 %) was obtained by performing the same method as in [Synthesis Example 17] except that 2-(3-chlorophenyl)-4,6-diphenyl-1,3,5-triazine (5.48 g, 15.95 mmol) was used instead of 2-(4-chlorophenyl)-4,6-diphenyl-1,3,5-triazine used in Synthesis Example 17.
[LCMS] . 704

### [Synthesis Example 19] Synthesis of Compound Inv 322

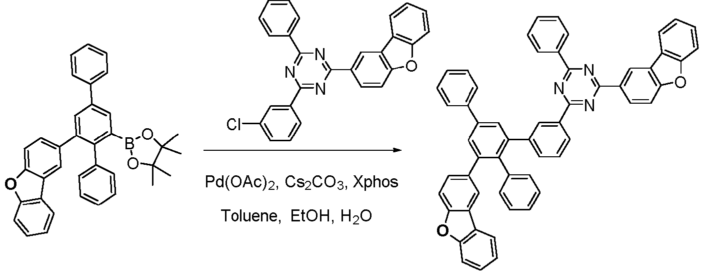

Compound Inv 322 (7.8 g, yield: 61 %) was obtained by performing the same method as in [Synthesis Example 17] except that 2-(3-chlorophenyl)-4-(dibenzo[b,d]furan-2-yl)-6-phenyl-1,3,5-triazine (6.92 g, 15.95 mmol) was used instead of 2-(4-chlorophenyl)-4,6-diphenyl-1,3,5-triazine used in Synthesis Example 17.
[LCMS] . 794

### [Synthesis Example 20] Synthesis of Compound Inv 330

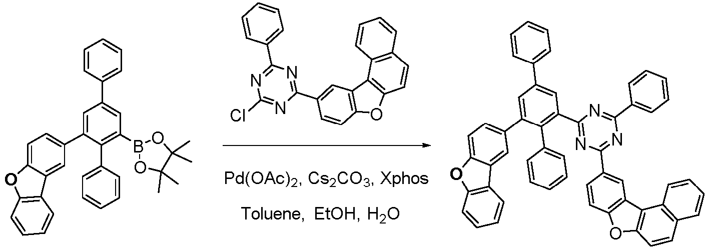

Compound Inv 330 (7.7 g, yield: 63 %) was obtained by performing the same method as in [Synthesis Example 17] except that 2-chloro-4-(naphtho[2,1-b]benzofuran-10-yl)-6-phenyl-1,3,5-triazine (6.5 g, 15.95 mmol) was used instead of 2-(4-chlorophenyl)-4,6-diphenyl-1,3,5-triazine used in Synthesis Example 17.
[LCMS] . 768

### [Synthesis Example 21] Synthesis of Compound Inv 348

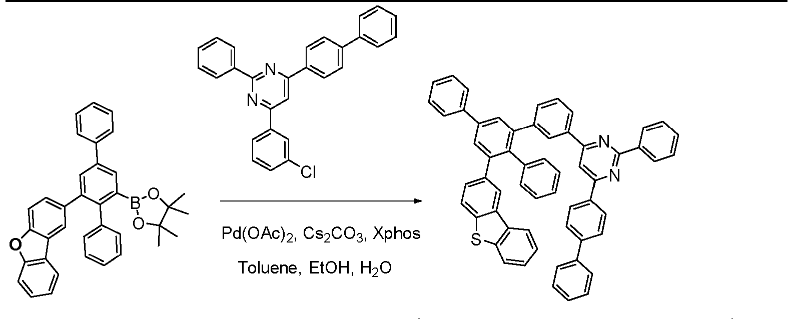

Compound Inv 348 (7.9 g, yield: 62 %) was obtained by performing the same method as in [Synthesis Example 17] except that 4-([1,1'-biphenyl]-4-yl)-6-(3-chlorophenyl)-2-phenylpyrimidine (6.68 g, 15.95 mmol) was used instead of 2-(4-chlorophenyl)-4,6-diphenyl-1,3,5-triazine used in Synthesis Example 17.
[LCMS] . 796

### [Synthesis Example 22] Synthesis of Compound Inv 352

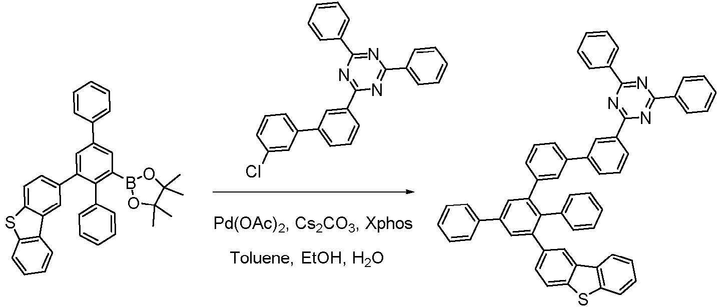

Compound Inv 352 (7.8 g, yield: 63 %) was obtained by performing the same method as in [Synthesis Example 1] except that Core 8 (10 g, 18.56 mmol) and 2-(3'-chloro-[1,1'-biphenyl]-3-yl)-4,6-diphenyl-1,3,5-triazine (6.490 g, 15.47 mmol) were respectively used instead of Core 1 and 4-([1,1'-biphenyl]-4-yl)-6-(4-chlorophenyl)-2-phenylpyrimidine used in Synthesis Example 1.
[LCMS] . 797

### [Synthesis Example 23] Synthesis of Compound Inv 368

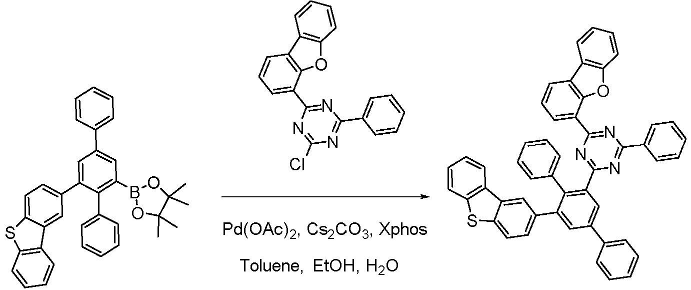

Compound Inv 368 (7.2 g, yield: 63 %) was obtained by performing the same method as in [Synthesis Example 22] except that 2-chloro-4-(dibenzo[b,d]furan-4-yl)-6-phenyl-1,3,5-triazine (5.53 g, 15.47 mmol) was used instead of 4-([1,1'-biphenyl]-4-yl)-6-(4-chlorophenyl)-2-phenylpyrimidine used in Synthesis Example 22.
[LCMS] . 734

### [Synthesis Example 24] Synthesis of Compound Inv 382

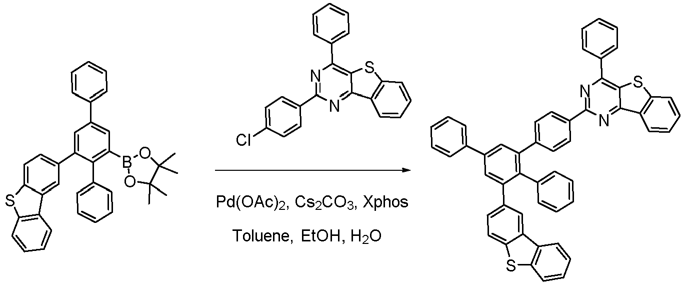

Compound Inv 382 (7.0 g, yield: 60 %) was obtained by performing the same method as in [Synthesis Example 22] except that 2-(4-chlorophenyl)-4-phenylbenzo[4,5]thieno[3,2-d]pyrimidine (5.77 g, 15.47 mmol) was used instead of 4-([1,1'-biphenyl]-4-yl)-6-(4-chlorophenyl)-2-phenylpyrimidine used in Synthesis Example 22.
[LCMS] . 749

### [Synthesis Example 25] Synthesis of Compound Inv 385

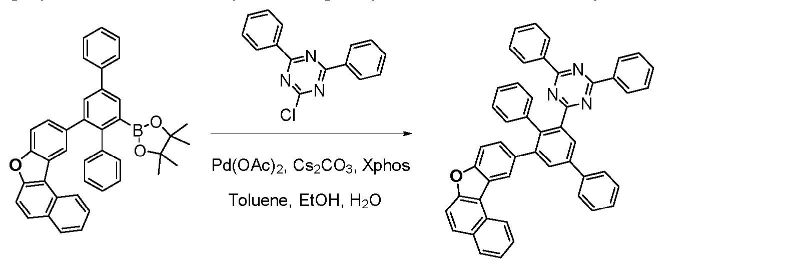

Compound Inv 385 (6.5 g, yield: 65 %) was obtained by performing the same method as in [Synthesis Example 1] except that Core 9 (10 g, 17.46 mmol) and 2-chloro-4,6-diphenyl-1,3,5-triazine (3.89 g, 14.55 mmol) were respectively used instead of Core 1 and 4-([1,1'-biphenyl]-4-yl)-6-(4-chlorophenyl)-2-phenylpyrimidine used in Synthesis Example 1.
[LCMS] . 678

### [Synthesis Example 26] Synthesis of Compound Inv 396

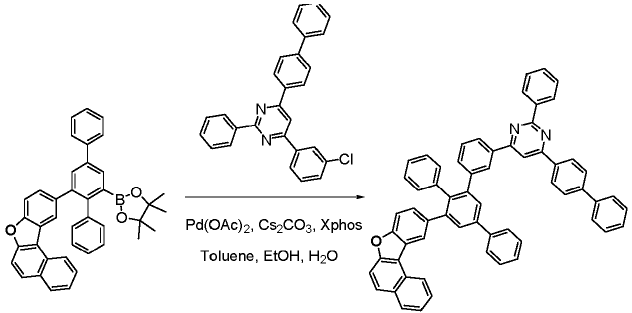

Compound Inv 396 (7.6 g, yield: 63 %) was obtained by performing the same method as in [Synthesis Example 25] except that 4-([1,1'-biphenyl]-4-yl)-6-(3-chlorophenyl)-2-phenylpyrimidine (6.09 g, 14.55 mmol) was used instead of 2-chloro-4,6-diphenyl-1,3,5-triazine used in Synthesis Example 25.
[LCMS] . 830

### [Synthesis Example 27] Synthesis of Compound Inv 414

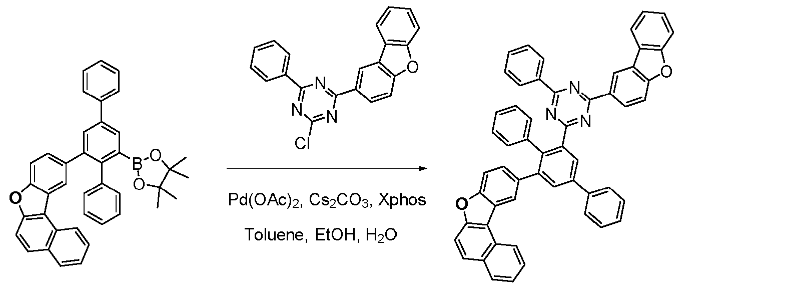

Compound Inv 414 (7.1 g, yield: 63 %) was obtained by performing the same method as in [Synthesis Example 25] except that 2-chloro-4-(dibenzo[b,d]furan-2-yl)-6-phenyl-1,3,5-triazine (5.2 g, 14.55 mmol) was used instead of 2-chloro-4,6-diphenyl-1,3,5-triazine used in Synthesis Example 25.
[LCMS] . 768

### [Synthesis Example 28] Synthesis of Compound Inv 439

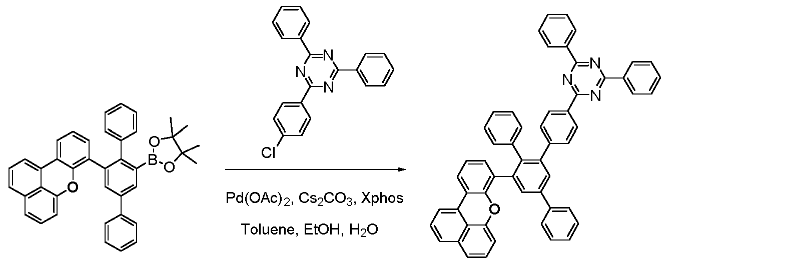

Compound Inv 439 (7.0 g, yield: 64 %) was obtained by performing the same method as in [Synthesis Example 1] except that Core 10 (10 g, 17.46 mmol) and 2-(4-chlorophenyl)-4,6-diphenyl-1,3,5-triazine (5.0 g, 14.55 mmol) were respectively used instead of Core 1 and 4-([1,1'-biphenyl]-4-yl)-6-(4-chlorophenyl)-2-phenylpyrimidine used in Synthesis Example 1.
[LCMS] . 754

### [Synthesis Example 29] Synthesis of Compound Inv 442

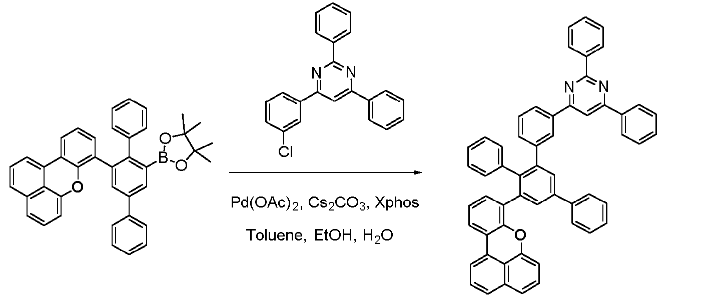

Compound Inv 442 (6.9 g, yield: 63 %) was obtained by performing the same method as in [Synthesis Example 28] except that 4-(3-chlorophenyl)-2,6-diphenylpyrimidine (4.99 g, 14.55 mmol) was used instead of 2-(4-chlorophenyl)-4,6-diphenyl-1,3,5-triazine used in Synthesis Example 28.
[LCMS] . 753

### [Synthesis Example 30] Synthesis of Compound Inv 477

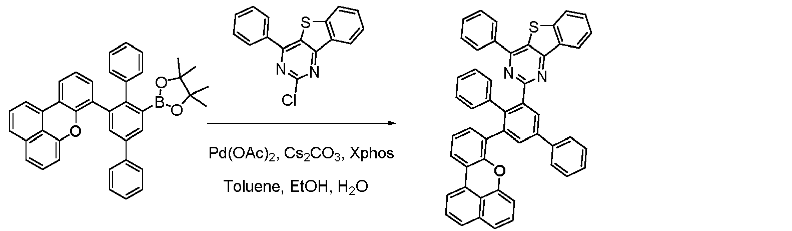

Compound Inv 477 (6.6 g, yield: 64 %) was obtained by performing the same method as in [Synthesis Example 28] except that 2-chloro-4-phenylbenzo[4,5]thieno[3,2-d]pyrimidine (4.32 g, 14.55 mmol) was used instead of 2-(4-chlorophenyl)-4,6-diphenyl-1,3,5-triazine used in Synthesis Example 28.
[LCMS] . 707

### [Example 1]- Manufacture of blue organic electroluminescent device

Compound Inv 121 was subjected to high purity sublimation purification by a typically known method, and then a blue organic electroluminescent device was manufactured as follows.

First, a glass substrate on which a thin film of indium tin oxide (ITO) was coated with a thickness of 1500 Å was washed with distilled water under ultrasonic wave. After washed with distilled water, the glass substrate was ultrasonically washed with a solvent, such as isopropyl alcohol, acetone, or methanol, dried, and then transferred to a UV Ozone cleaner (Power sonic 405, Hwashin Tech), and subsequently, the substrate was cleaned for 5 minutes by using UV and transferred to a vacuum deposition system.

On the ITO transparent electrode thus prepared, DS-205 (Solus Advanced Materials, 80 nm) / NPB (15 nm) / ADN + 5% DS-405 (Solus Advanced Materials, 30 nm) / compound Inv 121 (30 nm) / LiF (1 nm) / Al (200 nm) were deposited in this order to manufacture an organic electroluminescent device.

The structures of NPB and ADN used are as follows.

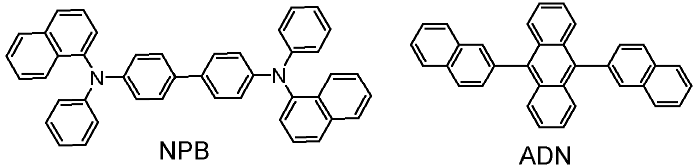

### [Examples 2 to 10]- Manufacture of organic electroluminescent devices

Blue organic electroluminescent devices were manufactured in the same manner as in Example 1 except that electron transport layer materials on Table 1 were respectively used instead of Compound Inv 121 used as an electron transport layer material.

### [Comparative Example 1]- Manufacture of blue organic electroluminescent device

A blue organic electroluminescent device was manufactured in the same manner as in Example 1 except that Alq₃ was used instead of Compound Inv 121 used as an electron transport layer material. The structure of Alq₃ used are as follows.

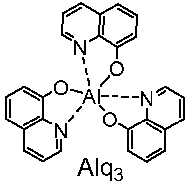

### [Comparative Example 2 to 5]- Manufacture of blue organic electroluminescent devices

Blue organic electroluminescent devices of Comparative Examples 2 to 5 were manufactured in the same manner as in Example 1 except that Compounds A to D below were respectively used instead of Compound Inv 121 used as an electron transport layer material. The structures of Compounds A to D used in Comparative Examples 2 to 5 are as follows.

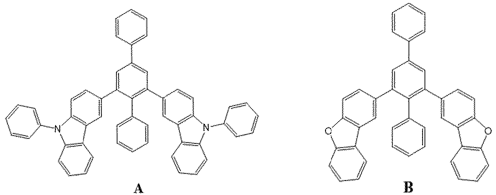

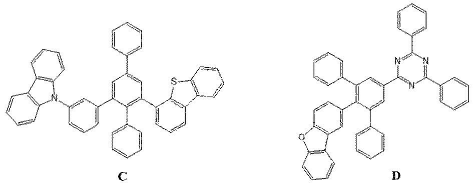

### [Evaluation Example 1]

Each of the blue organic electroluminescent devices manufactured in Examples 1 to 10 and Comparative Examples 1 to 5 was measured for driving voltage, current efficiency, and light emission peak at a current density of 10 mA/cm², and the results are shown in Table 1 below.

**TABLE 1**

| Sample | Electron transport layer material | Driving voltage (V) | EL peak (nm) | Current efficiency (cd/A) |
|---|---|---|---|---|
| Example 1 | Inv 121 | 4.0 | 459 | 7.6 |
| Example 2 | Inv 127 | 3.8 | 458 | 7.8 |
| Example 3 | Inv 189 | 3.7 | 458 | 7.4 |
| Example 4 | Inv 196 | 3.8 | 459 | 7.2 |
| Example 5 | Inv 220 | 3.6 | 458 | 8.0 |
| Example 6 | Inv 224 | 3.9 | 459 | 7.6 |
| Example 7 | Inv 269 | 4.0 | 458 | 7.4 |
| Example 8 | Inv 330 | 3.6 | 459 | 7.6 |
| Example 9 | Inv 368 | 3.7 | 459 | 7.2 |
| Example 10 | Inv 385 | 4.1 | 458 | 7.8 |
| Comparative Example 1 | Alq₃ | 4.8 | 460 | 5.8 |
| Comparative Example 2 | A | 5.2 | 456 | 5.4 |
| Comparative Example 3 | B | 4.6 | 454 | 5.2 |
| Comparative Example 4 | C | 5.0 | 458 | 5.5 |
| Comparative Example 5 | D | 4.6 | 459 | 6.8 |

As shown in Table 1, the blue organic electroluminescent devices of Examples 1 to 10 using the compounds of the present invention for electron transport layers exhibited excellent performance in terms of the driving voltage, light emission peak, and current efficiency, compared to the blue organic electroluminescent device of Comparative Example 1 using Alq₃, a conventional electron transport layer material.

Additionally, the blue organic electroluminescent devices of Examples 1 to 10 had low driving voltages and high current efficiencies compared with the blue organic electroluminescent devices of Comparative Examples 2 to 5 respectively containing Compounds A to D with similar structures to the compounds of the present invention.

### [Example 11]- Manufacture of blue organic electroluminescent device

Compound Inv 11 was subjected to high purity sublimation purification by a typically known method, and then a blue organic electroluminescent device was manufactured as follows.

First, a glass substrate on which a thin film of indium tin oxide (ITO) was coated with a thickness of 1500 Å was washed with distilled water under ultrasonic wave. After washed with distilled water, the glass substrate was ultrasonically washed with a solvent, such as isopropyl alcohol, acetone, or methanol, dried, and then transferred to a UV Ozone cleaner (Power sonic 405, Hwashin Tech), and subsequently, the substrate was cleaned for 5 minutes by using UV and transferred to a vacuum deposition system.

On the ITO transparent electrode thus prepared, DS-205 (80 nm) / NPB (15 nm) / ADN + 5% DS-405 (Solus Advanced Materials, 30 nm) / compound Inv 11 (30 nm) / Alq₃ (25 nm) / LiF (1 nm) / Al (200 nm) were deposited in this order to manufacture an organic electroluminescent device.

The structures of NPB, ADN, and Alq₃ used are as follows.

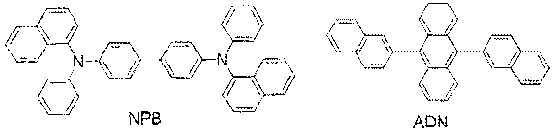

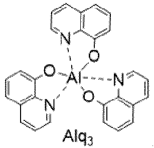

### [Examples 12 to 40]- Manufacture of blue organic electroluminescent devices

Blue organic electroluminescent devices were manufactured in the same manner as in Example 11 except that electron transport auxiliary layer materials on Table 2 were respectively used instead of Compound Inv 11 used as an electron transport auxiliary layer material.

### [Comparative Example 6]- Manufacture of blue organic electroluminescent device

A blue organic electroluminescent device was manufactured in the same manner as in Example 11, except that Compound Inv 11 used as an electron transport auxiliary layer material in Example 11 was not used and the electron transport layer material Alq₃ was deposited with a thickness of 30 nm instead of 25 nm.

### [Comparative Examples 7 to 10]- Manufacture of blue organic electroluminescent devices

Blue organic electroluminescent devices of Comparative Examples 7 to 10 were manufactured in the same manner as in Example 11 except that Compounds A to D were respectively used instead of Compound Inv 11 used as an electron transport auxiliary layer material. The structures of Compounds A to D used are as described in Comparative Examples 2 to 5.

### [Evaluation Example 2]

Each of the blue organic electroluminescent devices manufactured in Examples 11 to 40 and Comparative Examples 6 to 10 was measured for driving voltage, light emission wavelength, current efficiency, and light emission wavelength at a current density of 10 mA/cm², and the results are shown in Table 2 below.

**TABLE 2**

| Sample | Electron transport auxiliary material | Driving voltage (V) | Current efficiency (cd/A) | EL peak (nm) |
|---|---|---|---|---|
| Example 11 | Inv 11 | 4.0 | 7.8 | 458 |
| Example 12 | Inv 34 | 3.8 | 7.7 | 458 |
| Example 13 | Inv 55 | 3.9 | 7.6 | 457 |
| Example 14 | Inv 60 | 3.9 | 7.8 | 457 |
| Example 15 | Inv 121 | 4.2 | 7.6 | 458 |
| Example 16 | Inv 127 | 4.1 | 7.2 | 458 |
| Example 17 | Inv 154 | 3.6 | 8.0 | 457 |
| Example 18 | Inv 189 | 3.9 | 7.3 | 458 |
| Example 19 | Inv 196 | 4.2 | 7.4 | 458 |
| Example 20 | Inv 220 | 4.1 | 7.2 | 457 |
| Example 21 | Inv 224 | 4.2 | 7.6 | 458 |
| Example 22 | Inv 239 | 3.7 | 7.8 | 458 |
| Example 23 | Inv 254 | 4.0 | 7.6 | 457 |
| Example 24 | Inv 263 | 3.5 | 8.2 | 458 |
| Example 25 | Inv 269 | 4.0 | 7.2 | 458 |
| Example 26 | Inv 280 | 3.7 | 8.0 | 457 |
| Example 27 | Inv 295 | 3.9 | 7.8 | 458 |
| Example 28 | Inv 296 | 3.6 | 8.1 | 458 |
| Example 29 | Inv 322 | 3.9 | 7.8 | 457 |
| Example 30 | Inv 330 | 4.2 | 7.2 | 458 |
| Example 31 | Inv 348 | 3.7 | 7.6 | 457 |
| Example 32 | Inv 352 | 3.8 | 7.8 | 457 |
| Example 33 | Inv 368 | 4.0 | 7.6 | 457 |
| Example 34 | Inv 382 | 3.9 | 7.5 | 458 |
| Example 35 | Inv 385 | 3.8 | 7.6 | 458 |
| Example 36 | Inv 396 | 3.6 | 8.1 | 457 |
| Example 37 | Inv 414 | 4.2 | 7.6 | 458 |
| Example 38 | Inv 439 | 3.6 | 8.2 | 458 |
| Example 39 | Inv 442 | 3.9 | 7.8 | 457 |
| Example 40 | Inv 477 | 3.6 | 7.8 | 458 |
| Comparative Example 6 | - | 4.7 | 5.8 | 457 |
| Comparative Example 7 | A | 5.0 | 5.6 | 456 |
| Comparative Example 8 | B | 4.8 | 5.4 | 454 |
| Comparative Example 9 | C | 5.2 | 6.0 | 458 |
| Comparative Example 10 | D | 4.6 | 6.2 | 459 |

As shown in Table 2, the blue organic electroluminescent devices of Examples 11 to 40 including electron transport auxiliary layers formed of the compounds of the present invention exhibited excellent performance in terms of the driving voltage and current efficiency, compared to the blue organic electroluminescent device of Comparative Example 6 including only the electron transport layer of Alq₃ without an electron transport auxiliary layer.

Additionally, the blue organic electroluminescent devices of Examples 11 to 40 had low driving voltages and high current efficiencies, compared to the blue organic electroluminescent devices of Comparative Examples 7 to 10 respectively containing as electron transport auxiliary layer materials Compounds A to D with similar structures to the compounds of the present invention.

## Claims

1. A compound represented by Chemical Formula 1: 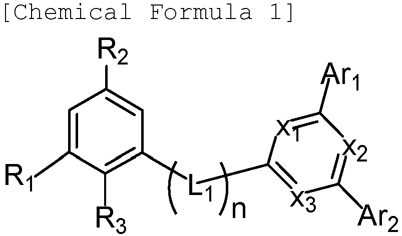 (where, in Chemical Formula 1,
X₁ to X₃ are the same as or different from each other and are each independently N or C(Ar₃), but at least one of X₁ to X₃ is N, where when C (Ar₃) is present in a plurality of numbers, a plurality of Ar₃s are the same as or different from each other,
Ar₂ to Ar₃ are the same or different from each other and are each independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkyl boron group, a C₆ to C₆₀ arylboron group, a C₆ to C₆₀ arylphosphine group, a C₆ to C₆₀ arylphosphine oxide group, and a C₆ to C₆₀ arylamine group, or bind to an adjacent group to form a fused ring;
n is an integer of 0 to 3;
L₂ is a single bond or is selected from the group consisting of a C₆ to C₆₀ arylene group and a heteroarylene group having 5 to 60 nuclear atoms; and
R₁ to R₃ are the same as or different from each other and are each independently selected from the group consisting of a C₆ to C₆₀ aryl group and a heteroaryl group having 5 to 60 nuclear atoms,
provided that at least one of R₁ to R₃ is a substituent represented by any one of Chemical Formulas 2 to 6: 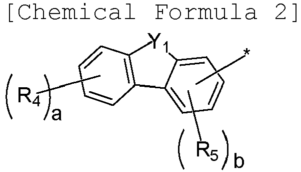 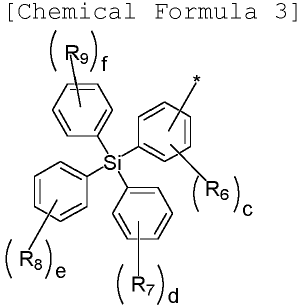 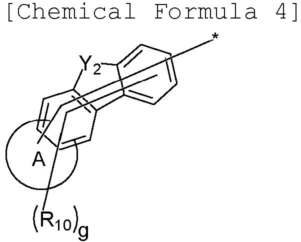 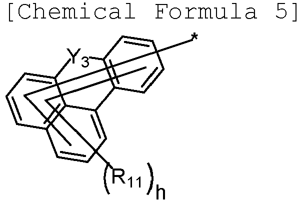 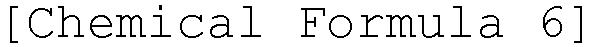 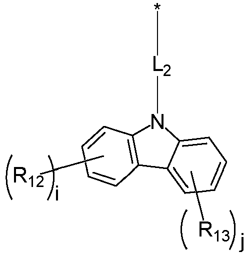 where, in Chemical Formulas 2 to 6,
Y₂ is selected from the group consisting of C(Ar₄)(Ar₅), N(Ar₆), O, and S;
Y₂ and Y₃ are the same as or different from each other and are each independently O or S;
ring A is a benzene ring;
L₂ is a single bond or is selected from the group consisting of a C₆ to C₆₀ arylene group and a heteroarylene group having 5 to 60 nuclear atoms;
a, c, i, and j are each an integer of 0 to 4;
b is an integer of 0 to 3;
d, e, and f are each an integer of 0 to 5;
g is an integer of 0 to 8;
h is an integer of 0 to 9;
Ar₄ to Ar₆ and R₄ to R₁₃ are the same as or different from each other and are each independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and
the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, arylamine group, and fused ring of Ar₂ to Ar₃, the arylene group and heteroarylene group of L₁, the aryl group and heteroaryl group of R₁ to R₃, and the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, and arylamine of Ar₄ to Ar₆ and R₄ to R₁₃ are each independently substituted or unsubstituted with at least one selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkyl boron group, a C₆ to C₆₀ arylboron group, a C₆ to C₆₀ arylphosphine group, a C₆ to C₆₀ arylphosphine oxide group, and a C₆ to C₆₀ arylamine group, and when the substituent is present in a plurality of numbers, a plurality of substituents are the same as or different from each other).

2. The compound of claim 1, wherein the substituent represented by any one of Chemical Formulas 2 to 6 is selected from the group consisting of Substituents S1-1 to S1-16.

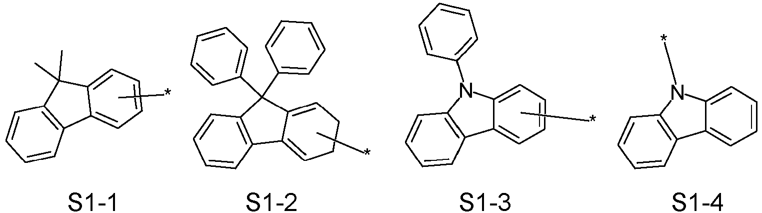

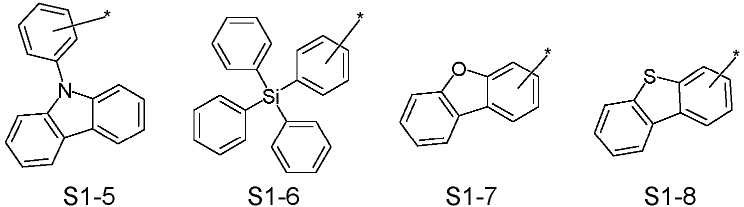

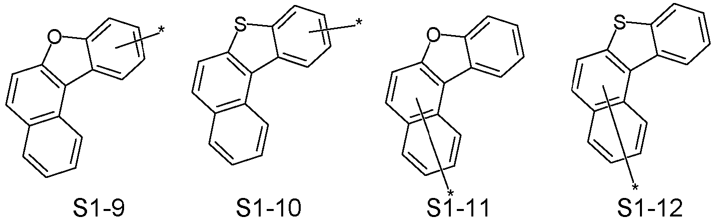

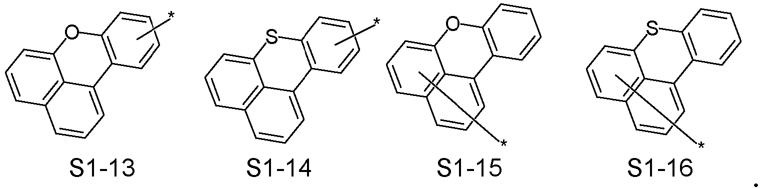

3. The compound of claim 1, wherein any one of R₁ to R₃ is a substituent represented by any one of Chemical Formulas 2 to 6, and the others are the same as or different from each other and are each independently an C₆ to C₆₀ aryl group.

4. The compound of claim 1, wherein the compound represented by Chemical Formula 1 is a compound represented by any one of Chemical Formulas 7 to 11: 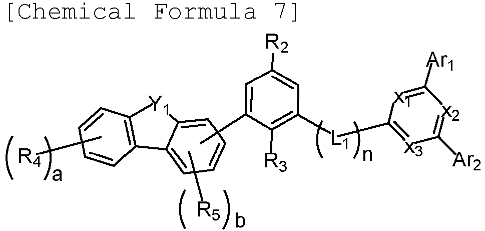 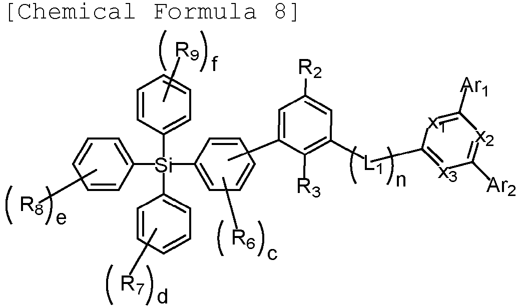 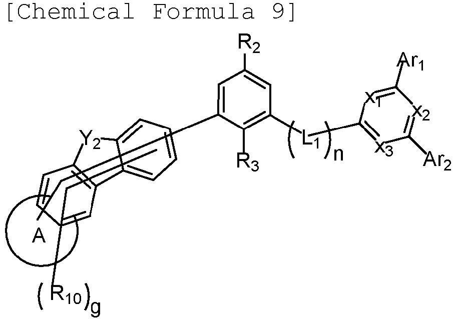 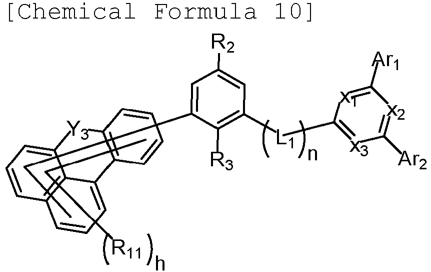 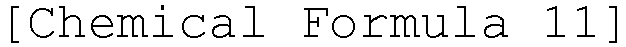 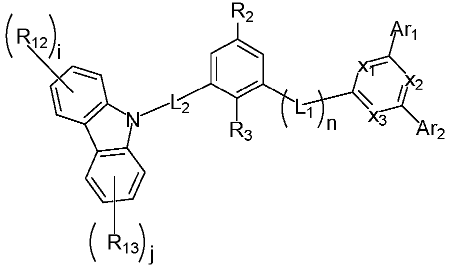 where, in Chemical Formulas 7 to 11,
R₂ to R₁₃, a, b, c, d, e, f, g, h, i, j, X₁ to X₃, Ar₂, Ar₂, n, L₂, L₂, Y₁ to Y₃, and ring A are each as defined in claim 1).

5. The compound of claim 1, wherein the compound represented by Chemical Formula 1 is a compound represented by any one of Chemical Formulas 12 to 16: 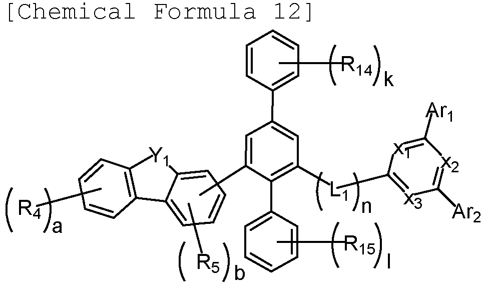 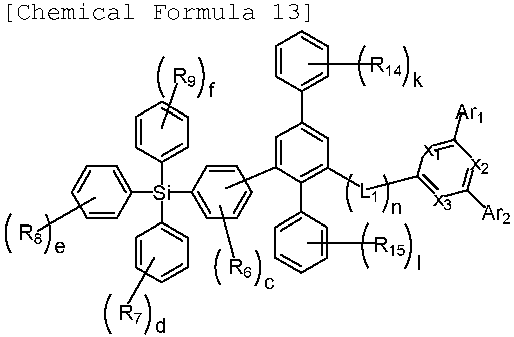 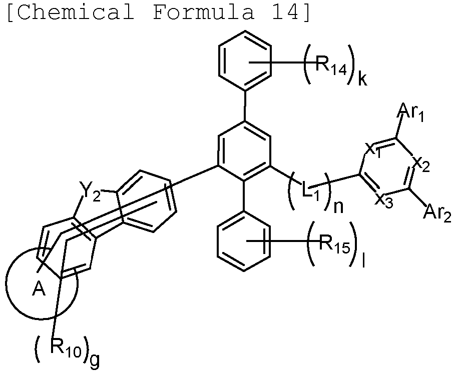 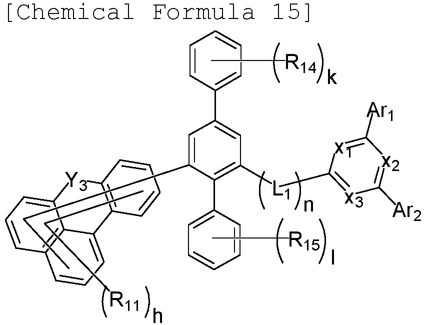 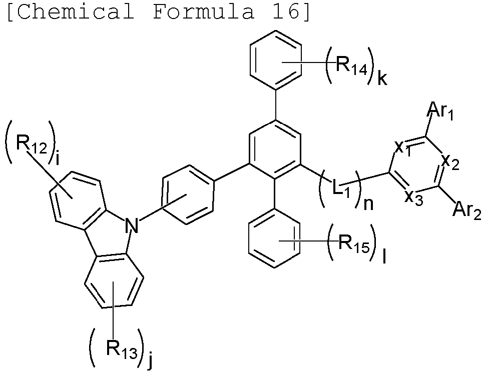 (where, in Chemical Formulas 12 to 16,
R₂ to R₁₃, a, b, c, d, e, f, g, h, i, j, X₁ to X₃, Ar₂, Ar₂, n, L₁ , Y₁ to Y₃, and ring A are each as defined in Chemical Formula 1;
k and l are each an integer of 0 to 5; and
R₁₄ to R₁₅ are the same as or different from each other and are each independently selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group) .

6. The compound of claim 1, wherein L₁ is a direct bond or is selected from the group consisting of a phenylene group, a biphenylene group, a terphenylene group, and a naphthalene group.

7. The compound of claim 1, wherein L₁ is a direct bond or is any one of Linker Groups L-1 to L-6: 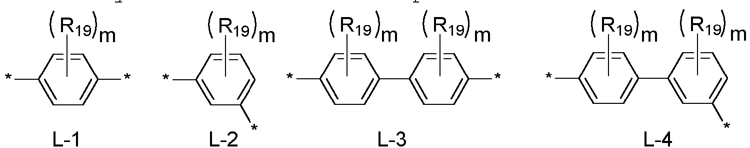 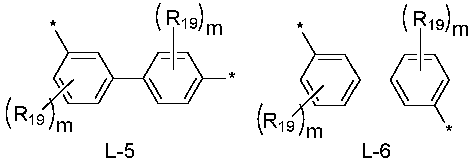 (where, in Linker Groups L-1 to L-6,
the mark * is a site linked to Chemical Formula 1;
a plurality of ms are the same as or different from each other;
m is an integer of 0 to 4;
a plurality of R₁₉s are the same as or different from each other;
R₁₉ is selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group).

8. The compound of claim 1, wherein X₁- to X₃-containing rings are each any one of Structural Formulas Az1-1 to Az1-7: 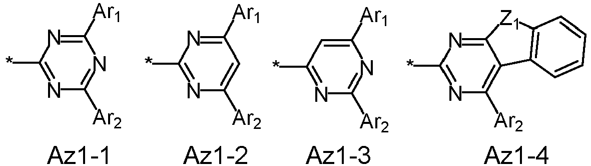 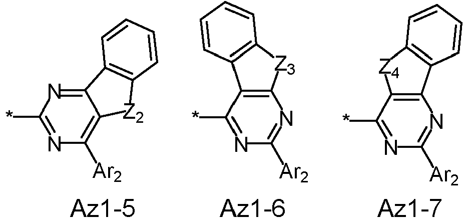 (where, in Structural Formulas Az1-1 to Az1-7,
Ar₁ and Ar₂ are the same as or different from each other and are each independently selected from the group consisting of a C₁ to C₄₀ alkyl group, a C₃ to C₄₀ cycloalkyl group, a heterocycloalkyl group having 3 to 40 nuclear atoms, a C₆ to C₆₀ aryl group, and a heteroaryl group having 5 to 60 nuclear atoms;
Z₁ to Z₄ are each selected from the group consisting of C(Ar₇)(Ar₈), N(Ar₉), O, and S;
Ar₇ to Ar₉ are the same as or different from each other and are each independently selected from the group consisting of hydrogen, deuterium, a halogen group, a cyano group, a nitro group, an amino group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₂ to C₄₀ alkylsilyl group, a C6 to C60 arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group; and
the alkyl group, cycloalkyl group, heterocycloalkyl group, aryl group, and heteroaryl group of Ar₁ and Ar₂ and the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, arylphosphine group, arylphosphine oxide group, and arylamine group of Ar₇ to Ar₉ are each independently substituted or unsubstituted with at least one selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and when the substituent is present in a plurality of numbers, a plurality of substituents are the same as or different from each other).

9. The compound of claim 1, wherein the compound represented by Chemical Formula 1 is a compound represented by any one of Chemical Formulas 17 to 21: 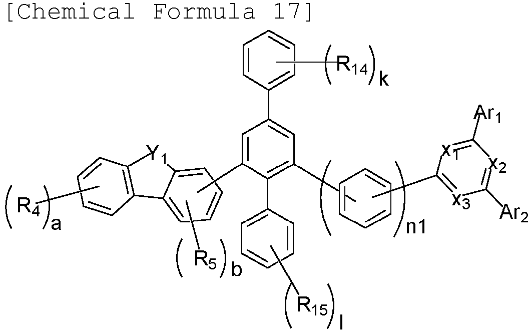 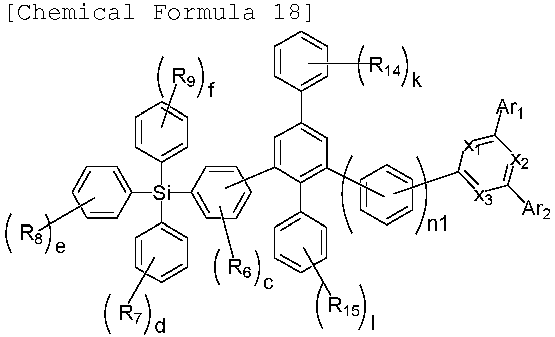 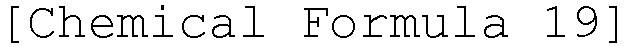 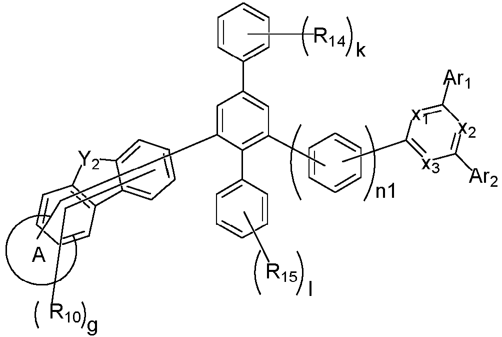 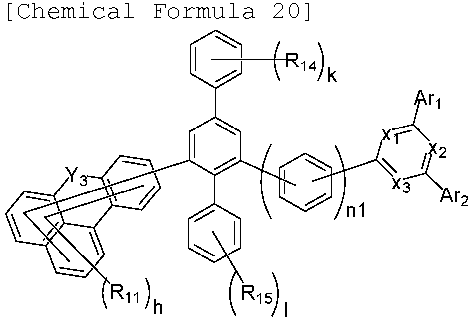 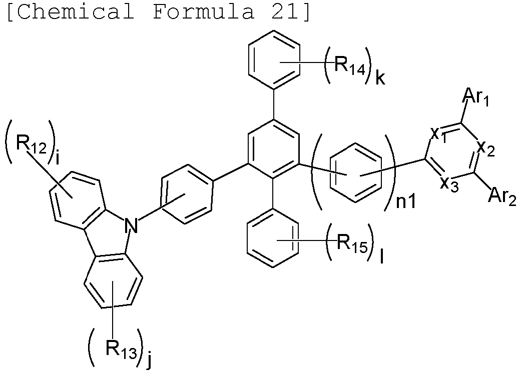 (where, in Chemical Formulas 17 to 21,
R₂ to R₁₃, a, b, c, d, e, f, g, h, i, j, X₁ to X₃, Ar₂, Ar₂, Y₁ to Y₃, and ring A are each as defined in claim 1;
n1 is an integer of 0 to 2;
k and l are each an integer of 0 to 5;
a plurality of R₁₄s are the same as or different from each other;
a plurality of R₁₅s are the same as or different from each other;
R₁₄ to R₁₅ are the same as or different from each other and are each independently selected from the group consisting of deuterium, a halogen, a cyano group, a nitro group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₁ to C₄₀ alkyl group, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group) .

10. The compound of claim 1, wherein the compound represented by Chemical Formula 1 is any one of Compounds Inv 1 to Inv 480:

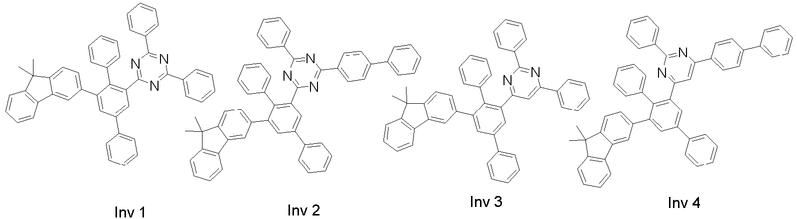

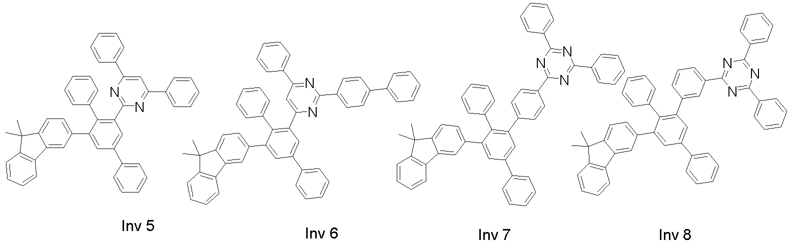

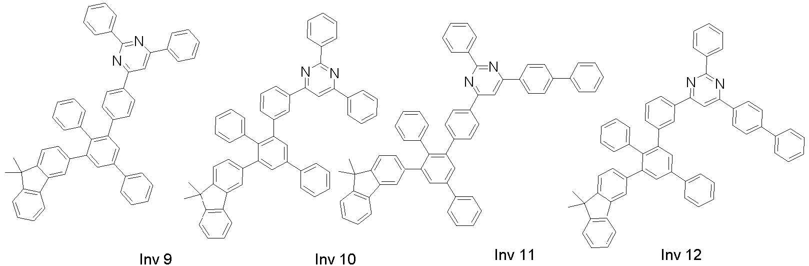

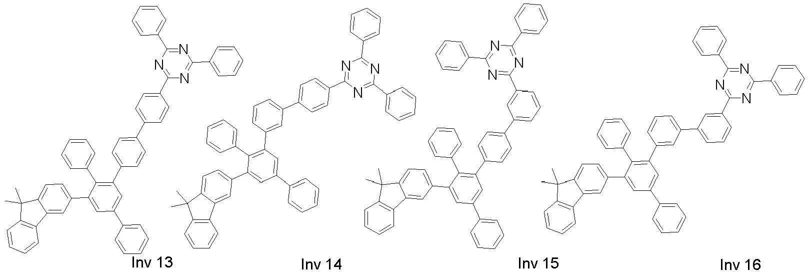

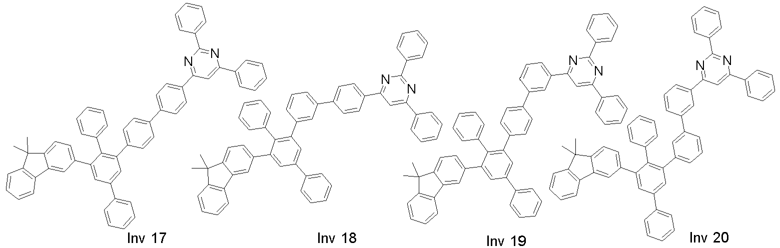

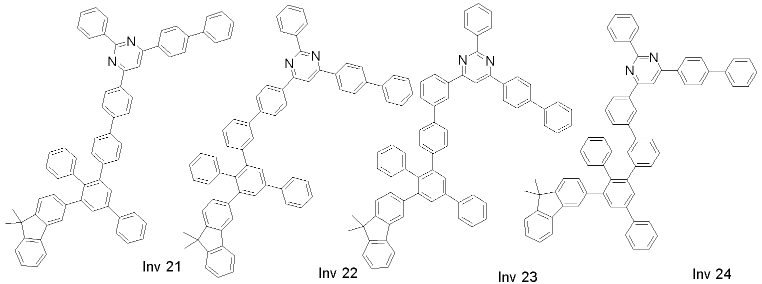

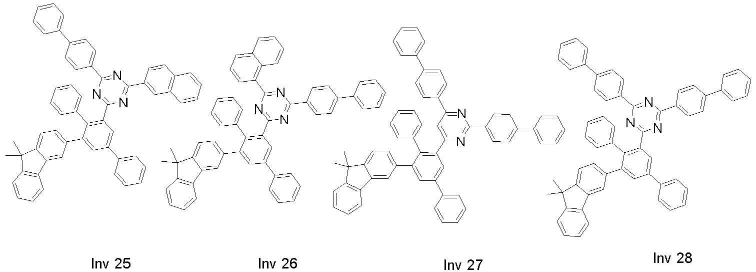

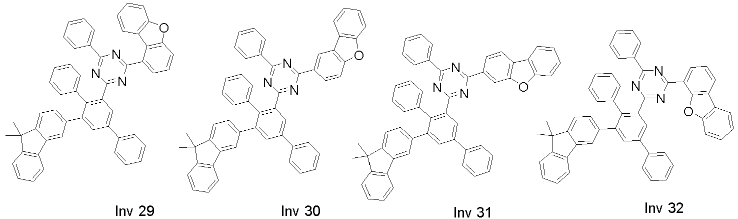

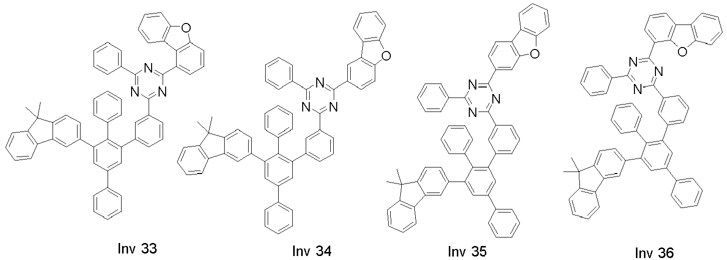

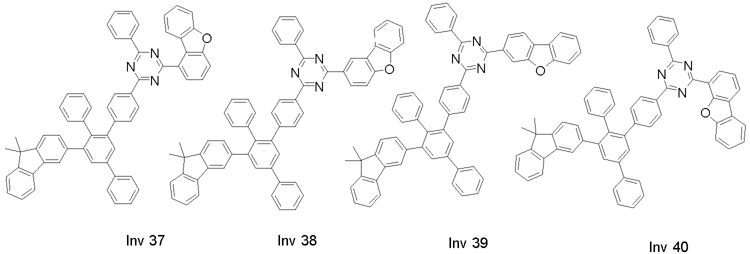

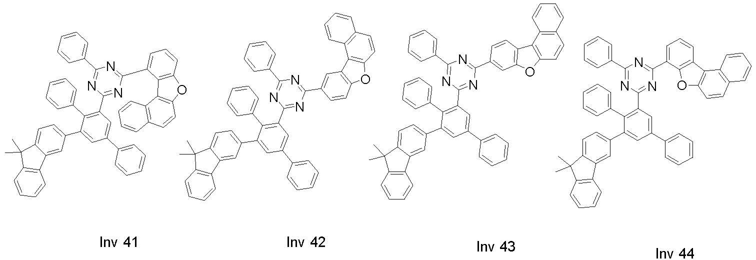

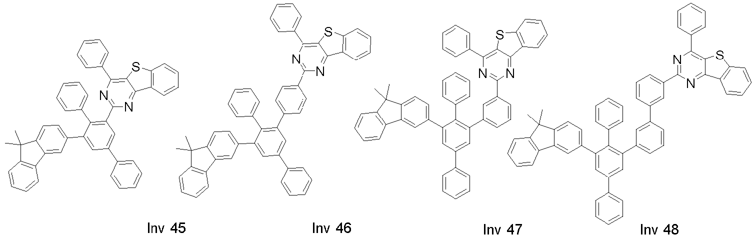

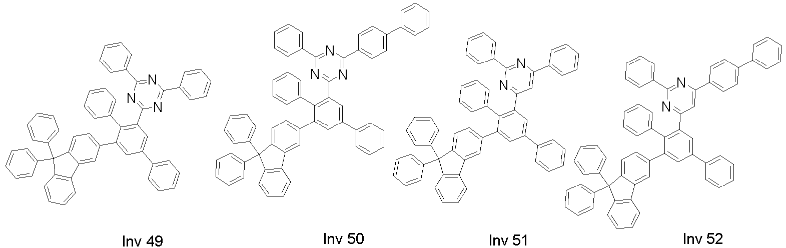

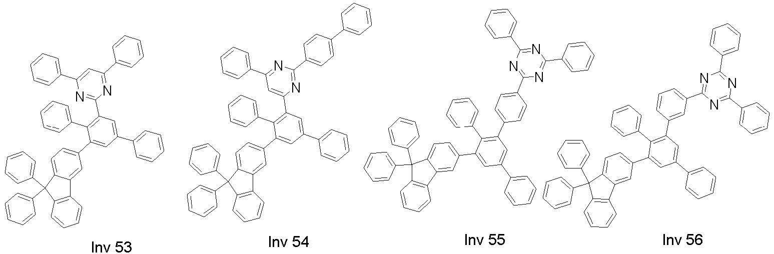

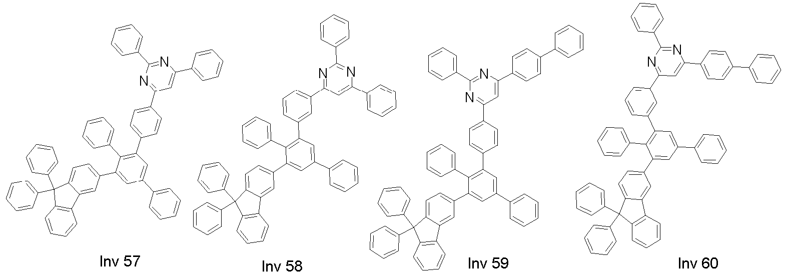

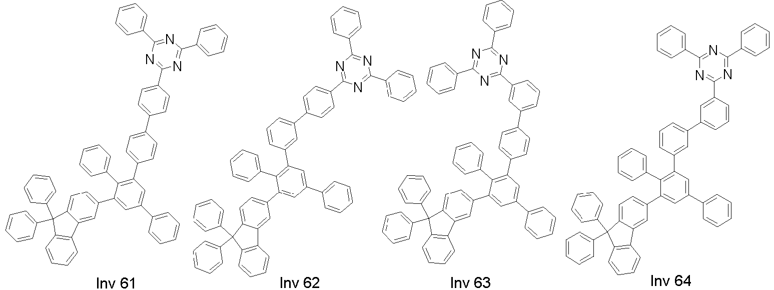

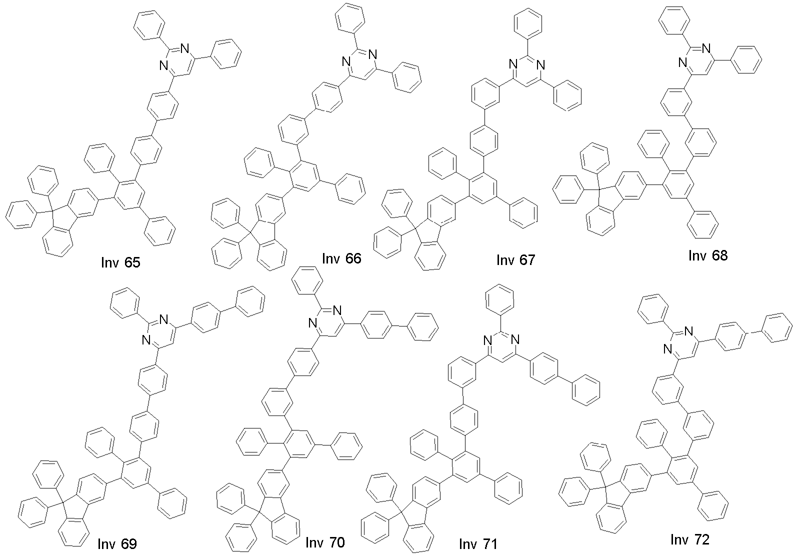

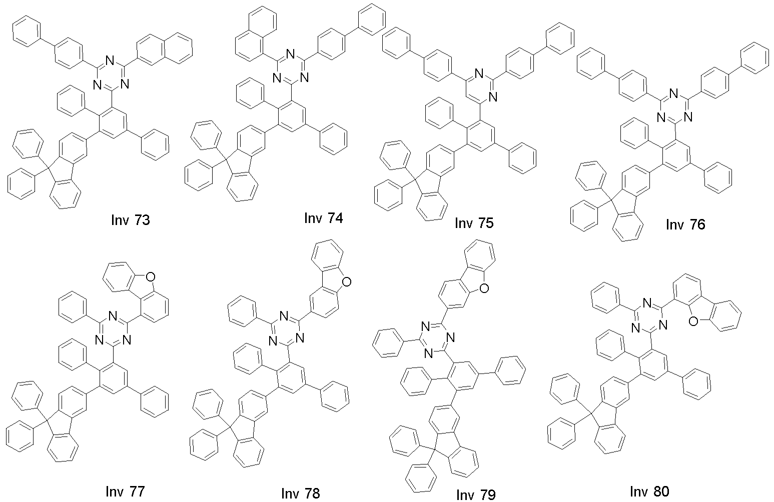

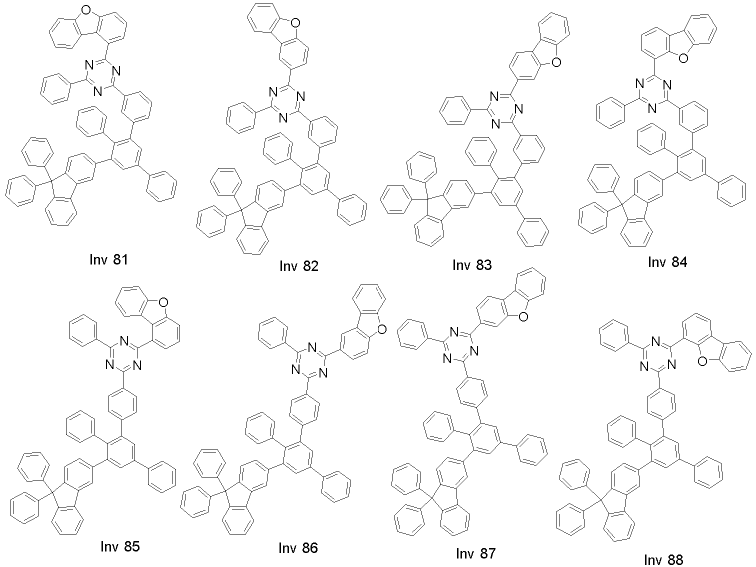

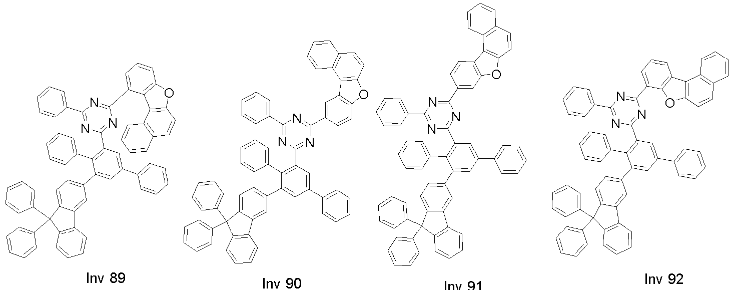

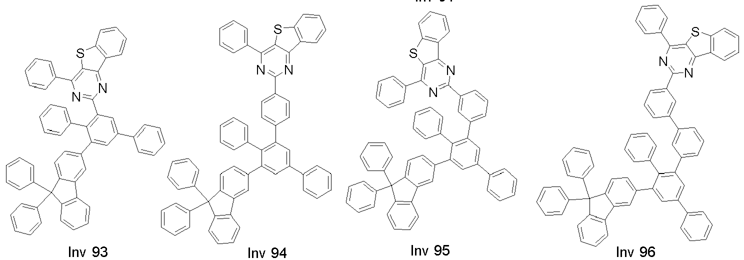

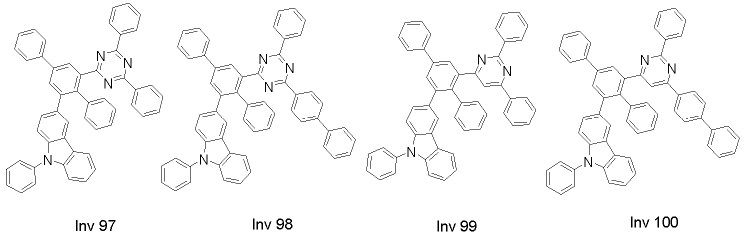

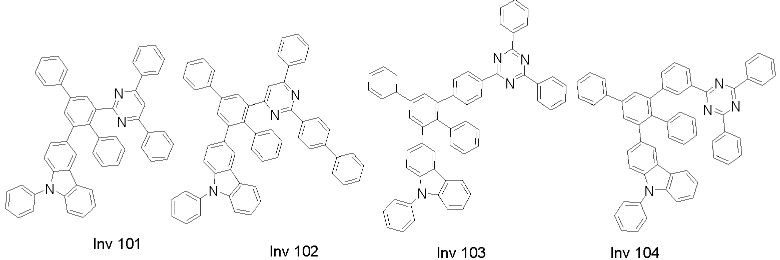

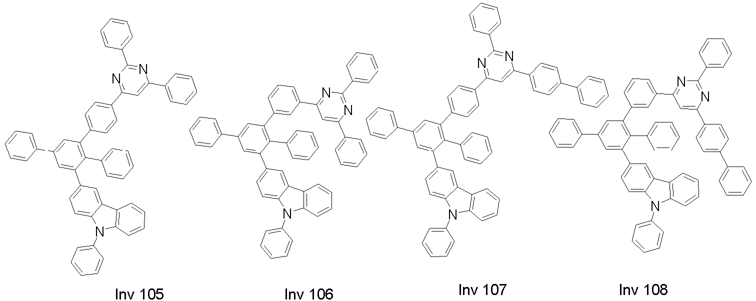

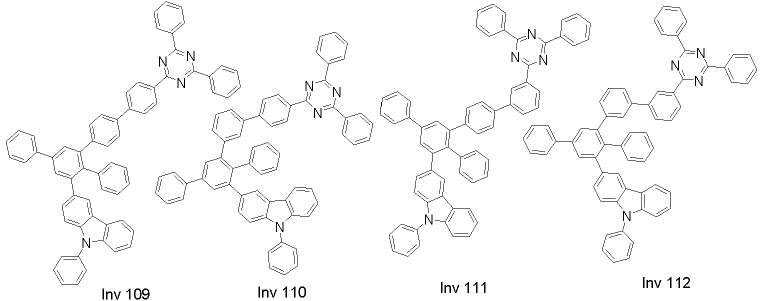

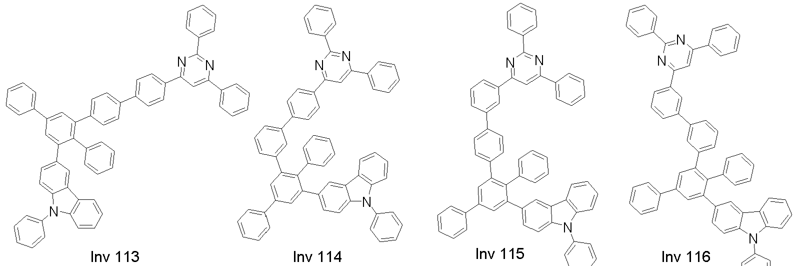

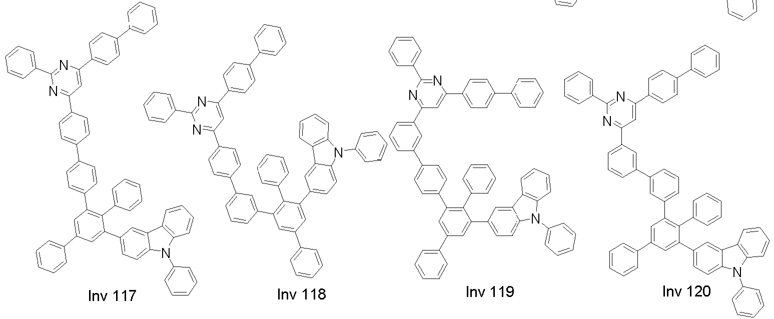

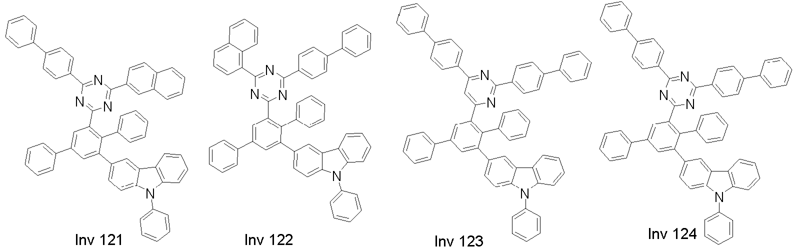

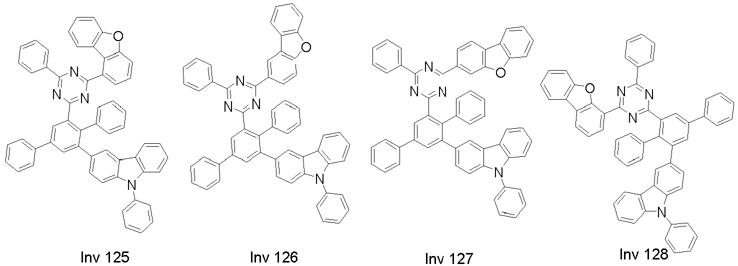

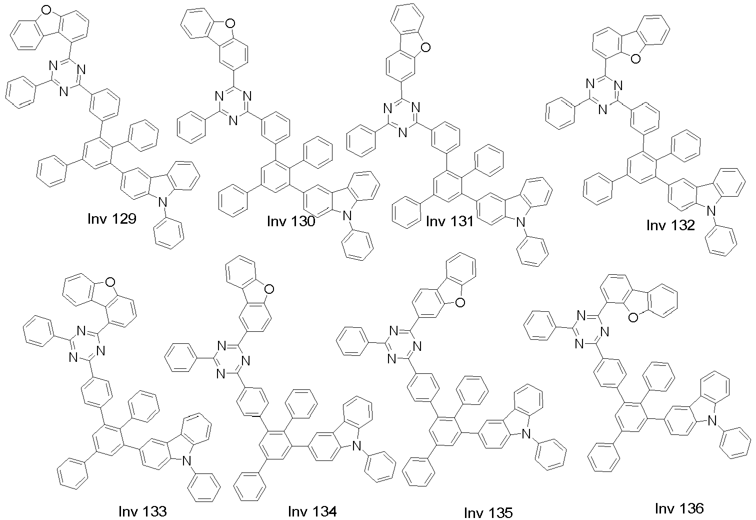

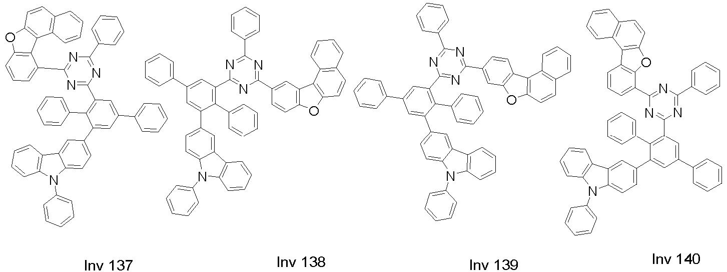

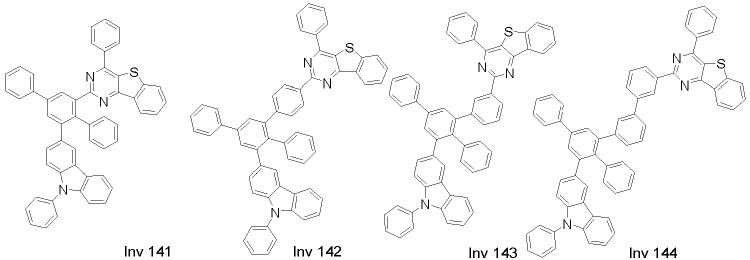

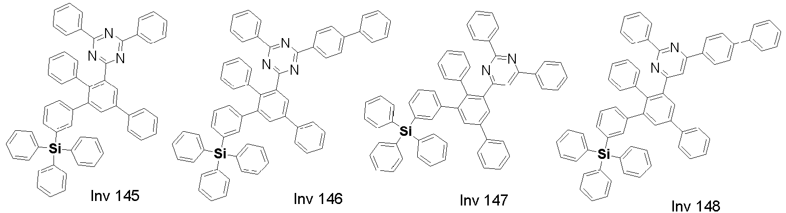

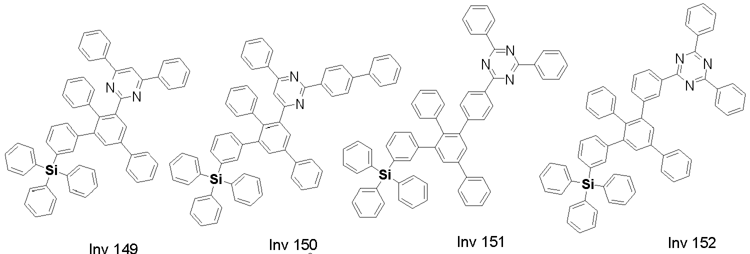

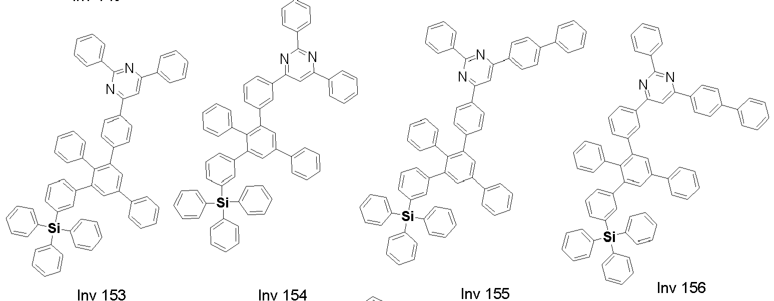

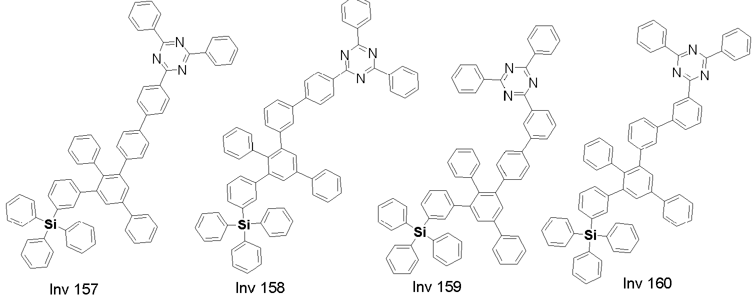

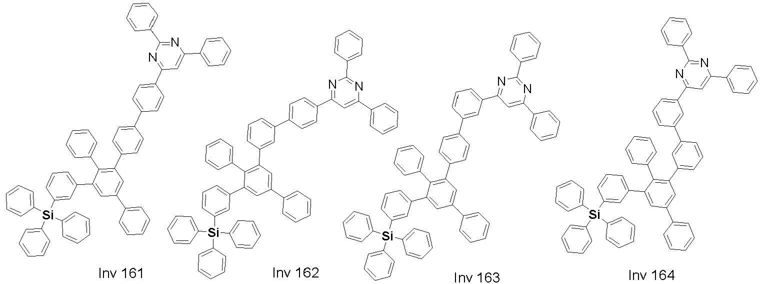

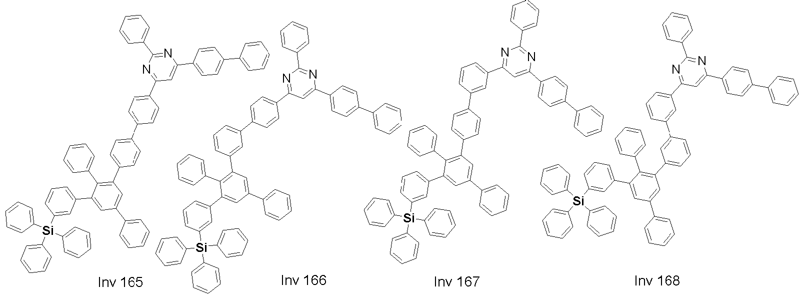

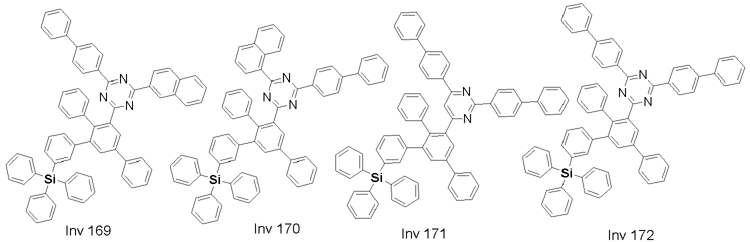

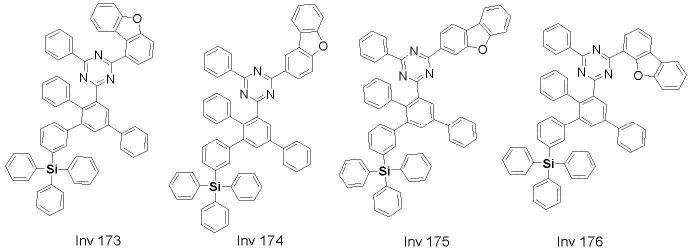

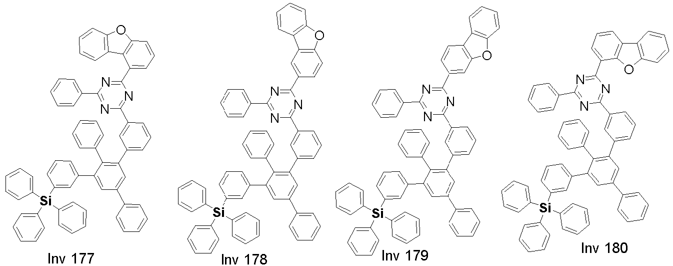

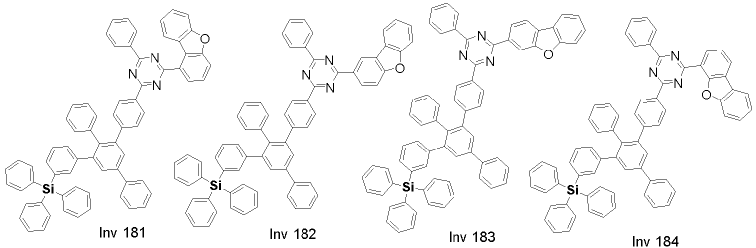

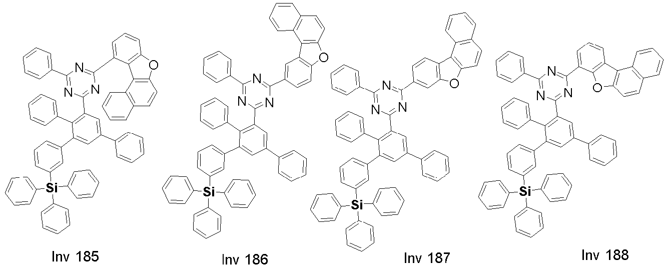

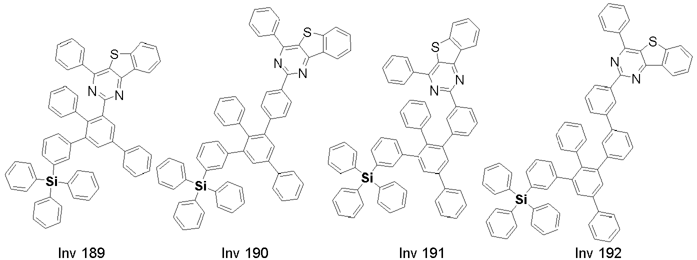

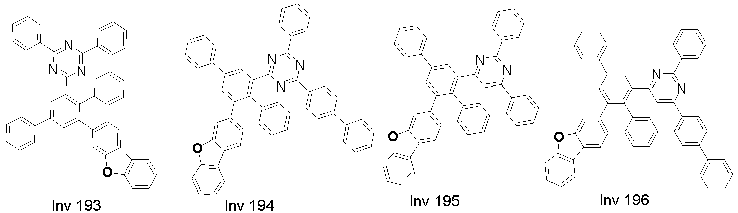

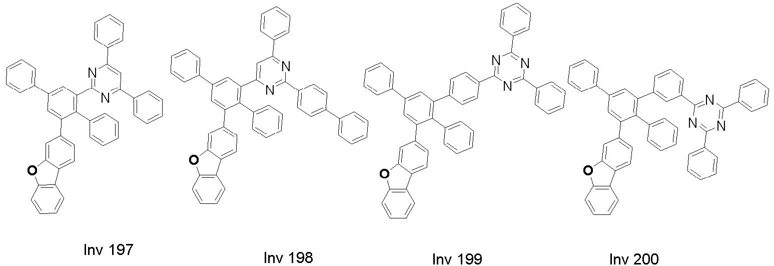

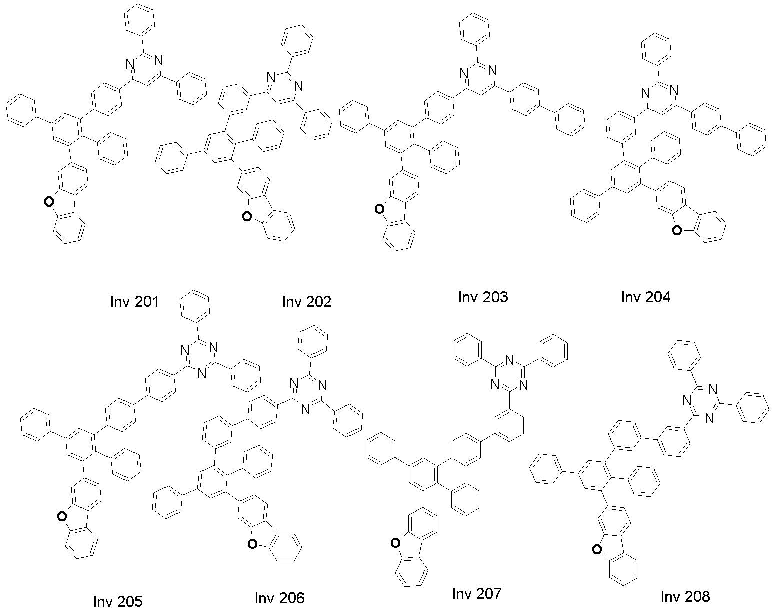

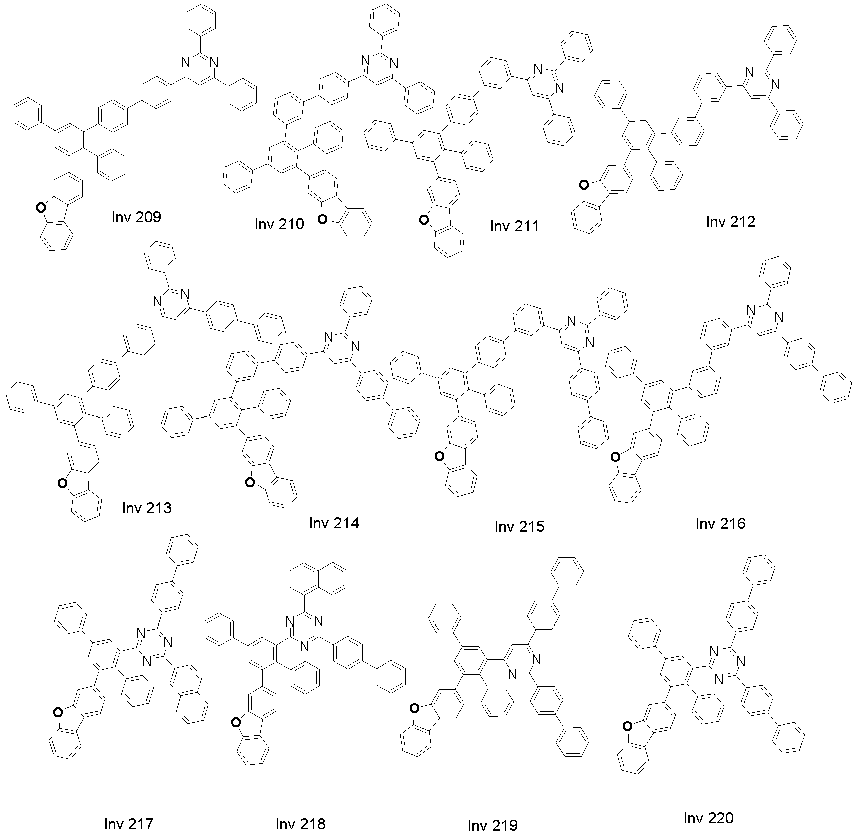

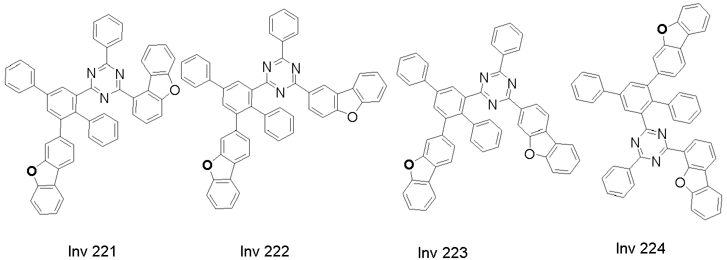

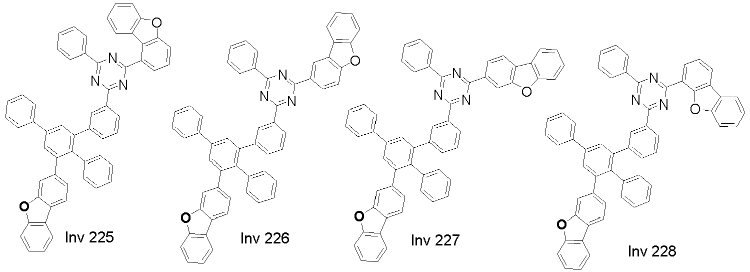

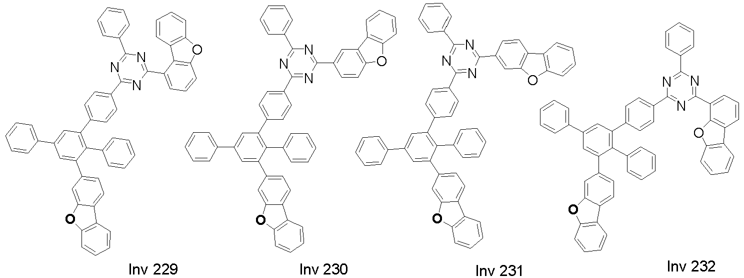

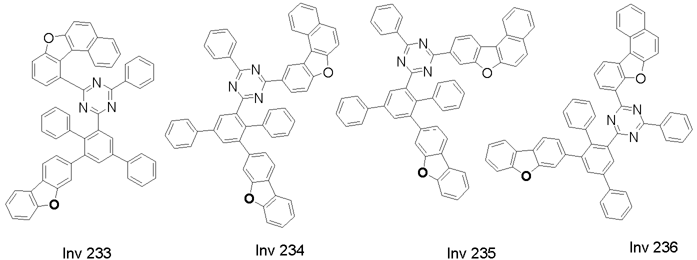

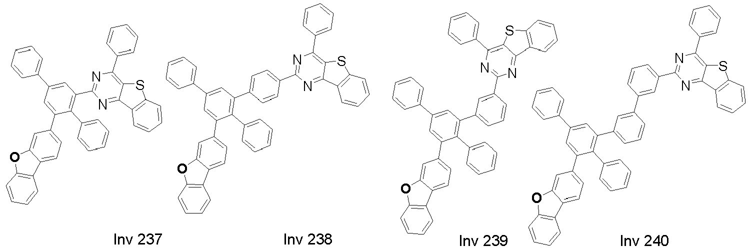

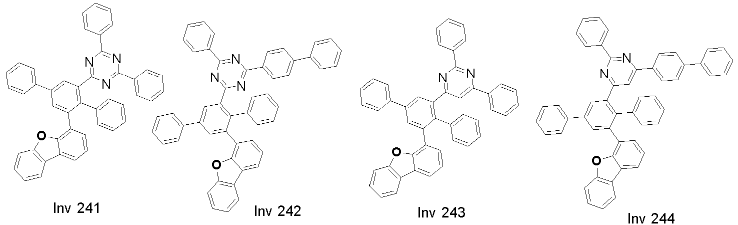

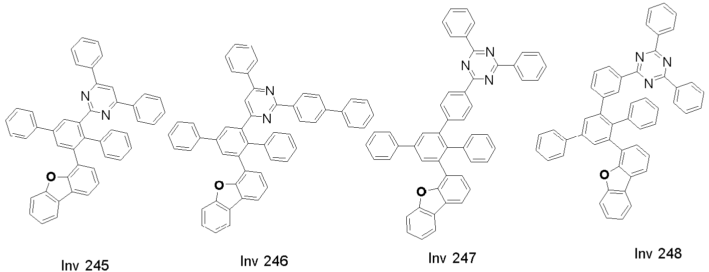

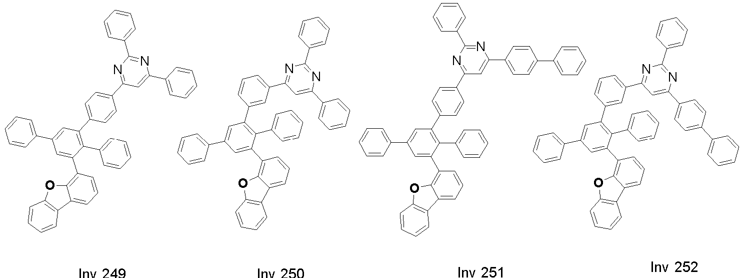

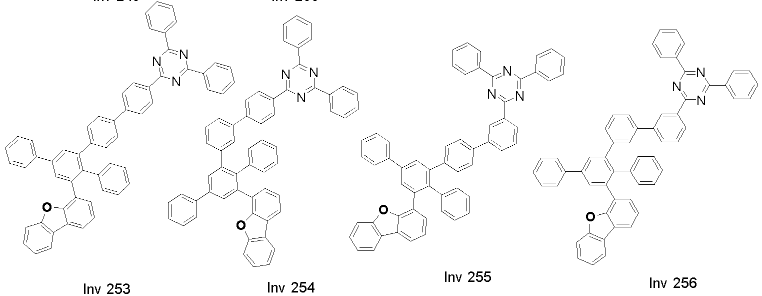

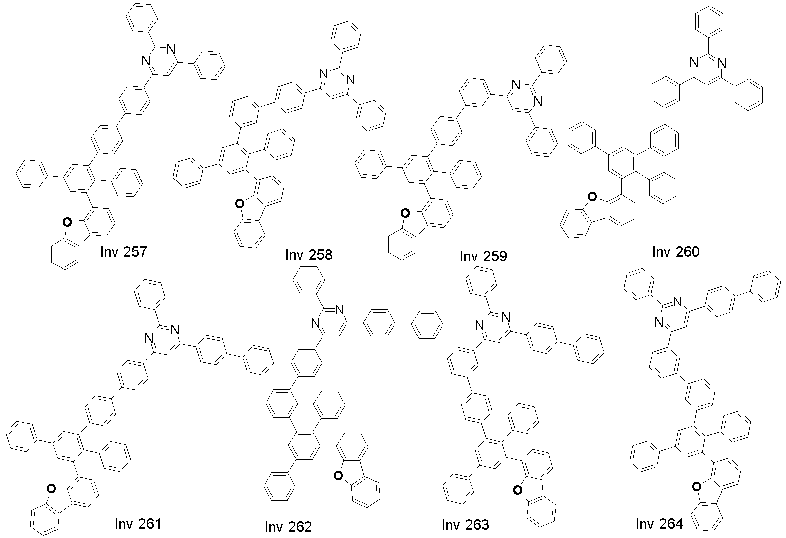

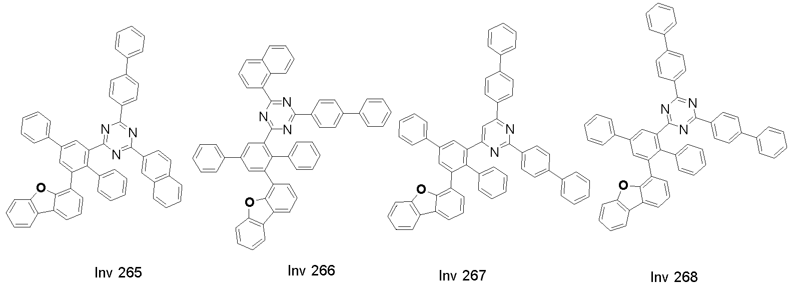

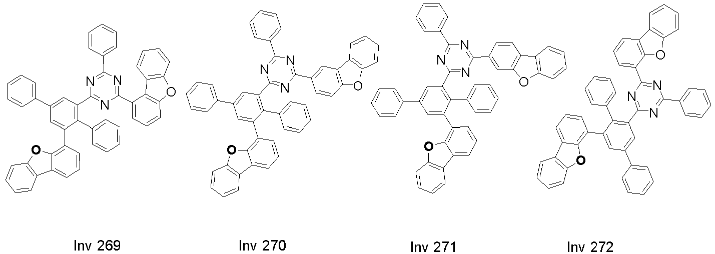

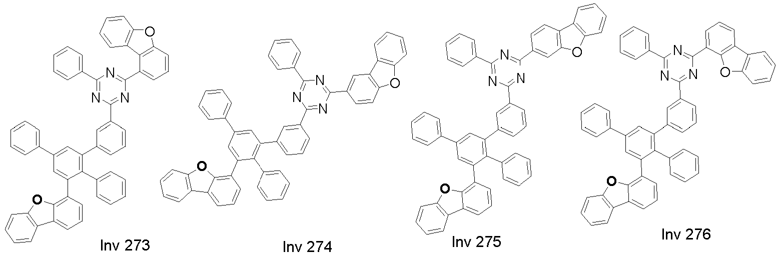

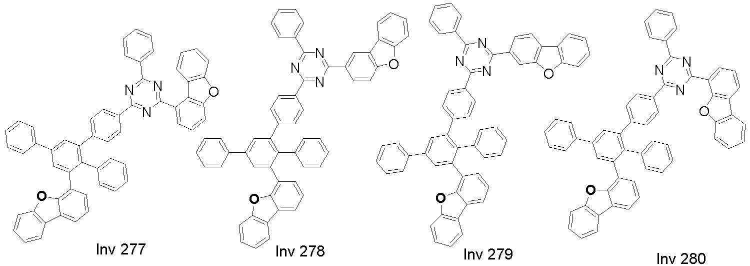

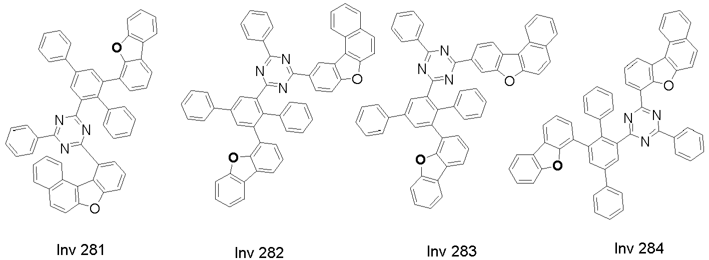

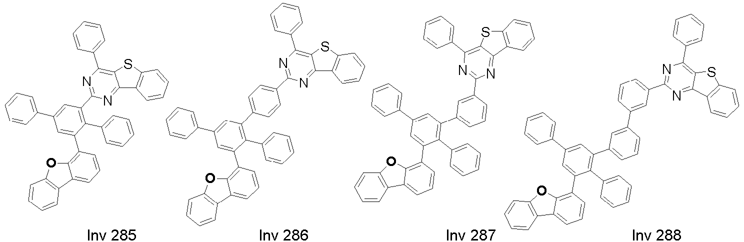

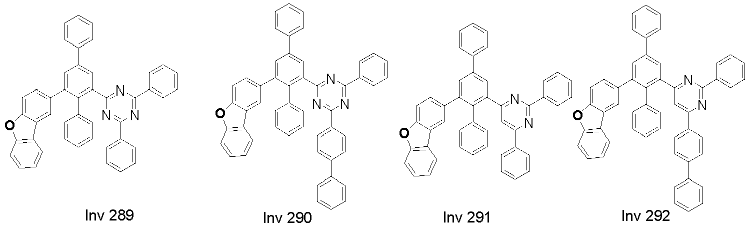

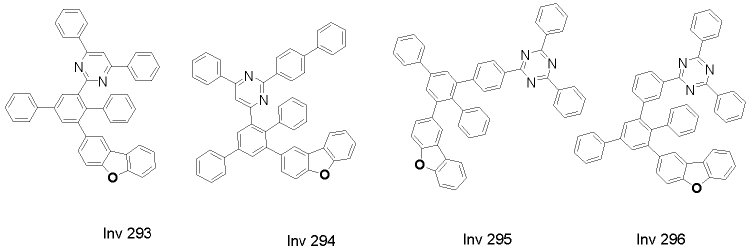

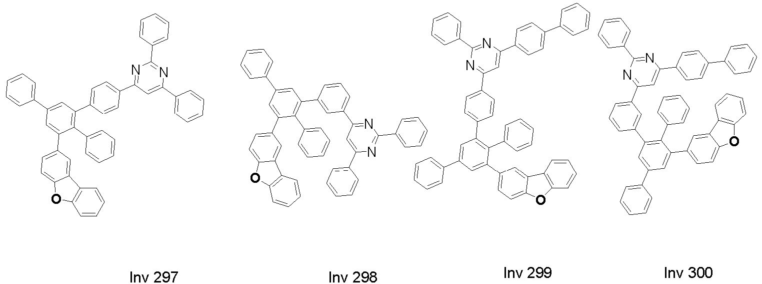

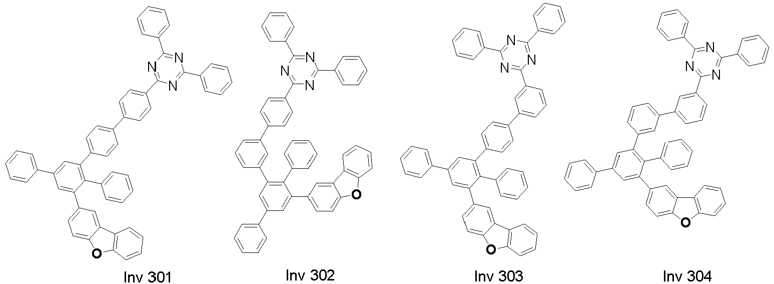

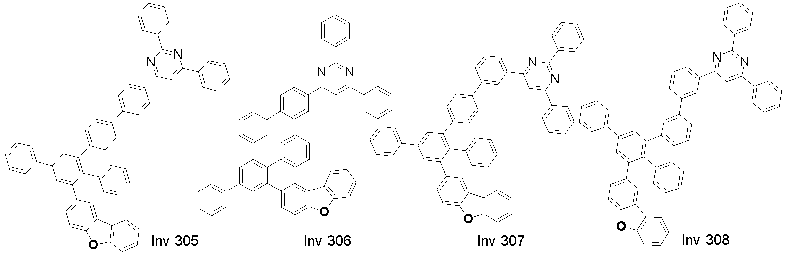

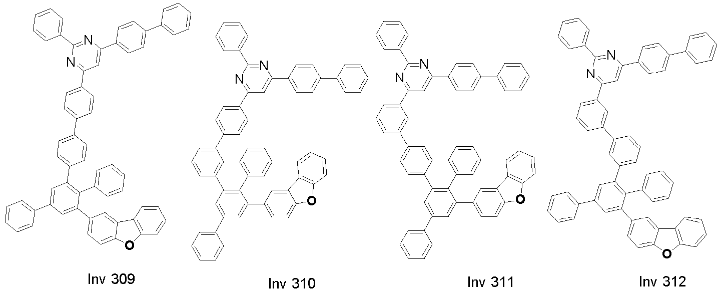

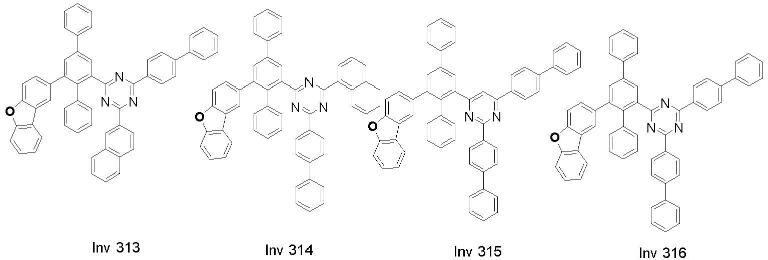

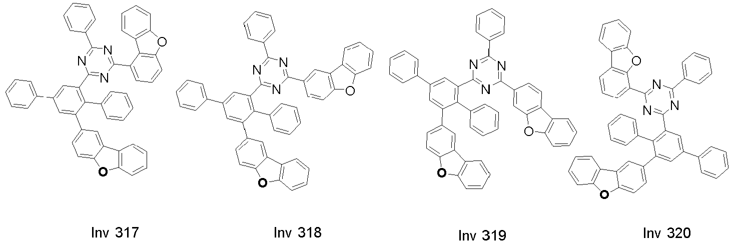

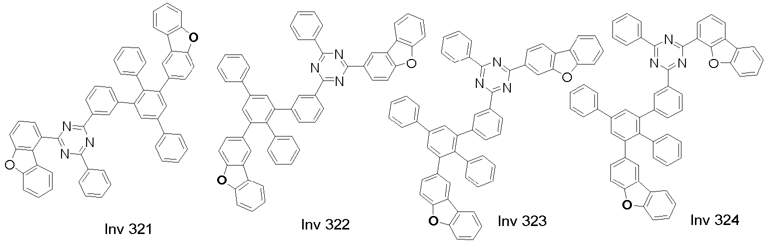

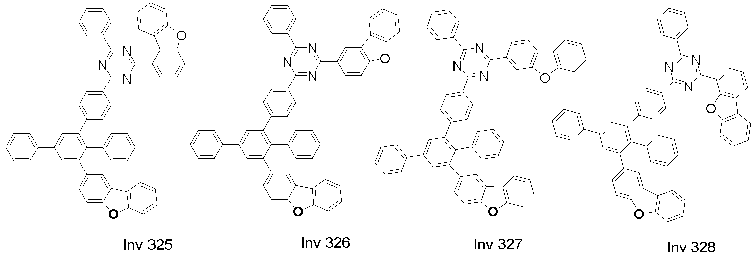

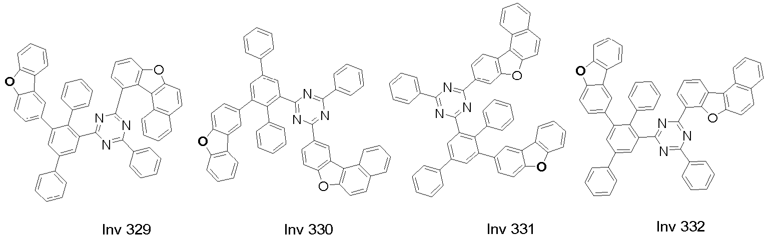

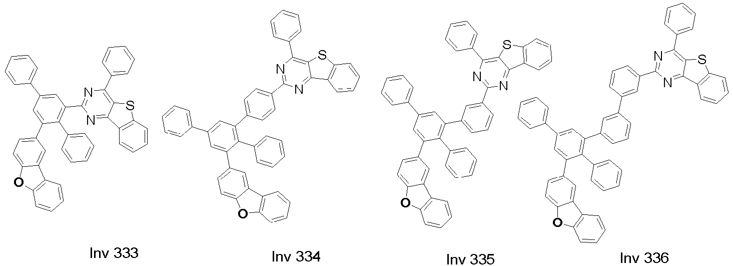

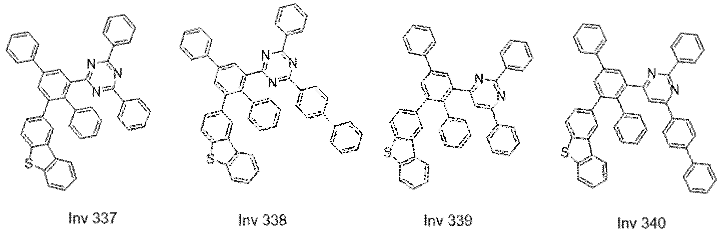

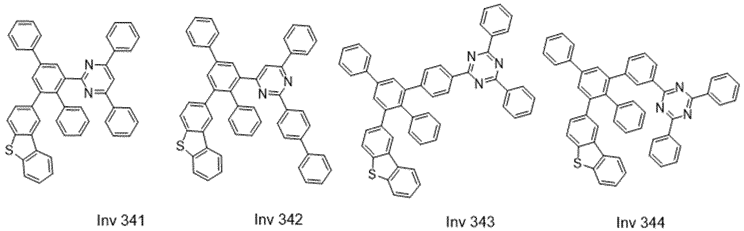

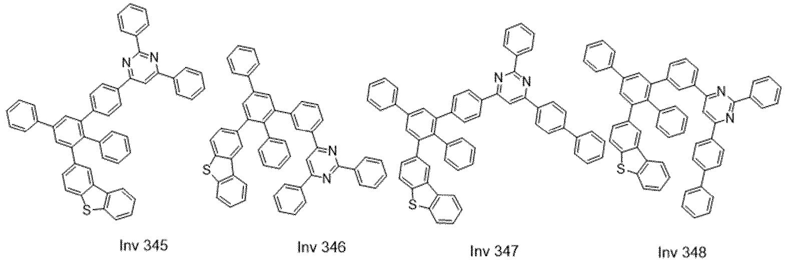

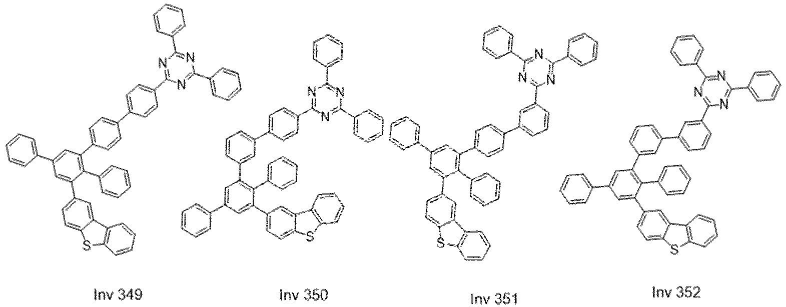

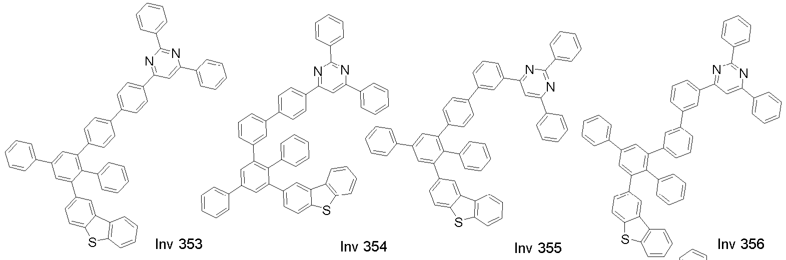

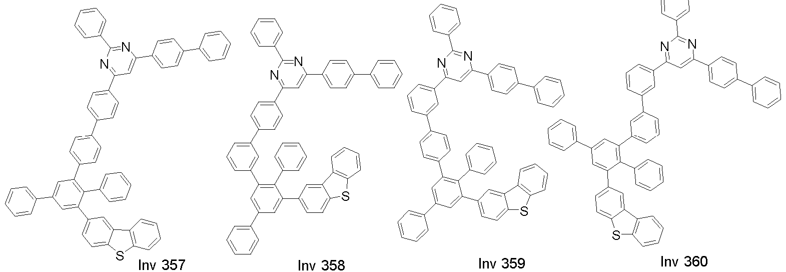

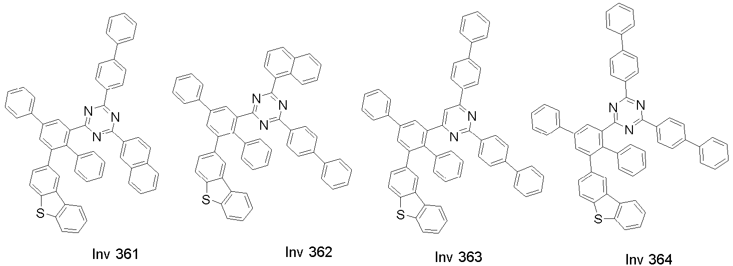

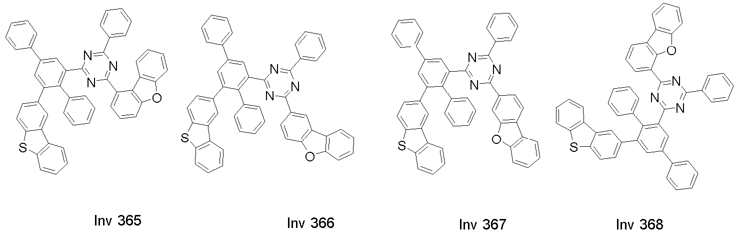

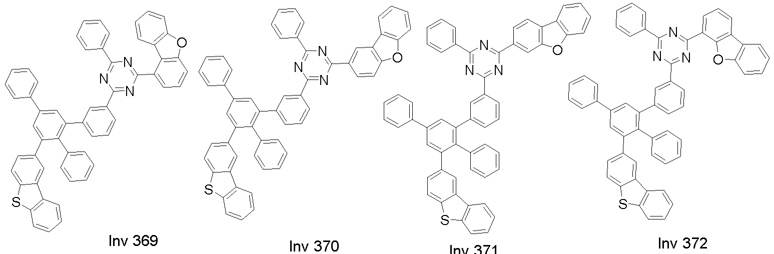

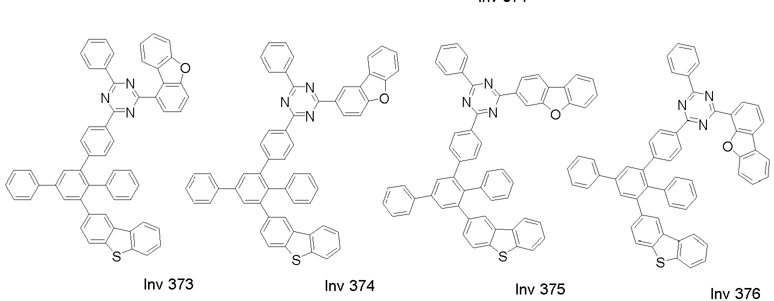

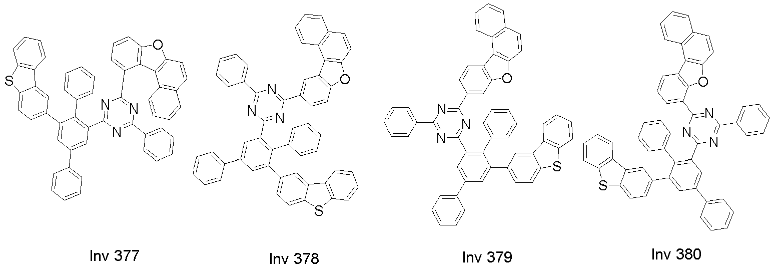

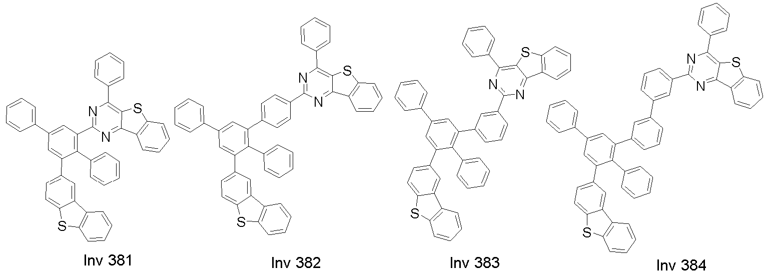

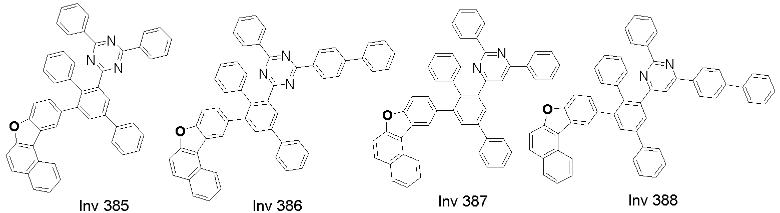

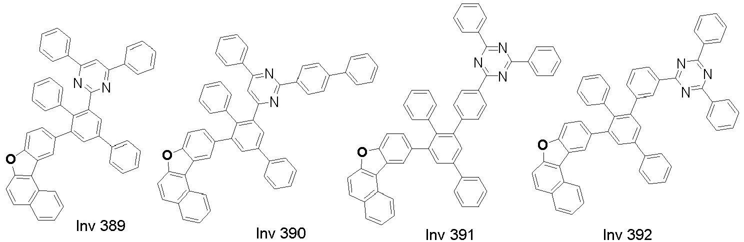

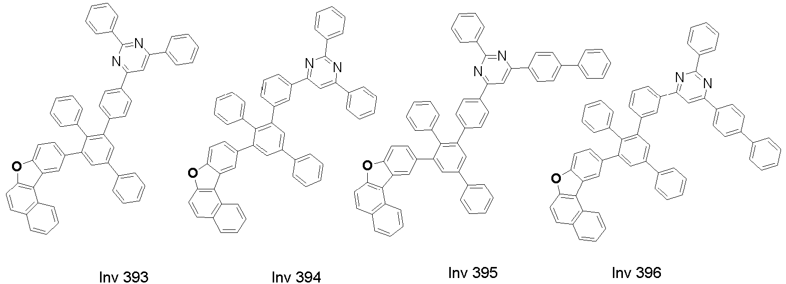

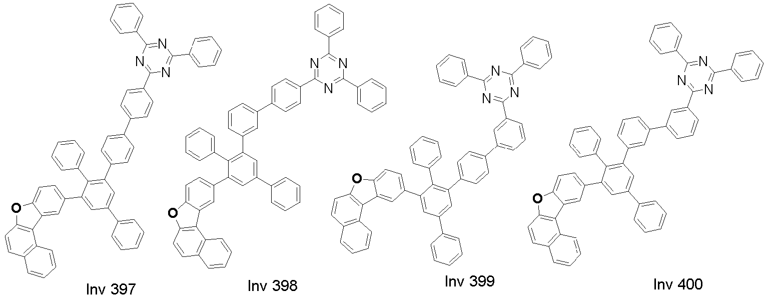

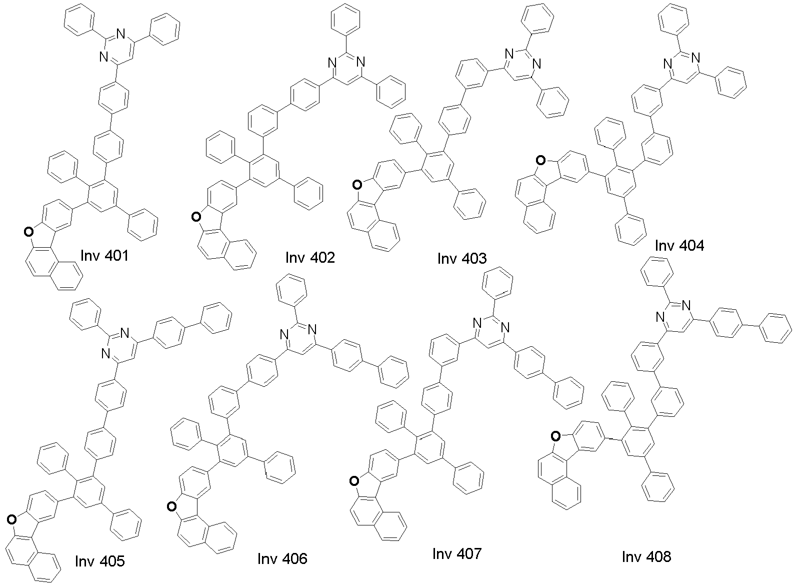

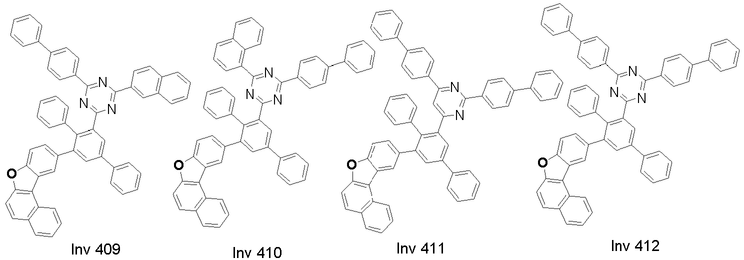

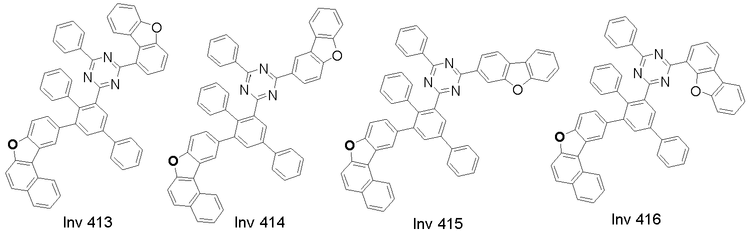

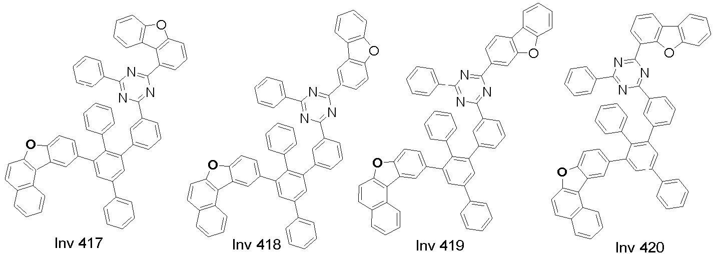

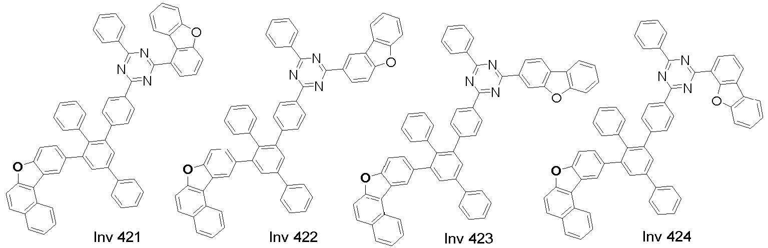

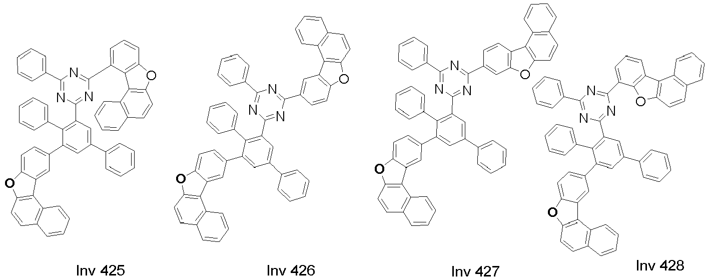

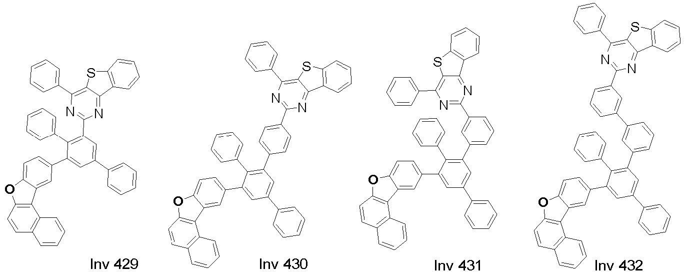

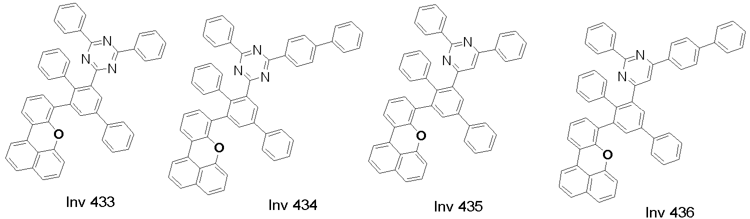

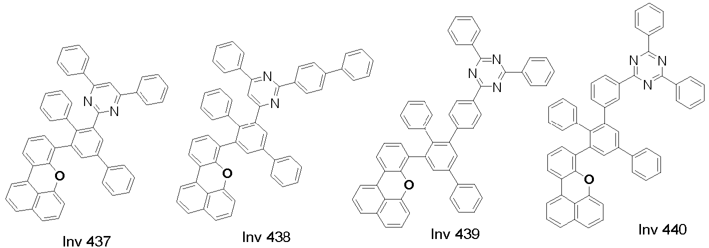

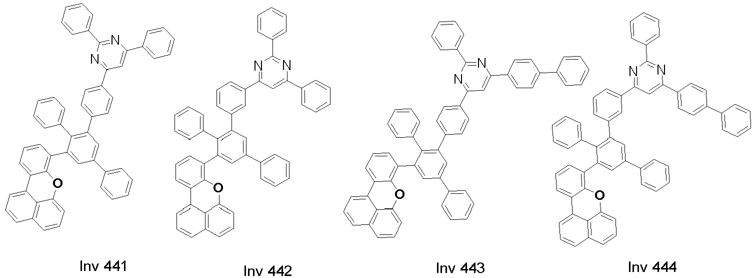

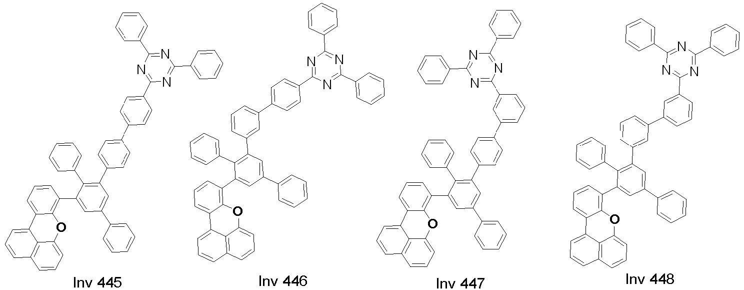

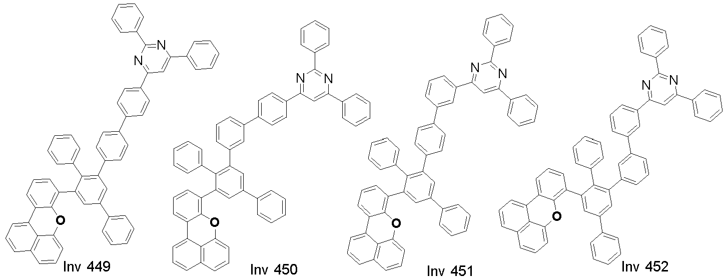

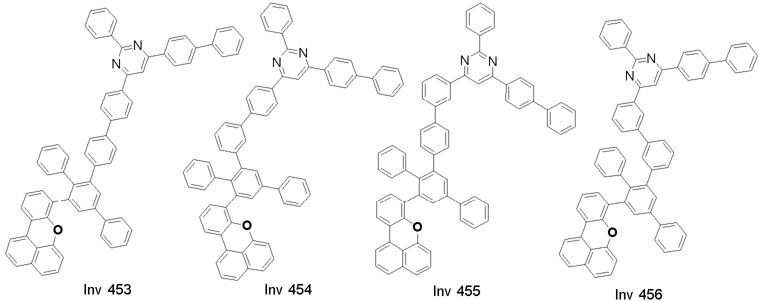

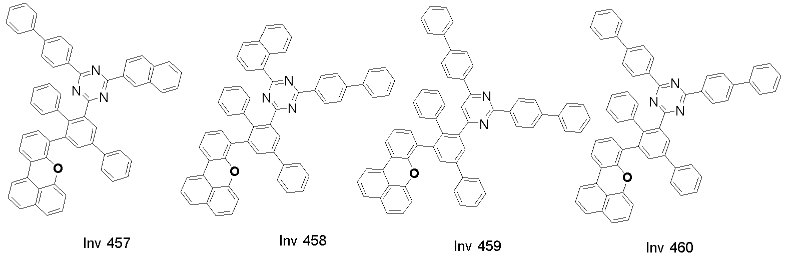

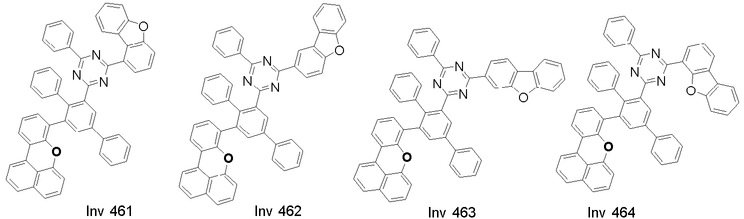

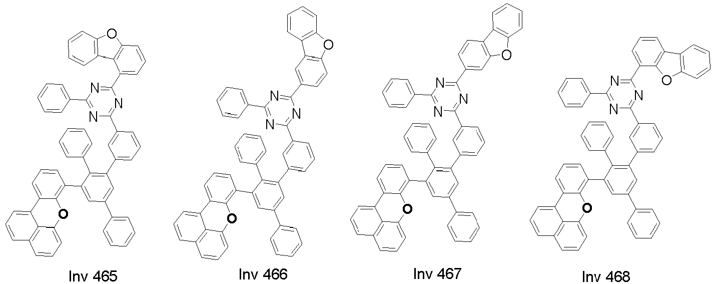

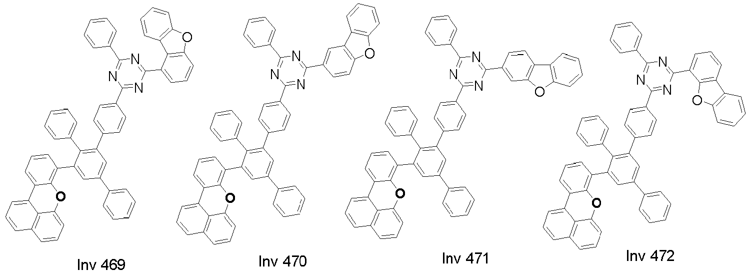

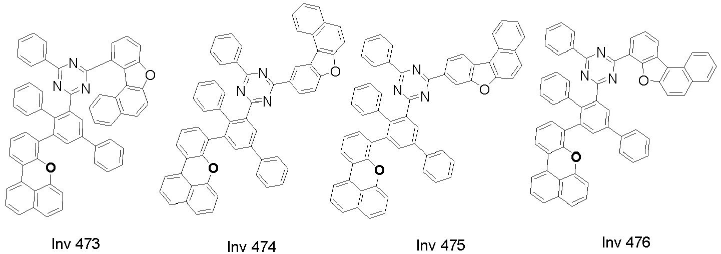

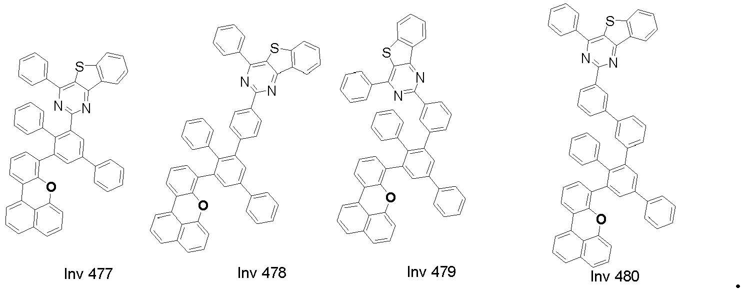

11. An organic electroluminescent device, comprising: an anode; a cathode; and one or more organic layers interposed between the anode and the cathode,
wherein at least one of the one or more organic layers contains the compound of any one of claims 1 to 10.

12. The organic electroluminescent device of claim 11, wherein the organic layer containing the compound is at least one selected from the group consisting of a light emission layer, an electron transport layer, and an electron transport auxiliary layer.
